# EUROPÄISCHE PATENTSCHRIFT

(11) **EP 0 529 452 B1**
(45) Veröffentlichungstag und Bekanntmachung des Hinweises auf die Patenterteilung: **11.11.1998**
(21) Anmeldenummer: 92113964.8
(22) Anmeldetag: 17.08.1992
(51) Int. Cl.: C07D 471/06, A61K 31/435, C07D 487/06, A61K 31/55, A61K 31/40, C07D 498/06, A61K 31/535, C07D 513/06, A61K 31/54

(54) **1,7-anellierte 3-(Piperazino-alkyl)indol-Derivate sowie Verfahren und Zwischenprodukte zu ihrer Herstellung und diese Verbindungen enthaltende Arzneimittel**
1,7-Condensed 3-(piperazinoalkyl)indol derivatives, process and intermediates for their preparation and pharmaceutical compositions containing them
Dérivés 1,7 condensés du 3-(pipérazinoalkyl)indole, procédé et intermédiaires pour leur préparation et compositions pharmaceutiques les contenant

(30) Priorität: 23.08.1991 DE 4128015
(43) Veröffentlichungstag der Anmeldung: 03.03.1993
(73) Patentinhaber: Solvay Pharmaceuticals GmbH, 30173 Hannover (DE)
(72) Erfinder: Jasserand, Daniel, F-69006 Lyon (FR); Paris, Dominique, F-01330 Amberieux en Dombes (FR); Demonchaux, Patrice, F-01400 Châtillon-sur-Chalaronne (FR); Cottin, Michel, F-01400 Châtillon-sur-Chalaronne (FR); Floc'h, François, F-69760 Limonest (FR); Dupassieux, Pierre, F-01400 Châtillon-sur-Chalaronne (FR); White, Richard, F-01000 Bourg-en-Bresse (FR)
(74) Vertreter: Lauer, Dieter, Dr.

(56) Entgegenhaltungen:
- EP-A- 384 349
- US-A- 3 468 882
- JOURNAL OF HETEROCYCLIC CHEMISTRY. Bd. 27, Nr. 7, November 1990, PROVO US Seiten 2151 - 2163 B. MCKITTRICK ET AL. 'Synthetic entries to 6-fluoro-7- substituted indole derivatives.'
- CHEMICAL ABSTRACTS, vol. 54, 1960, Columbus, Ohio, US; abstract no. 21091h, A. N. KOST ET AL. 'Some substituted lilolidines'
- CHEMICAL ABSTRACTS, vol. 58, 1963, Columbus, Ohio, US; abstract no. 4511h, A. N. KOST ET AL. 'Condensed systems with a common nitrogen atom. IV. Synthesis of benzopyrrolizines with functional groups.'
- JOURNAL OF HETEROCYCLIC CHEMISTRY. Bd. 11, Nr. 3, 1974, PROVO US Seiten 387 - 393 E. A. STECK ET AL. 'Some 5,6-dihydro-4H-pyrrolo(3,2,1-i,j)quinolines'

## Beschreibung

Die vorliegende Erfindung betrifft neue, in 3-Stellung des Indolringes einen substituierten Piperazinoalkylrest tragende 1,7-anellierte Indol-Derivate und deren Salze sowie diese Verbindungen enthaltende pharmazeutische Zubereitungen und Verfahren und Zwischenprodukte zur Herstellung dieser Verbindungen.

Aus der europäischen Patentanmeldung Veröffentlichungs- Nr. 0 322 016 Al sind Ester und Amide von 1,7-anellierten Indol-2-carbonsäure-Derivaten und cyclischen Alkoholen oder Aminen bekannt, welche selektive Antagonisten von neuronalen 5-HT-Rezeptoren darstellen und zur Behandlung von durch Überstimulation dieser Rezeptoren induzierten Beschwerden, beispielsweise im gastrointestinalen Bereich, geeignet sind.

Aus der europäischen Patentanmeldung Veröffentlichungs- Nr. 0 387 618 A1 sind Amide aus 1,7-anellierten Indol-2-carbonsäure-Derivaten mit 3-Amino-1,4-benzodiazepin-Derivaten bekannt. Diese Verbindungen besitzen CCK-antagonistische Wirkungen mit einer die Magenentleerung fördernden Wirkungskomponente.

Der vorliegenden Erfindung liegt die Aufgabe zugrunde, neue als Antiallergica einsetzbare pharmazeutische Wirkstoffe zu entwickeln. Ferner liegt der Erfindung die Aufgabe zugrunde, neue Derivate von 1,7-anellierten Indol-Verbindungen mit wertvollen pharmakologischen Eigenschaften herzustellen.

Es wurde nun gefunden, daß die erfindungsgemäßen neuen 1,7-anellierten 3-(Piperazinoalkyl) indol-Derivate wertvolle pharmakologische Eigenschaften besitzen und antiinflammatorische und antiallergische Wirkungen zeigen und ein günstiges Wirkungsprofil mit geringer Toxizität und guter Verträglichkeit aufweisen. Aufgrund ihres Wirkungsprofils eignen sich die erfindungsgemäßen Substanzen als antiinflammatorisch wirksame Wirkstoffe und Antiallergica zur Behandlung von entzündlichen und allergischen Erkrankungen.

Die vorliegende Erfindung betrifft daher neue Verbindungen der allgemeinen Formel I
(s. Formel I) worin
- R¹: Wasserstoff, C₁-C₄-Alkoxy, C₁-C₄-Alkylthio, Hydroxy, Halogen, Trifluormethyl, Nitro, Amino, C₁-C₄ Mono- oder Dialkylamino, C₁-C₇-Alkyl, welches gegebenenfalls durch Hydroxy substituiert sein kann, eine Phenyl-C₁-C₄-alkylgruppe, welche im Phenylring gegebenenfalls durch C₁-C₄ Alkyl, C₁-C₄-Alkoxy, Hydroxy oder Halogen substituiert sein kann, C₃-C₆-Cycloalkyl, C₄-C₇-Cycloalkylalkyl, C₃-C₇-Alkenyl, C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl-, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet,
- R²: Wasserstoff, Halogen, C₁-C₄-Alkyl oder, falls R¹ nicht Hydroxy oder eine hydroxyphenylhaltige Gruppe ist, auch C₁-C₄-Alkoxy bedeutet,
- R³: Wasserstoff, C₁-C₄-Alkyl, welches gegebenenfalls durch Hydroxy substituiert sein kann, C₁-C₄-Alkenyl, C₃-C₆-Cycloalkyl, C₄-C₇-Cycloalkylalkyl oder eine Phenyloder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, bedeutet, wobei jedoch R³ nur dann eine freie Hydroxygruppe enthalten kann, wenn R¹ keine Carbonyloxygruppe enthält,
- R⁴: Wasserstoff, C₁-C₇-Alkyl, welches gegebenenfalls durch Hydroxy substituiert sein kann, C₃-C₇-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, bedeutet, wobei jedoch R⁴ nur dann eine freie Hydroxygruppe enthalten kann, wenn R¹ keine Carbonyloxygruppe enthält,
- A: eine gegebenenfalls durch C₁-C₄-Alkyl substituierte Alkylenkette mit 1 - 2 Kohlenstoffatomen, eine Bindung, Sauerstoff oder Schwefel bedeutet,
- Z: eine- Alkylenkette mit 2 - 4 Kohlenstoffatomen, welche gegebenenfalls durch C₁-C₄-Alkyl oder, falls R¹ keine Carbonyloxygruppe enthält, auch durch Hydroxy substituiert sein kann, bedeutet,
- B: Stickstoff oder die CH-Gruppe bedeutet,
- R⁵: einen gegebenenfalls durch C₁-C₄-Alkyl, C₁-C₄-Alkoxy oder Halogen substituierten Pyridyl- oder Phenylrest bedeutet, und
- D: eine Bindung darstellt oder, falls B die CH-Gruppe und R⁵ einen gegebenenfalls substituierten Phenylrest bedeuten, auch die CO-Gruppe darstellen kann,
und deren Säureadditionssalze und/oder S-Mono- oder -Dioxide von schwefelhaltigen Verbindungen der Formel I.

Sofern in den Verbindungen der Formel I die Substituenten C₁-C₄-Alkylgruppen bedeuten oder enthalten, können diese Alkylgruppen gerade oder verzweigt sein und insbesondere 1 - 4, vorzugsweise 1 - 2, Kohlenstoffatome enthalten und stellen bevorzugt Methyl dar. Sofern die Substituenten Halogen bedeuten oder Halogensubstituenten enthalten, kommen insbesondere Fluor, Chlor oder Brom, vorzugsweise Chlor, in Frage.

Der Substituent R¹ kann Wasserstoff bedeuten. Als günstig erweisen sich auch gegebenenfalls durch Hydroxy substituierte Alkylgruppen mit 1 - 7, vorzugsweise 1 - 6 Kohlenstoffatomen, insbesondere C₁-C₄-Alkylgruppen, beispielsweise Methyl, eine Alkenylgruppe mit bis zu 7, insbesondere bis zu 4 Kohlenstoffatomen, Halogen, Hydroxy, eine C₁-C₄-Alkoxygruppe, insbesondere Methoxy, und Acyl-, Acyloxy- und Acylamino-Reste, welche Alkanoyl mit 2 - 7, vorzugsweise 2 - 5 Kohlenstoffatomen, gegebenenfalls substituiertes Benzoyl oder gegebenenfalls substituiertes Cinnamoyl enthalten, insbesondere C₁-C₄-Alkanoyl oder Benzoyl enthaltende Reste. In dem Substituenten R¹ enthaltene Phenylringe sind vorzugsweise unsubstituiert, können aber auch durch C₁-C₄-Alkyl, insbesondere Methyl, C₁-C₄-Alkoxy, insbesondere Methoxy, Hydroxy oder Halogen, insbesondere Chlor, mono- oder disubstituiert sein. Ein Substituent R¹ kann günstigerweise in 5- oder 4-Stellung des Indolgerüstes angeordnet sein.

Der Substituent R² stellt vorzugsweise Wasserstoff dar oder kann auch Halogen, insbesondere Chlor, C₁-C₄-Alkyl, insbesondere Methyl, oder C₁-C₄-Alkoxy, insbesondere Methoxy, bedeuten.

Der Substituent R³ kann Wasserstoff, einen aliphatischen Kohlenwasserstoffrest wie C₁-C₄-Alkyl oder Alkenyl oder cyclisches Alkyl mit bis zu 7 Kohlenstoffatomen bedeuten, wobei ein Alkylrest gegebenenfalls durch Hydroxy substituiert sein kann, oder R³ kann auch gegebenenfalls im Phenylring substituiertes Phenyl oder Phenyl-C₁-C₄-alkyl bedeuten. Als günstig erweisen sich C₁-C₄-Alkylgruppen R³, insbesondere Methyl. In dem Rest R³ enthaltene Phenylgruppen können unsubstituiert oder mit den vorstehend angegebenen Resten mono- oder disubstituiert sein. Als- Substituenten der Phenylgruppe eignen sich z.B. C₁-C₄-Alkoxy, insbesondere Methoxy, oder auch Hydroxy.

Der Substituent R⁴ kann Wasserstoff oder einen aliphatischen Kohlenwasserstoffrest mit bis zu 7 Kohlenstoffatomen wie geradkettiges, verzweigtes oder cyclisches Alkyl oder Alkenyl bedeuten, wobei ein Alkylrest gegebenenfalls durch Hydroxy substituiert sein kann, oder R⁴ kann eine gegebenenfalls im Phenylring substituierte Phenyl- oder Phenyl-C₁-C₄-alkylgruppe darstellen.

Als günstig erweist sich beispielsweise Alkyl mit bis zu 7 Kohlenstoffatomen.

A kann eine gegebenenfalls durch C₁-C₄-Alkyl substituierte Alkylenkette mit 1 - 2 Kohlenstoffatomen oder eine Bindung darstellen oder auch Sauerstoff oder Schwefel bedeuten. Als günstig erweisen sich beispielsweise 5,6-Dihydro-4H-pyrrolo[3,2,1-ij]chinolin-derivate der Formel I, das sind Verbindungen, worin A eine gegebenenfalls durch C₁-C₄-Alkyl substituierte Methylengruppe darstellt.

Z steht für eine gegebenenfalls durch C₁-C₄-Alkyl oder Hydroxy substituierte Alkylenkette mit 2 - 4, insbesondere 2 oder 3, Kohlenstoffatomen. Als günstig erweist sich beispielsweise eine unsubstituierte oder gegebenenfalls auch durch C₁-C₄-Alkyl oder Hydroxy substituierte Ethylenkette.

Falls der Substituent R⁵ einen Pyridylrest darstellt, kann dieser unsubstituiert oder durch C₁-C₄-Alkyl, C₁-C₄-Alkoxy oder Halogen substituiert sein. Beispielsweise eignen sich durch C₁-C₄-Alkyl, insbesondere Methyl, substituierte oder unsubstituierte Pyridylreste. Bevorzugt kommt eine Pyridin-2-yl-gruppe in Frage, welche gegebenenfalls substituiert sein kann. Als besonders günstig erweist sich der 4-Methylpyridin-2-yl-Rest. Falls R⁵ einen Phenylrest bedeutet, kann dieser unsubstituiert oder durch Halogen, C₁-C₄-Alkyl, insbesondere Methyl, oder C₁-C₄-Alkoxy, insbesondere Methoxy, mono- oder disubstituiert sein. Falls R⁵ einen gegebenenfalls substituierten Phenylrest darstellt, steht D vorzugsweise für die CO-Gruppe.

Erfindungsgemäß werden die neuen Verbindungen der Formel I und deren Säureadditionssalze und/oder S-Mono- oder -Dioxide von schwefelhaltigen Verbindungen der Formel I erhalten, indem man in an sich bekannter Weise
a) zur Herstellung von Verbindungen der allgemeinen Formel Ia
   (s. Formel Ia)
   worin R^{1'} die für R¹ angegebene Bedeutung mit Ausnahme von hydroxy-substituiertem C₁-C₇-Alkyl oder Mono- oder Di-C₁-C₄-alkylamino besitzt, R^{3'} und R⁴' die für R³ und R⁴ angegebenen Reste mit Ausnahme von C₁-C₄-Hydroxyalkylgruppen enthaltenden Resten bedeuten, Z' die für Z angegebene Bedeutung besitzt, wobei jedoch die Alkylenkette Z nur in der dem Indolgerüst benachbarten Position einen allfälligen Hydroxysubstituenten enthalten kann, und R², R⁵, A, B und D obige Bedeutung besitzen, Verbindungen der allgemeinen Formel II
   (s. Formel II)
   worin R^{1'}, R², R^{3'}, R^{4'}, A und Z' obige Bedeutung besitzen, und X für eine aminolytisch abspaltbare Fluchtgruppe steht, mit Verbindungen der allgemeinen Formel III
   (s. Formel III)
   worin B, D und R⁵ obige Bedeutung besitzen, umsetzt, oder
b) zur Herstellung von Verbindungen der allgemeinen Formel Ib
   (s. Formel Ib)
   worin R², R³, R⁴, A, B und R⁵ obige Bedeutung besitzen, R^{1"} die für R¹ angegebenen Reste mit Ausnahme von CO-haltigen Resten darstellt und Z" eine -Z"' -CH₂-Kette darstellt, worin Z"' eine Alkylenkette mit 1 - 3 Kohlenstoffatomen, welche gegebenenfalls durch C₁-C₄-Alkyl oder auch durch Hydroxy substituiert sein kann, bedeutet, oder, falls R^{1"}, R³ und/oder R⁴ einen Hydroxyalkyl enthaltenden Rest bedeuten oder R^{1"} eine Mono- oder Di-C₁-C₄-alkylaminogruppe bedeutet, Z" auch die für Z' angegebene Bedeutung besitzen kann, Verbindungen der allgemeinen Formel IV
   (s. Formel IV)
   worin R², R⁵, A und B obige Bedeutung besitzen, R^{1III} die für R¹ angegebenen Reste mit Ausnahme von die -COO-Gruppe, die -CONH-Gruppe oder eine Hydroxyalkyl-Gruppe enthaltenden Resten bedeutet oder eine N-Formyl- oder N-C₁-C₄-alkanoylsubstituierte Amino- oder C₁-C₄-alkylaminogruppe bedeutet, R⁶ die für R³ angegebene Bedeutung besitzt oder eine C₁-C₄-alkoxycarbonyl- oder C₁-C₄-alkoxycarbonyl-C₁-C₄-alkylgruppe oder eine im Phenylring durch C₁-C₄-alkoxycarbonyl-C₁-C₄-alkoxy substituierte Phenyl- oder PhenylC₁-C₄-alkylgruppe bedeutet, R⁷ die für R⁴ angegebene Bedeutung besitzt oder eine C₁-C₄-alkoxycarbonyl- oder C₁-C₄-alkoxycarbonyl-C₁-C₄-alkylgruppe oder eine im Phenylring durch C₁-C₄-alkoxycarbonyl-C₁-C₄-alkoxy substituierte Phenyl- oder Phenyl-C₁-C₄-alkylgruppe bedeutet und Q eine -Q'-CO-Kette darstellt, worin Q eine Alkylenkette mit 1 - 3 Kohlenstoffatomen, welche gegebenenfalls durch C₁-C₄-Alkyl oder durch Oxo substituiert sein kann, darstellt, oder Q eine in der dem Indolgerüst benachbarten Position durch Oxo substituierte Alkylenkette mit 2 - 4 Kohlenstoffatomen, welche gegebenenfalls durch C₁-C₄-Alkyl substituiert sein kann, bedeutet oder, falls R^{1III}, R⁶ und/oder R⁷ einen CO-haltigen Rest bedeuten, Q auch die für Z' angegebene Bedeutung besitzen kann, reduziert, oder
c) zur Herstellung von Verbindungen der allgemeinen Formel Ic
   (s. Formel Ic)
   worin R², R^{3'}, R^{4'}, A und Z obige Bedeutung besitzen, R^{5'} für einen gegebenenfalls durch C₁-C₄-Alkyl, C₁-C₄-Alkoxy oder Halogen substituierten Pyridylrest steht und R^{1IV} die für R¹ angegebene Bedeutung mit Ausnahme von hydroxysubstituiertem C₁ - C₇-Alkyl besitzt, Verbindungen der allgemeinen Formel V
   (s. Formel V)
   worin R^{1IV}, R², R^{3'}, R^{4'}, A und Z obige Bedeutung besitzen, mit einer Verbindung der allgemeinen Formel VI

   X' -R^{5'} VI

   worin R^{5'} obige Bedeutung besitzt und X' Halogen bedeutet, umsetzt, oder
d) zur Herstellung von Verbindungen der allgemeinen Formel Id
   (s. Formel Id)
   worin R², R³, R⁴, A, Z, B, D und R⁵ obige Bedeutung besitzen und R^{1V} C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl-, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet, Verbindungen der allgemeinen Formel Ie
   (s. Formel Ie)
   worin R², R³, R⁴, A, Z, B, D und R⁵ obige Bedeutung besitzen und R^{1VI} Wasserstoff, Amino oder, sofern R³, R⁴ und/oder Z keine freien Hydroxygruppen enthalten, auch Hydroxy bedeutet, mit Säuren oder reaktionsfähigen Säurederivaten der allgemeinen Formel VII

   R⁸-Y VII

   worin R⁸ C₂-C₇-Alkanoyl, eine Benzoylgruppe, deren Phenylring gegebenenfalls durch C₁-C₄-Alkyl, C₁-C₄-Alkoxy, Hydroxy oder Halogen substituiert sein kann, oder eine Cinnamoylgruppe, deren Phenylring gegebenenfalls durch C₁-C₄-Alkyl, C₁-C₄-Alkoxy, Hydroxy oder Halogen substituiert sein kann, bedeutet und Y Hydroxy oder eine reaktive Gruppe bedeutet, acyliert, und gewünschtenfalls in erhaltenen Verbindungen der Formel I, worin R¹ Methoxy bedeutet oder eine Methoxyphenylgruppe enthält und/oder R³ und/oder R⁴ eine Methoxyphenylgruppe darstellen oder enthalten, die Methoxygruppe zur Hydroxygruppe spaltet und/oder in Verbindungen der Formel I, worin R¹, R³ und/oder R⁴ eine Hydroxyalkylgruppe mit mindestens 2 Kohlenstoffatomen enthalten, diese durch Wasserabspaltung in eine entsprechende Alkenylgruppe überführt oder in Verbindungen der Formel I, worin nur R¹ eine Hydroxyalkylgruppe enthält, diese zu der entsprechenden Alkanoylgruppe oxidiert und/oder Verbindungen der Formel I, worin R¹ Amino bedeutet, zu entsprechenden Verbindungen der Formel I, worin R¹ C₁-C₄-Mono- oder Dialkylamino bedeutet, alkyliert und/oder schwefelhaltige Verbindungen der Formel I in die entsprechenden S-Mono- oder -Dioxide überführt und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III gemäß Verfahrensvariante a) kann nach an sich zur Alkylierung von Aminen üblichen Methoden ausgeführt werden. Die Umsetzung wird zweckmäßigerweise in einem unter den Reaktionsbedingungen inerten organischen Lösungsmittel unter basischen Bedingungen durchgeführt.

Als aminolytisch abspaltbare Reste X in den Verbindungen der Formel II eignen sich Halogene wie Chlor, Brom oder Jod, vorzugsweise Brom, oder auch ein Acyloxyrest O-E, worin E einen C₁-C₄-Alkanoylrest oder einen organischen Sulfonsäurerest darstellt, beispielsweise den Rest von einer C₁-C₄-alkansulfonsäure, wie z.B. Methansulfonsäure, oder von aromatischen Sulfonsäuren, wie Benzolsulfonsäure oder durch C₁-C₄-Alkyl oder durch Halogen substituierten Benzolsulfonsäuren, z.B. Toluolsulfonsäuren oder Brombenzolsulfonsäuren. Sofern Verbindungen der Formel II freie Hydroxygruppen in den Resten R^{1'}, R^{3'} und/oder R^{4'} enthalten, stellt X zweckmäßigerweise Halogen dar. Als inerte organische Lösungsmittel eignen sich insbesondere aprotische Lösungsmittel, beispielsweise aromatische Kohlenwasserstoffe wie Toluol, Xylol oder Benzol, cyclische Ether wie Dioxan, Dimethylformamid, C₁-C₄-Alkanole wie Ethanol oder Gemische aus den vorgenannten Lösungsmitteln. Zweckmäßigerweise wird bei erhöhten Temperaturen, beispielsweise Temperaturen zwischen 50 und 150 °C, gearbeitet. Zweckmäßigerweise wird die Reaktion unter Zusatz einer organischen oder anorganischen Base durchgeführt. Es kann jedoch auch ein Überschuß der Verbindung der Formel III verwendet und dieser als interne Base benutzt werden. Beispiele geeigneter organischer Basen sind tertiäre organische Amine, insbesondere tertiäre C₁-C₄-alkylamine wie Triethylamin, Tripropylamine, N-C₁-C₄-alkylmorpholine oder N-C₁-C₄-alkylpiperidine. Geeignete anorganische Basen sind insbesondere Alkalimetallcarbonate oder -bicarbonate. Gewünschtenfalls kann die Reaktion durch Zusatz einer katalytisch wirksamen Menge Kaliumjodid gefördert werden. Die Reaktionszeit kann je nach Reaktionsbedingungen zwischen 1 und 15 Stunden betragen. Freie Aminogruppen R^{1'} müssen während der Umsetzung der Verbindungen der Formel II mit den Piperazinen der Formel III auf an sich bekannte Weise durch leicht wieder abspaltbare Schutzgruppen geschützt werden. Gewünschtenfalls können auch freie phenolische Hydroxygruppen während der Reaktion durch eine anschließend leicht wieder abspaltbare Schutzgruppe geschützt werden. Als Schutzgruppen können an sich bekannte Schutzgruppen gewählt werden, welche anschließend auf an sich bekannte Weise solvolytisch oder hydrogenolytisch wieder abgespalten werden können. Geeignete leicht wieder abspaltbare Schutzgruppen für Aminogruppen und für phenolische OH-Gruppen sind z.B. bekannt aus E. Mc Omie "Protective Groups in Organic Chemistry" Plenum Press 1971. Beispielsweise eignen sich zum Schutz einer Amino- oder C₁-C₄-alkylaminogruppe die Formyl- oder die Acetylgruppe, welche nach Beendigung der Reaktion hydrolytisch wieder abgespalten werden können. Als Schutzgruppen für eine allfällige phenolische Hydroxygruppe können an sich bekannte Etherschutzgruppen gewählt werden, welche anschließend auf an sich bekannte Weise solvolytisch oder hydrogenolytisch wieder abgespalten werden, z.B. C₁-C₄-Alkyl- oder Benzylgruppen. Schutzgruppen müssen selbstverständlich unter Berücksichtigung der übrigen in der zu schützenden Verbindung enthaltenen Reste jeweils so ausgewählt werden, daß sie anschließend leicht unter Bedingungen, unter denen andere im Molekül enthaltene Reste nicht angegriffen werden, abspaltbar sind.

Die Reduktion von Verbindungen der Formel IV gemäß Verfahrensvariante b) kann nach an sich zur Reduktion von Amiden und/oder zur Reduktion von Alkoxycarbonylgruppen üblichen Methoden erfolgen. Als Reduktionsmittel eignen sich komplexe Metallhydride. So erweisen sich beispielsweise zur Reduktion von Amiden der Formel IV (Q = Q'-CO und/oder R^{1III} = Acylamino) zur Amidreduktion befähigte komplexe Metallhydride, inbesondere Aluminiumhydride wie Lithiumaluminiumhydrid oder Natrium-(2-methoxyethoxy)-dihydroaluminat, oder auch Lithiumborhydrid oder Diboran als geeignet. Die Reaktion findet in einem unter den Reaktionsbedingungen inerten ausreichend wasserfreien Lösungsmittel statt. Als Lösungsmittel eignen sich beispielsweise cyclische Ether wie Tetrahydrofuran oder Dioxan oder offenkettige Ether wie Diethylether, Ethylenglycoldimethylether oder Diethylenglycoldimethylether, gegebenenfalls im Gemisch mit aromatischen Kohlenwasserstoffen wie Benzol oder Toluol. In dem Rest R^{1III} oder der Kette Q' enthaltene -CO-Gruppen werden unter den Reaktionsbedingungen zu der entsprechenden -CH-OH-Gruppe reduziert. Eine zur CO-Gruppe benachbarte CO-Gruppe der Q'-Kette kann je nach Reaktionstemperatur und Art und Menge des eingesetzten Reduktionsmittels, inbesondere bei Verwendung von Lithiumaluminiumhydrid oder Diboran, auch bis zur -CH₂-Gruppe durchreduziert werden, oder es können Gemische aus Verbindungen, worin die CO-Gruppe der Q'-Kette zur -CH-OH-Gruppe reduziert wurde, und durchreduzierten Verbindungen entstehen. Derartige Gemische können nach üblichen Methoden, beispielsweise chromatographisch, aufgetrennt werden. Sofern die Verbindungen der Formel IV keine Amidfunktion enthalten, also nur C₁-C₄-Alkoxycarbonylgruppen in den Resten R⁶ oder R⁷, eine CO-Gruppe in dem Rest R^{1III} und/oder eine dem Indolgerüst benachbarte CO-Gruppe der Kette Q zur Hydroxymethylgruppe zu reduzieren sind, eignen sich außer den vorgenannten komplexen Metallhydriden als Reduktionsmittel auch Di-C₁-C₄-alkylaluminiumhydride wie Dibutylaluminiumhydrid oder auch Di-C₁-C₄-alkylborhydride oder Natriumborhydrid.

Die Reaktionstemperatur kann je nach Art des verwendeten Reduktionsmittels und der zu reduzierenden Funktion zwischen 0 °C und Siedetemperatur des Reaktionsgemisches variieren. Als günstig erweist sich beispielsweise zur Reduktion einer Amidfunktion die Reaktion mit Lithiumaluminiumhydrid bei Siedetemperatur des Reaktionsgemisches. Die Reaktionszeit kann zwischen 1 und 10 Stunden betragen.

Die Umsetzung von Verbindungen der Formel V mit Verbindungen der Formel VI gemäß Verfahrensvariante c) kann nach an sich zur Alkylierung von Aminen üblichen Methoden ausgeführt werden. Sie kann beispielsweise in der für die Umsetzung der Verbindungen der Formel II mit Verbindungen der Formel III beschriebenen Weise erfolgen. Freie Amino- oder C₁-C₄-alkylaminogruppen R^{1IV} müssen während der Umsetzung auf an sich bekannte Weise durch anschließend wieder abspaltbare Schutzgruppen geschützt werden.

Die Acylierung von Verbindungen der Formel Ie gemäß Verfahrensvariante d) kann nach an sich bekannten Methoden erfolgen. Als Acylierungsmittel können die Säuren der Formel VIIa
(s. Formel VIIa)
worin R⁸ obige Bedeutung besitzt, oder deren reaktionsfähige Säurederivate eingesetzt werden. Als reaktionsfähige Derivate kommen insbesondere Säurehalogenide, gegebenenfalls gemischte Säureanhydride und Ester in Frage. So können reaktive Gruppen Y beispielsweise Halogene wie Chlor oder Brom, C₁-C₄-Alkoxy, oder Acyloxyreste wie C₁-C₄-Alkanoyloxy, den R⁸-O-Rest oder organische Sulfonsäurereste darstellen, beispielsweise Reste von C₁-C₄-alkansulfonsäuren wie z.B. Methansulfonsäure oder von aromatischen Sulfonsäuren wie Benzolsulfonsäure oder durch C₁-C₄-Alkyl oder Halogen substituierten Benzolsulfonsäuren.

Die Acylierung solcher Verbindungen der Formel Ie, worin R^{1VI} Amino oder Hydroxy bedeutet, kann nach an sich zur Bildung von Ester- oder Amidgruppierungen durch Acylierung von aromatischen Aminen oder Phenolen üblichen Methoden ausgeführt werden. So kann die Acylierung zweckmäßig in einem unter den Reaktionsbedingungen inerten Lösungsmittel bei Temperaturen zwischen Raumtemperatur und dem Siedepunkt des Lösungsmittels erfolgen. Als Lösungsmittel eignen sich halogenierte Kohlenwasserstoffe wie Dichlormethan, Chloroform, Dichlorethan oder Tetrachlorkohlenstoff, aromatische Kohlenwasserstoffe wie Benzol, Toluol, Xylol oder Chlorbenzol, cyclische Ether wie Tetrahydrofuran oder Dioxan, Dimethylformamid oder Gemische dieser Lösungsmittel. Die Acylierung kann gegebenenfalls, insbesondere wenn ein Säurehalogenid oder -anhydrid der Formel VII verwendet wird, in Gegenwart eines säurebindenden Reagenzes durchgeführt werden. Als säurebindende Mittel eignen sich organische oder anorganische Basen. Beispiele geeigneter organischer Basen sind tertiäre organische Amine, insbesondere tertiäre C₁-C₄-alkylamine wie Triethylamin, Tripropylamine oder N-C₁-C₄-alkylpiperidine. Geeignete anorganische Basen sind insbesondere Alkalimetallcarbonate oder -bicarbonate.Falls als Acylierungsmittel die Säure selbst oder auch ein Ester eingesetzt werden, kann die Umsetzung der Verbindung der Formel Ie mit der Säure der Formel VII zweckmäßig in Gegenwart eines dehydratisierenden Reagenzes, beispielsweise eines aus der Peptidchemie als zur Amidbildung geeignet bekannten Kopplungsreagenzes, durchgeführt werden. Als Beispiele derartiger Reagenzien, welche die Acylierung auch dadurch fördern, daß sie mit der Säure in situ unter Bildung eines reaktionsfähigen Säurederivates reagieren, seien insbesondere genannt Alkylcarbodiimide, z.B. Cycloalkylcarbodiimide wie Dicyclohexylcarbodiimid oder 1-Ethyl-3-[3-(dimethylamino)-propyl]-carbodiimid, oder Carbonyldiimidazol oder N-C₁-C₄-alkyl-2-halogenpyridiniumsalze, insbesondere Halogenide oder Tosylate, vorzugsweise N-Methyl-2-chlorpyridiniumjodid. Die Umsetzung in Gegenwart eines dehydratisierenden Kopplungsreagenzes kann zweckmäßigerweise bei Temperaturen von -30 °C bis +50 °C unter neutralen Reaktionsbedingungen in Lösungsmitteln wie halogenierten Kohlenwasserstoffen und/oder aromatischen Kohlenwasserstoffen durchgeführt werden.

Die Acylierung solcher Verbindungen der Formel Ie worin R^{1VI} Wasserstoff bedeutet, kann unter an sich zur Acylierung von Aromaten üblichen Bedingungen, beispielsweise den Bedingungen einer Friedel-Crafts-Acylierung, durchgeführt werden. So kann die Acylierung in einem unter den Reaktionsbedingungen inerten organischen, möglichst wasserfreien Lösungsmittel in Gegenwart einer Lewissäure durchgeführt werden. Als Lewissäuren eignen sich insbesondere als Friedel-Crafts-Katalysatoren bekannte Verbindungen wie Aluminiumhalogenide, insbesondere Aluminiumtrichlorid, Zinkhalogenide wie Zinkdichlorid, Zinn- oder Titanhalogenide wie Zinntetrachlorid oder Titantetrachlorid oder auch Borhalogenide wie Bortrichlorid oder Bortrifluorid. Als unter den Reaktionsbedingungen inerte Lösungsmittel eignen sich beispielsweise die vorgenannten aliphatischen halogenierten Kohlenwasserstoffe, z.B. Chloroform, oder auch Schwefelkohlenstoff oder Nitrobenzol. Die Umsetzung kann bei Temperaturen zwischen 0 °C und Siedetemperatur des Lösungsmittels erfolgen.

Die Reaktionszeit für die Acylierung kann je nach dem zu acylierenden Ausgangsprodukt der Formel Ie und den Reaktionsbedingungen zwischen 1 und 15 Stunden betragen. Falls in den Verbindungen der Formel Ie R³, R⁴ und/oder Z freie Hydroxygruppen enthalten, werden diese bei der Acylierung mitacyliert. Die gebildeten Estergruppen können anschließend auf an sich bekannte Weise hydrolytisch wieder gespalten werden. Phenolische Hydroxygruppen in den Resten R³ oder R⁴ können gewünschtenfalls auch durch an sich bekannte Etherschutzgruppen, welche anschließend hydrogenolytisch oder solvolytisch abspaltbar sind, geschützt werden.

In Verbindungen der Formel I, worin R¹ Methoxy bedeutet oder eine Methoxyphenylgruppe enthält und/oder R³ und/oder R⁴ eine Methoxyphenylgruppe darstellen oder enthalten, kann die Methoxygruppe mit zur Spaltung von Methoxyarylethern geeigneten Methoden auf an sich bekannte Weise zur Hydroxygruppe gespalten werden. Beispielsweise kann die Etherspaltung durch Behandeln mit Jodwasserstoff oder Bromwasserstoff in einem unter den Reaktionsbedingungen inerten Lösungsmittel, z.B. Acetanhydrid oder Essigsäure, oder mit Jodtrimethylsilan oder mit Bortribromid in einem halogenierten Kohlenwasserstoff wie Dichlormethan erfolgen.

Aus Verbindungen der Formel I, worin R¹, R³ und/oder R⁴ eine mindestens 2 Kohlenstoffatome enthaltende Hydroxyalkylgruppe enthalten, können durch Wasserabspaltung Verbindungen der Formel I mit einer entsprechenden Alkenylgruppe erhalten werden. Die Wasserabspaltung kann nach an sich zur Dehydratisierung von Alkoholen üblichen Methoden durch Behandeln mit sauren Wasserabspaltungsmitteln erfolgen. Zweckmäßig erfolgt die Wasserabspaltung in einem inerten organischen Lösungsmittel, welches mit Wasser ein leicht abdestillierbares azeotropes Gemisch bildet. So eignen sich beispielsweise aromatische Kohlenwasserstoffe wie Benzol oder Toluol. Zweckmäßig wird die hydroxyalkylhaltige Verbindung der Formel I mit dem Dehydratisierungsmittel bei Siedetemperatur des Lösungsmittels behandelt. Als Dehydratisierungsmittel eignen sich starke anorganische Säuren wie z.B. Schwefelsäure oder starke organische Säuren wie z.B. Benzolsufonsäuren, welche im Benzolring gegebenenfalls durch C₁-C₄ Alkyl oder Halogen substituiert sein können, oder C₁-C₄-aliphatische Halogencarbonsäuren wie Trifluoressigsäure. Sofern die Hydroxyalkylgruppe in den Verbindungen der Formel I eine tertiäre Alkoholgruppe darstellt, können auch weniger stark wirksame Säuren wie z.B. konzentrierte Chlorwasserstoffsäure eingesetzt werden. Die Reaktionstemperatur und die Reaktionszeit können je nach Stärke der für die Wasserabspaltung eingesetzten Säuren variieren. So können die Reaktionszeiten zwischen 1 und 15 Stunden betragen.

In Verbindungen der Formel I, worin nur der Rest R¹ eine Hydroxyalkylgruppe enthält, kann diese gewünschtenfalls zu der entsprechenden Alkanoylgruppe oxidiert werden. Die Oxidation kann nach an sich zur Oxidation von Alkoholen zu Aldehyden oder Ketonen üblichen Methoden durch Behandeln mit einem Oxidationsmittel erfolgen. Als Oxidationsmittel eignen sich beispielsweise anorganische Oxidationsmittel wie z.B. Chrom-VI-Verbindungen, beispielsweise Chromat-Salze oder Pyridiniumchlorochromat, Mangan-IV-Verbindungen oder Permanganate oder auch organische Oxidationsmittel, beispielsweise Dimethylsulfoxidkomplexe wie Dimethylsulfoxid/Oxalylchlorid (= Swern-Reagenz) oder Dimethylsulfoxid/Essigsäureanhydrid oder Dimethylsulfoxid/ Trifluoressigsäureanhydrid. Die Umsetzung kann in unter den Reaktionsbedingungen inerten nicht oxidierbaren Lösungsmitteln, beispielsweise halogenierten Kohlenwasserstoffen, bei Temperaturen zwischen -80 °C und Raumtemperatur durchgeführt werden.

Erhaltene Verbindungen der Formel I, worin R¹ Amino bedeutet, können gewünschtenfalls nachträglich in an sich bekannter Weise zu den entsprechenden N-Mono- oder Di-C₁-C₄-alkylverbindungen alkyliert werden. Als Alkylierungsmittel kommen Alkylhalogenide, insbesondere Jodide, Alkylsulfate oder Alkylsulfonsäureester in Frage. Die Alkylierung kann nach an sich zur Alkylierung von Anilinen üblichen Methoden in einem unter den Reaktionsbedingungen inerten Lösungsmittel unter basischen Bedingungen ausgeführt werden. Sie kann beispielsweise in der für die Umsetzung der Verbindungen der Formel II mit Verbindungen der Formel III beschriebenen Weise erfolgen. Im allgemeinen wird bei der nachträglichen Alkylierung von Verbindungen der Formel I ein Gemisch von mono- und dialkylierten Verbindungen erhalten, worin der Anteil an dialkylierten Verbindungen je nach der eingesetzten Menge an Alkylierungsmittel und den Reaktionsbedingungen variieren kann. Die monoalkylierten und dialkylierten Verbindungen können auf an sich bekannte Weise, beispielsweise durch Chromatographie an Kieselgel, von einander getrennt werden. Die nachträgliche Alkylierung kann auch als reduktive Alkylierung auf an sich bekannte Weise durch Umsetzen mit einem C₁-C₄-Aldehyd, insbesondere Formaldehyd, unter reduzierenden Bedingungen erfolgen. Z.B. können die Verbindungen mit dem Aldehyd in Gegenwart eines Reduktionsmittels, beispielsweise Ameisensäure, umgesetzt werden. Gewünschtenfalls kann die reduktive Alkylierung auch durch Umsetzen der Verbindung mit dem Aldehyd und katalytische Hydrierung des Reaktionsgemisches erfolgen. Als Hydrierungskatalysator eignet sich zum Beispiel Palladium auf Kohle.

Schwefelhaltige Verbindungen der Formel I, z.B. Verbindungen der Formel I, worin A Schwefel bedeutet, können gewünschtenfalls: auf an sich bekannte Weise zu den entsprechenden S-Mono- oder -Dioxiden oxidiert werden. Hierbei werden allfällige Alkylthiogruppen R¹ ebenfalls oxidiert. Als: Oxidationsmittel eignen sich beispielsweise Wasserstoffperoxid in Gegenwart eines- Hydroxygruppen enthaltenden organischen Lösungsmittels, beispielsweise Essigsäure oder Methanol, oder Persäuren, beispielsweise Peressigsäure in einem aromatischen Kohlenwasserstoff wie Benzol, 3-Chlorperbenzoesäure in einem unter den Reaktionsbedingungen inerten aprotischen Lösungsmittel wie einem Halogenkohlenwasserstoff, z.B. Dichlormethan oder Chloroform, oder auch Aceton, oder Natriumperjodid in einem Gemisch aus Aceton und einem C₁-C₄-Alkohol, insbesondere Methanol. Die Reaktionstemperatur kann je nach Art des verwendeten Oxidationsmittels variieren und kann z.B. zwischen -10 °C und 50 °C betragen. Gewünschtenfalls können dem Reaktionsmedium noch weitere unter den Reaktionsbedingungen inerte organische Lösungsmittel, beispielsweise aromatische Kohlenwasserstoffe wie Benzol oder Toluol, zugesetzt werden. Bei der Oxidation wird im allgemeinen ein Gemisch von S-Monooxid- und S-Dioxid-Verbindungen erhalten, worin der Anteil an S-Dioxid-Verbindung je nach der eingesetzten Menge an Oxidationsmittel, der Oxidationstemperatur und der Oxidationsdauer variieren kann. Das S-Monooxid und das S-Dioxid können auf an sich bekannte Weise, beispielsweise durch Chromatographie an Kieselgel, von einander getrennt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren, oder mit organischen Säuren, beispielsweise C₁-C₄-aliphatischen Mono- oder Dicarbonsäuren wie Maleinsäure, Fumarsäure-, Milchsäure, Weinsäure oder Essigsäure, oder Sulfonsäuren, beispielsweise C₁-C₄-alkansulfonsäuren wie Methansulfonsäure oder gegebenenfalls im Benzolring durch Halogen oder C₁-C₄-Alkyl substituierte Benzolsulfonsäuren wie p-Toluolsulfonsäure, oder Cyclohexylaminsulfonsäure.

Sofern R⁴ nicht Wasserstoff bedeutet, enthalten die Verbindungen der Formel I ein Chiralitätszentrum. Weitere Chiralitätszentren können möglicherweise in einzelnen Substituenten, beispielsweise im Falle einer substituierten Alkylenkette Z, enthalten sein. Verbindungen der Formel I, welche ein Chiralitätszentrum enthalten, können in mehreren optisch aktiven enantiomeren Formen oder als Racemat vorliegen. Die vorliegende Erfindung umfaßt sowohl die racemischen Gemische wie auch die reinen optischen Isomeren der Verbindungen der Formel I.

Falls bei der Synthese Racemate der Ausgangsverbindungen der Formeln II, IV oder V eingesetzt werden, werden die Verbindungen der Formel I in Form von Racematen erhalten. Ausgehend von optisch aktiven Formen der Ausgangsverbindungen können optisch aktive Verbindungen der Formel I erhalten werden. Die optisch aktiven Verbindungen der Formel I können aus den racemischen Gemischen in an sich bekannter Weise erhalten werden, z.B. durch chromatographische Trennung an chiralen Trennmaterialien oder durch Umsetzung mit geeigneten optisch aktiven Säuren, beispielsweise Weinsäure oder 10-Camphersulfonsäure, und anschließende Auftrennung in ihre optisch aktiven Antipoden durch fraktionierte Kristallisation der gewonnenen Salze.

Die Ausgangsverbindungen der Formel II sind neue Verbindungen, welche wertvolle Zwischenprodukte zur Herstellung von pharmakologisch wirksamen Verbindungen, beispielsweise den Verbindungen der Formel I, darstellen.

Verbindungen der allgemeinen Formel IIa
(s. Formel IIa)
worin R², R^{3'}, R^{4'}, A und X obige Bedeutung besitzen und R^{1VII} die für R^{1'} angegebene Bedeutung mit Ausnahme von Hydroxy, Amino, Nitro oder CO-haltigen Resten besitzt, und Z^{IV} die für Z angegebene Bedeutung mit Ausnahme von hydroxy-substituierten Ketten besitzt, können auf an sich bekannte Weise aus den entsprechenden Alkoholen der allgemeinen Formel VIII
(s. Formel VIII)
worin R^{1VII}, R², R^{3'}, R^{4'}, A und Z^{IV} obige Bedeutung besitzen, erhalten werden, indem die Hydroxygruppe in an sich bekannter Weise in eine Fluchtgruppe X überführt wird. So können die Verbindungen der Formel VIII beispielsweise zur Einführung eines Halogenrestes X mit Thionylchlorid oder mit Phosphorhalogeniden, z.B. Phosphortribromid, auf an sich bekannte Weise in einem unter den Reaktionsbedingungen inerten Lösungsmittel, z.B. einem halogenierten Kohlenwasserstoff wie Chloroform, umgesetzt werden. Sulfonsäurereste X können auf an sich bekannte Weise eingeführt werden, indem man Verbindungen der Formel VIII mit einem entsprechenden Sulfonsäurechlorid acyliert.

Verbindungen der allgemeinen Formel IIb
(s. Formel IIb)
worin R^{1VII}, R², R^{3'}, R^{4'}, A und X' obige Bedeutung besitzen und Z^{V} eine in der dem Indolgerüst benachbarten Position durch Hydroxy substituierte, gegebenenfalls durch C₁-C₄-Alkyl substituierte Alkylenkette mit 2 - 4 Kohlenstoffatomen bedeutet, können auf an sich bekannte Weise ausgehend von Verbindungen der allgemeinen Formel IXa
(s. Formel IXa)
worin R^{1VII}, R², R^{3'}, R^{4'} und A obige Bedeutung besitzen, erhalten werden, indem man Verbindungen der Formel IXa mit einem Halogencarbonsäurederivat der allgemeinen Formel XXVI
(s. Formel XXVI)
worin X' obige Bedeutung besitzt, Q" eine in der dem Rest Y' benachbarten Position durch Oxo substituierte Alkylenkette mit 1 - 3 Kohlenstoffatomen, welche gegebenenfalls durch C₁-C₄-Alkyl substituiert sein kann, bedeutet und Y' Halogen oder den Rest X'-Q"-O, worin X' und Q" obige Bedeutung besitzen, darstellt, in einem unter den Reaktionsbedingungen inerten Lösungsmittel in Gegenwart eines Friedel-Crafts-Katalysators wie Aluminiumtrichlorid auf an sich bekannte Weise umsetzt zu Verbindungen der allgemeinen Formel XXVIIa
(s. Formel XXVIIa)
worin R^{1VII}, R², R^{3'}, R^{4'}, A und X obige Bedeutung besitzen und Q" eine in der dem Indolgerüst benachbarten Position durch Oxo substituierte Alkylenkette mit 1 - 3 Kohlenstoffatomen, welche gegebenenfalls durch C₁-C₄-Alkyl substituiert sein kann, bedeutet, und diese anschließend mit einem den Halogensubstituenten X' nicht angreifenden Reduktionsmittel, z.B. Natriumborhydrid, auf an sich bekannte Weise reduziert.

Die Umsetzung von Verbindungen der Formel IXa mit Verbindungen der Formel XXVI kann unter an sich für Acylierungen von Aromaten nach der Methode von Friedel Crafts üblichen Bedingungen erfolgen.

Sofern Verbindungen hergestellt werden, worin Q" für die -CO-CH₂-Gruppe steht, können die Verbindungen der Formel IXa auch zunächst in einer Friedel-Crafts-Reaktion mit Acetanhydrid acyliert werden, und die erhaltenen Acetylderivate in an sich bekannter Weise zu entsprechenden Verbindungen der Formel XXVIIa halogeniert, z.B. bromiert, werden.

In Verbindungen der Formeln IIa und IIb, worin R^{1VII} Methoxy bedeutet oder eine Methoxyphenylgruppe enthält und/oder R^{3'} und/oder R^{4'} eine Methoxyphenylgruppe darstellen oder enthalten, kann die Methoxygruppe gewünschtenfalls auf an sich bekannte Weise zur Hydroxygruppe gespalten werden. Die Spaltung kann unter zur Spaltung von Phenolethern üblichen Bedingungen, beispielsweise unter den vorstehend zur Freisetzung einer Hydroxygruppe aus einer Methoxygruppe R¹ in den Verbindungen der Formel I angegebenen Bedingungen, erfolgen.

Gewünschtenfalls kann in die Verbindungen der Formel II ein Chlor- oder Brom-Substituent R^{1'} oder R² auf an sich bekannte Weise, z.B. durch Behandeln der Verbindungen mit elementarem Chlor oder Brom in Eisessig, eingeführt werden. Gewünschtenfalls kann auch ein Nitro-Substituent R^{1'} oder R² auf an sich bekannte Weise, z.B. durch Behandeln mit einem Salpetersäure/ Schwefelsäure-Gemisch, eingeführt werden.

In Verbindungen der Formel II, worin R Nitro bedeutet und X einen nicht reduzierbaren Rest, vorzugsweise Tosyloxy bedeutet, kann die Nitrogruppe auf an sich bekannte Weise zur Aminogruppe reduziert werden. Die Reduktion kann mit zur Reduktion von Nitrogruppen zu Aminogruppen üblichen Reduktionsmitteln erfolgen, beispielsweise durch katalytische Hydrierung in Gegenwart eines Palladium/Kohle Katalysators in einem C₁-C₄-Alkohol oder durch Reduktion mittels Natriumborhydrid in Gegenwart eines Palladium/Kohle Katalysators in einem Ether wie Tetrahydrofuran.

Verbindungen der Formel II, worin R^{1'} Wasserstoff, Hydroxy oder Amino bedeutet, können auf an sich bekannte Weise durch Acylierung mit Verbindungen der Formel VII in solche Verbindungen der Formel II überführt werden, worin R^{1'} die für R^{1V} in den Verbindungen der Formel Id angegebene Bedeutung besitzt. Die Acylierung kann nach an sich bekannten Methoden erfolgen und beispielsweise unter den für die Acylierung von Verbindungen der Formel Ie gemäß Verfahrensvariante d) angegebenen Bedingungen durchgeführt werden.

Alkohole der Formel VIII sind neue Verbindungen, welche wertvolle Zwischenprodukte zur Herstellung von pharmakologisch aktiven Verbindungen, z.B. den Verbindungen der Formel I, darstellen. Sie können auf an sich bekannte Weise durch Reduktion von Estern der allgemeinen Formel Xb
(s. Formel Xb)
worin R^{1VII}, R², R^{3'}, R^{4'} und A obige Bedeutung besitzen, Z^{VI} für eine gegebenenfalls durch C₁-C₄-Alkyl substituierte Alkylenkette mit 1 - 3 Kohlenstoffatomen steht und R^{9'} C₁-C₄ Alkyl bedeutet, erhalten werden. Als Reduktionsmittel eignen sich beispielsweise zur Reduktion von Estern befähigte Hydridreduktionsmittel, beispielsweise die vorstehend unter Verfahrensvariante b) zur Reduktion von Verbindungen der Formel IV angegebenen Reduktionsmittel, insbesondere Lithiumaluminiumhydrid oder Diboran. Die Reduktion kann nach üblichen Methoden, beispielsweise bei den für die Reduktion von Verbindungen der Formel IV gemäß Verfahrensvariante b) angegebenen Reaktionsbedingungen, durchgeführt werden. Als zweckmäßig erweist sich insbesondere die Reduktion mit Lithiumaluminiumhydrid in Tetrahydrofuran.

Verbindungen der allgemeinen Formel X
(s. Formel X)
worin R^{1III}, R², R⁶, R⁷, A und Q' obige Bedeutung besitzen, und R⁹ Wasserstoff oder C₁-C₄-Alkyl bedeutet, können auf an sich bekannte Weise erhalten werden. So können Verbindungen der allgemeinen Formel Xa
(s. Formel Xa)
worin R^{1VII}, R², R⁶, R⁷, A, Z^{VI} und R⁹ obige Bedeutung besitzen, erhalten werden, indem man Hydrazinverbindungen der allgemeiner Formel XIa
(s. Formel XIa)
worin R^{1VII}, R², R⁷, und A obige Bedeutung besitzen, mit Verbindungen der allgemeinen Formel XII
(s. Formel XII)
worin R⁹ und Z^{VI} obige Bedeutung besitzen und R¹⁰ die für R⁶ angegebene Bedeutung mit Ausnahme von Wasserstoff besitzt oder eine Carboxylgruppe, darstellt, auf an sich bekannte Weise, beispielsweise unter den Bedingungen der Fischer'schen Indolsynthese, umsetzt, wobei intermediär Hydrazon-Verbindungen der allgemeinen Formel XIII
(s. Formel XIII)
worin R^{1VII}, R², R⁷, A, Z^{VI}, R⁹ und R¹⁰ obige Bedeutung besitzen, gebildet werden, welche weiter zu den Verbindungen der Formel Xa kondensieren. Hierbei wird eine allfällige Carboxylgruppe R¹⁰ abgespalten, so daß bei Verwendung von Verbindungen der Formel XII, worin R¹⁰ eine Carboxylgruppe darstellt, Verbindungen der Formel Xa erhalten werden, worin R⁶ Wasserstoff bedeutet. Die Umsetzung kann durch Erhitzen auf Temperaturen zwischen 50 und 100 °C in saurem Medium, z.B. in einem mit Wasser mischbaren säurehaltigen, z.B. mit wässriger Salzsäure, Schwefelsäure oder Phosphorsäure angesäuerten, organischen Lösungsmittel wie einem C₁-C₄-Alkohol oder Essigsäure, erfolgen oder in neutralem Medium in Gegenwart von Zeolith durchgeführt werden. Sofern bei der Umsetzung Ester der Formel XII eingesetzt werden, können Gemische von Estern und Säuren der Formel Xa erhalten werden, in denen das Verhältnis von Ester zu Säure je nach Art der Reaktionsbedingungen variieren kann, und aus welchen die Ester und/oder die Säuren durch chromatographische Reinigung isoliert werden können.

Ester der Formel Xa können gewünschtenfalls zu den entsprechenden Säuren der Formel Xa hydrolysiert werden und die Säuren oder Säuren und Ester enthaltende Gemische können auf an sich bekannte Weise durch Umsetzung mit C₁-C₄-Alkoholen in die entsprechenden Ester überführt werden. Bei einer Esterhydrolyse reagiert die Z^{VI}-COO-R^{9'}-Estergruppierung bevorzugt vor einer allfälligen direkt an das Ringgerüst gebundenen Alkoxycarbonylgruppe R⁶.

In Verbindungen der Formel X können gewünschtenfalls weitere Substituenten R^{1III} eingeführt werden. So können beispielsweise auf an sich bekannte Weise, z.B. wie vorstehend für die Verbindungen der Formel II beschrieben, die Nitrogruppe, Halogen oder ein Acylrest R^{1III} eingeführt werden, oder eine Methoxygruppe R^{1III} kann zur Hydroxygruppe gespalten werden. Ein Acylrest R^{1III} kann gewünschtenfalls auf an sich bekannte Weise zu dem entsprechenden Alkylrest reduziert werden. Hierzu eignet sich beispielsweise die Reduktion mit Hydrazin in Gegenwart einer anorganischen Base, z.B. einem Alkalimetallhydroxid, in einem höhersiedenden Ether. Eine allfällige Nitrogruppe R^{1III} kann gewünschtenfalls zur Aminogruppe reduziert werden. Die Reduktion kann auf an sich bekannte Weise unter Verwendung eines Reduktionsmittels, welches fähig ist, selektiv die Nitrogruppe zur Aminogrupe zu reduzieren ohne die Alkoxycarbonylfunktion reduktiv anzugreifen, erfolgen, z.B. durch katalytische Hydrierung. Eine Aminogruppe R^{1III} kann gewünschtenfalls auf an sich bekannte Weise acyliert werden. In Estern der Formel Xa kann eine Aminogruppe R^{1III} gewünschtenfalls zur Di-C₁-C₄-alkylaminogruppe alkyliert werden. Die Alkylierung kann auf an sich bekannte Weise, z.B. wie vorstehend für die Verbindungen der Formel I beschrieben, erfolgen.

Verbindungen der allgemeinen Formel XI
(s. Formel XI)
worin R², A und R⁷ obige Bedeutung besitzen und R^{1VIII} die für R^{1'} angegebene Bedeutung mit Ausnahme von Amino, Nitro und von die -COO-Gruppe oder die -CONH-Gruppe enthaltenden Resten besitzt, können auf an sich bekannte Weise ausgehend von Verbindungen der allgemeinen Formel XIV
(s. Formel XIV)
worin R^{1VIII}, R², R⁷ und A obige Bedeutung besitzen, erhalten werden. Hierzu werden die Verbindungen der Formel XIV durch Behandeln mit Natriumnitrit auf an sich bekannte Weise in die entsprechenden N-Nitroso-Verbindungen der Formel XV
(s. Formel XV)
worin R^{1VIII}, R², R⁷ und A obige Bedeutung besitzen, überführt und diese anschließend zu den Hydrazinverbindungen der Formel XI reduziert. Für die Reduktion der Nitrosoverbindungen der Formel XV können alle an sich zur Reduktion von Nitrosoverbindungen zu entsprechenden Hydrazinverbindungen bekannten Reduktionsmethoden eingesetzt werden. Als Reduktionsmittel eignen sich beispielsweise Lithiumaluminiumhydrid in Tetrahydrofuran oder metallisches Zinkpulver in Gegenwart von Säure oder Natriumdithionit. Auch eine katalytische Hydrierung der Nitrosoverbindungen zu den Hydrazinen der Formel XI ist möglich. Sofern der Substituent R⁷ eine C₁-C₄-alkoxycarbonylFunktion enthält, kann diese bei der Reduktion gegebenenfalls, beispielsweise bei der Verwendung von Lithiumaluminiumhydrid als Reduktionsmittel, ebenfalls reduziert werden zu der entsprechenden Hydroxymethyl-Funktion. Sofern R^{1VIII} eine Carbonylgruppe enthält, müssen solche Reduktionsmittel gewählt werden, welche diese Carbonylgruppe nicht angreifen.

Vorteilhaft kann die Herstellung von Estern der Formel Xa ausgehend von den Verbindungen der Formel XIV in einem Eintopf-Verfahren, ohne die einzelnen Zwischenstufen zu isolieren, durchgeführt werden. Hierbei wird zur Reduktion der N-Nitrosoverbindung metallisches Zinkpulver in Gegenwart von Säure eingesetzt und das nach der Reduktion erhaltene, die Hydrazinverbindung der Formel XI enthaltende, zinksalzhaltige Reaktionsgemisch durch Zugabe von Salzsäure weiter angesäuert, und dann wird der Reaktionsmischung ein Ester der Formel XII zugegeben. Bei Zugabe des Esters der Formel XII zu dem Reaktionsgemisch entsteht intermediär die Hydrazonverbindung der Formel XIII, welche unter den Reaktionsbedingungen weiter zu dem Ester der Formel Xa kondensiert.

Verbindungen der allgemeinen Formel Xc
(s. Formel Xc)
worin R^{1VII}, R², R^{3'}, R^{4'}, R⁹, A und Z"' obige Bedeutung besitzen, können ausgehend von Verbindungen der allgemeinen Formel IIc
(s. Formel IIc)
worin R^{1VII}, R², R^{3'}, R^{4'}, A, Z"' und X obige Bedeutung besitzen, erhalten werden, indem man die Verbindungen der Formel IIc zunächst durch Umsetzen mit Natriumcyanid auf an sich bekannte Weise in ein entsprechendes Nitril überführt und dieses anschließend zur Säure der Formel Xc hydrolysiert bzw. durch Solvolyse in einem C₁-C₄-Alkohol und anschließende Hydrolyse des intermediar gebildeten Iminoethers in einen Ester der Formel Xc überführt. Die Umsetzung der Verbindung der Formel IIc mit Natriumcyanid kann durch Behandeln einer Lösung einer Verbindung der Formel IIc in einem organischen Lösungsmittel mit wäßriger Natriumcyanidlösung erfolgen. Die Hydrolyse oder Solvolyse des gebildeten Nitrils wird zweckmäßig in saurem Medium durchgeführt.

Den Säuren der Formel Xd
(s. Formel Xd)
worin R^{1VIII}, R², R⁶, R⁷, Q" und A obige Bedeutung besitzen, entsprechende Säurehalogenide oder Salze können ausgehend von Verbindungen der allgemeinen Formel IX
(s. Formel IX)
worin R^{1VIII}, R², R⁶, R⁷ und A obige Bedeutung besitzen, erhalten werden, indem man Verbindungen der Formel IX in an sich bekannter Weise mit Dicarbonsäurehalogeniden der allgemeinen Formel XXIX
(s. Formel XXIX)
worin X', Q" und Y' obige Bedeutung besitzen, unter den Bedingungen einer Friedel-Crafts-Acylierung umsetzt. Die erhaltenen Halogenide der Säuren der Formel Xd können sofort in situ weiterverarbeitet werden oder auf an sich bekannte Weise in Salze der Säuren Xd überführt werden.

Verbindungen der Formel IX können auf an sich bekannte Weise hergestellt werden. So können die Hydrazin-Verbindungen der Formel XI mit Verbindungen der allgemeinen Formel XVI
(s. Formel XVI)
worin R⁶ obige Bedeutung besitzt, auf an sich bekannte Weise, z.B. unter den Bedingungen der Fischer'schen Indolsynthese, umgesetzt werden, wobei intermediär ein Hydrazon gebildet wird, welches weiter zu den Verbindungen der Formel IX kondensiert. Die Umsetzung kann beispielsweise unter den zur Umsetzung von Verbindungen der Formel XI mit Verbindungen der Formel XII angegebenen Reaktionsbedingungen durchgeführt werden. In Verbindungen der Formel IX können gewünschtenfalls Halogensubstituenten R^{1VIII} nachträglich eingeführt werden, beispielsweise in der vorstehend für Verbindungen der Formel II beschriebenen Weise. Allfällige Acylreste R⁶ können auf an sich bekannte Weise zu entsprechenden Alkylresten reduziert werden, beispielsweise mittels Hydrazin unter den vorstehend für Verbindungen der Formel Xa angegebenen Bedingungen.

Verbindungen der Formel XIV sind bekannt oder können nach an sich bekannten Methoden oder analog zu an sich bekannten Methoden hergestellt werden. Beispielsweise können Verbindungen der allgemeinen Formel XIVa
(s. Formel XIVa)
worin R^{1VIII}, R² und R⁷ obige Bedeutung besitzen und A' eine gegebenenfalls durch C₁-C₄-Alkyl substituierte Methylengruppe bedeutet, auf an sich bekannte Weise durch Reduktion von Verbindungen der allgemeinen Formel XVII oder Verbindungen der allgemeinen Formel XVIII
(s. Formeln XVII und XVIII)
worin R^{1VIII}, R² und R⁷ obige Bedeutung besitzen, A" eine gegebenenfalls durch C₁-C₄-Alkyl substituierte CH-Gruppe bedeutet und R^{4"} die für R⁴ angegebene Bedeutung mit Ausnahme von freie Hydroxygruppen enthaltenden Resten besitzt, erhalten werden. So können die Tetrahydrochinolinderivate der Formel XVII beispielsweise durch Behandeln mit metallischem Natrium in einem C₁-C₄-Alkohol vorzugsweise bei Siedetemperatur des Reaktionsgemisches reduziert werden. Die Reduktion der Chinolinderivate der Formel XVIII kann beispielsweise durch Reduktion mit Natriumcyanoborhydrid in saurem Medium, z.B. in Essigsäure oder einem Alkohol/Salzsäuregemisch, oder mit einem Diboran/Pyridin-Komplex in Essigsäure erfolgen.

Tetrahydrochinolinderivate der Formel XVII können beispielsweise erhalten werden, indem man Chinolinderivate der Formel XVIII, worin R⁷ Wasserstoff bedeutet auf an sich bekannte Weise mit einer lithiumorganischen Verbindung der allgemeinen Formel XIX
(s. Formel XIX)
worin R^{4"} obige Bedeutung besitzt, umsetzt. Die Umsetzung kann beispielsweise in einem Ether wie Tetrahydrofuran bei Temperaturen von 0°C bis Raumtemperatur erfolgen.

Die Chinolin-Derivate der Formel XVIII sind bekannt oder können nach an sich bekannten Methoden oder analog zu an sich bekannten Methoden hergestellt werden. Beispielsweise kann in Verbindungen der Formel XVIII, worin R⁷ für eine Methylgruppe steht, diese Methylgruppe auf an sich bekannte Weise in einen anderen Rest R⁷ überführt werden, z.B. durch Umsetzen mit einer Verbindung der allgemeinen Formel XX
(s. Formel XX)
worin X' obige Bedeutung besitzt und R^{4"'} einem solchen um eine CH₂-Gruppe verminderten Rest R⁴, welcher keine freie Hydroxygruppe enthält, entspricht. Eine solche Umsetzung kann beispielsweise in Gegenwart einer starken zur Deprotonierung der Methylgruppe befähigten Base wie Butyllithium in einem unter den Reaktionsbedingungen inerten Lösungsmittel, z.B. einem Ether, bei Temperaturen zwischen 0 °C und Raumtemperatur erfolgen.

Verbindungen der allgemeinen Formel XIVb
(s. Formel XIVb)
worin R^{1VII} und R² obige Bedeutung besitzen und A''' für Sauerstoff oder Schwefel steht, können durch Reduktion von Verbindungen der allgemeinen Formel XXI
(s. Formel XXI)
worin R^{1VII}, R² und A"' obige Bedeutung besitzen, erhalten werden. Die Reduktion kann beispielsweise mit Lithiumaluminiumhydrid in einem cyclischen Ether wie Tetrahydrofuran bei erhöhter Temperatur, vorzugsweise Siedetemperatur des Reaktionsgemisches, erfolgen.

Verbindungen der Formel XXI können aus entsprechenden Aminophenolen bzw. Aminothiophenolen der allgemeinen Formel XXII
(s. Formel XXII)
worin R^{1VII}, R² und A''' obige Bedeutung besitzen, auf an sich bekannte Weise durch Umsetzen mit Chloressigsäurechlorid erhalten werden.

Verbindungen der allgemeinen Formel XIVc
(s. Formel XIVc)
worin A''', R^{1VIII}, R² und R⁴ obige Bedeutung besitzen, können erhalten werden, indem man Nitrophenole oder Nitrothiophenole der allgemeinen Formel XXX
(s. Formel XXX)
worin R^{1VIII}, R² und A''' obige Bedeutung besitzen, mit Verbindungen der allgemeinen Formel XXXI
(s. Formel XXXI)
worin R⁴ und X' obige Bedeutung besitzen, umsetzt zu Verbindungen der allgemeinen Formel XXXII
(s. Formel XXXII)
worin R^{1VIII}, R², A"' und R⁴ obige Bedeutung besitzen und diese anschließend unter reduzierenden Bedingungen cylisiert. Die Umsetzung von Verbindungen der Formel XXX mit Verbindungen der Formel XXXI kann auf an sich bekannte Weise unter zur Phenoletherbildung üblichen Bedingungen erfolgen. Die reduktive Cyclisierung von Verbindungen der Formel XXXII kann auf an sich bekannte Weise erfolgen, indem die Verbindungen der Formel XXXII mit einem Reduktionsmittel in einem unter den Reaktionsbedingungen inerten Lösungsmittel behandelt werden. Als Reduktionsmittel eignen sich z.B. Wasserstoff in Gegenwart eines Hydrierungskatalysators, insbesondere Palladium/Kohle oder auch Hydrazin in Gegenwart von Raney-Nickel. Nach erfolgter Reduktion wird die Cyclisierung durch Erhitzen des Reduktionsproduktes auf Temperaturen von 20 bis 100 °C vervollständigt. Sofern R¹ und/oder R⁴ gegebenenfalls substituierte Benzylreste darstellen, können diese bei einer katalytischen Hydrierung mit abgespalten werden und allfällige Alkenylreste können ebenfalls hydriert werden.

Verbindungen der allgemeinen Formel XIVd
(s. Formel XIVd)
worin R^{1IX} die für R^{1VII} angegebene Bedeutung mit Ausnahme von Cyano besitzt und R² obige Bedeutung besitzt und AN eine gegebenenfalls durch C₁-C₄-Alkyl substituierte Ethylengruppe bedeutet, können ausgehend von Verbindungen der allgemeinen Formel XXVIII
(s. Formel XXVIII)
worin R^{1IX}, R² und A' obige Bedeutung besitzen, auf an sich bekannte Weise erhalten werden, indem man Verbindungen der Formel XXVIII zunächst mit Hydroxylamin zu den entsprechenden Oximverbindungen der allgemeinen Formel XXXV
(s. Formel XXXV)
worin R^{1IX}, R² und A' obige Bedeutung besitzen, umsetzt und diese anschließend einer reduktiven Umlagerung zu den Verbindungen der Formel XIVd unterwirft. Die Umlagerung kann auf an sich bekannte Weise unter Verwendung von Diisobutylaluminiumhydrid (="DIBAH") in einem unter den Reaktionsbedingungen inerten Lösungsmittel erfolgen.

In Verbindungen der allgemeinen Formel XIVe
(s. Formel XIVe)
worin R^{1VIII}, R² und A obige Bedeutung besitzen, können weitere Reste R⁷ auf an sich bekannte Weise, z.B. nach der von A. I. Meyers und S. Hellring in Tetrahedron Letters, 22 Seiten 5119 bis 5122 (1981) und von A. I. Meyers in Lectures in Heterocyclic Chemistry, Band VII, Seiten 75 bis 81 (1984) beschriebenen Methode eingeführt werden, indem man die Verbindungen der Formel XIVe zunächst in ihre Formamidin-Derivate der allgemeinen Formel XXXIII
(s. Formel XXXIII)
worin R^{1VIII}, R² und A obige Bedeutung besitzen und welche durch den elektronenziehenden Substituenten für eine Deprotonierung an dem dem Stickstoffatom benachbarten Kohlenstoffatom aktiviert sind, überführt. Die Verbindungen der Formel XXXIII können dann durch Umsetzen mit einer starken Base wie tert. Butyllithium in das entsprechende Anion überführt und mit Verbindungen der allgemeinen Formel XXXIV
(s. Formel XXXIV)
worin X' obige Bedeutung besitzt und R^{7'} die für R⁷ angegebene Bedeutung mit Ausnahme von Wasserstoff besitzt, umgesetzt werden, und die Formamidingruppe kann anschließend aus dem Reaktionsprodukt unter alkalischen oder sauren Bedingungen wieder abgespalten werden.

Verbindungen der Formel III sind bekannt oder können nach an sich bekannten Methoden oder analog zu an sich bekannten Methoden hergestellt werden.

Verbindungen der allgemeinen Formel IIIa
(s. Formel IIIa)
worin R^{5"} einen gegebenenfalls durch C₁-C₄-Alkyl, C₁-C₄-Alkoxy oder Halogen substituierten Phenylrest bedeutet, können beispielsweise ausgehend von 4-Piperidincarbonsäure erhalten werden, indem man die Säure nach Einführung einer Aminoschutzgruppe in ein Säurehalogenid überführt und dieses auf an sich bekannte Weise mit einer Verbindung der allgemeinen Formel XXIII
(s. Formel XXIII)
worin R^{5"} obige Bedeutung besitzt, umsetzt und anschließend die Aminoschutzgruppe wieder abspaltet. Die Umsetzung kann unter an sich zur Acylierung von Aromaten üblichen Bedingungen, beispielsweise den Bedingungen einer Friedel-Crafts-Acylierung in Gegenwart von Aluminiumtrichlorid, durchgeführt werden.

Verbindungen der Formel IV sind neue Verbindungen, welche wertvolle Zwischenprodukte zur Herstellung von pharmakologisch aktiven Verbindungen, beispielsweise den Verbindungen der Formel I darstellen. Sie können nach an sich bekannten Methoden hergestellt werden.

Verbindungen der allgemeinen Formel IVa
(s. Formel IVa)
worin R^{1III}, R², R⁶, R⁷, A, Q', B und R⁵ obige Bedeutung besitzen, können beispielsweise erhalten werden, indem man Säuren der Formel X oder deren reaktionsfähige Säurederivate mit einem Amin der allgemeinen Formel IIIb
(s. Formel IIIb)
worin B und R⁵ obige Bedeutung besitzen, umsetzt. Die Umsetzung kann nach an sich zur Bildung von Amidgruppierungen durch Aminoacylierung üblichen Methoden ausgeführt werden. So können Säuren oder reaktionsfähige Säurederivate der allgemeinen Formel X'
(s. Formel X')
worin R^{1III}, R², R⁶, R⁷, A, Q' und Y obige Bedeutung besitzen, eingesetzt werden. Die Umsetzung kann unter für Aminoacylierungen üblichen Bedingungen, beispielsweise bei den für die Acylierung von Aminoverbindungen der Formel Ie angegebenen Bedingungen, erfolgen. Zweckmäßig wird z.B. eine Säure in Gegenwart eines Kopplungsreagenzes wie Carbonyldiimidazol mit dem Piperazinderivat in einem halogenierten Kohlenwasserstoff wie Dichlormethan umgesetzt.

In Verbindungen der Formel IV können gewünschtenfalls weitere Substituenten R^{1III} eingeführt werden. So können beispielsweise auf an sich bekannte Weise wie vorstehend für die Verbindungen der Formel II beschrieben die Nitrogruppe, Halogen oder ein Acylrest R^{1III} eingeführt werden.

In Verbindungen der Formel IV, worin R^{1III} eine Nitrogruppe bedeutet, kann diese gewünschtenfalls auf an sich bekannte Weise zur Aminogruppe reduziert werden. Die Reduktion kann beispielsweise in der zur Reduktion einer Nitrogruppe in den Verbindungen der Formeln IIa und IIb oder X angegebenen Weise erfolgen. Eine Aminogruppe R^{1III} kann gewünschtenfalls auf an sich bekannte Weise acyliert oder zur Di-C₁-C₄-alkylgruppe alkyliert werden. Die Alkylierung kann auf an sich bekannte Weise, z.B. wie vorstehend für die Verbindungen der Formel I beschrieben, erfolgen.

Verbindungen der allgemeinen Formel IVb
(s. Formel IVb)
worin R^{1III}, R², R⁶, R⁷, A, Z', B und R⁵ obige Bedeutung besitzen, umfassen Verbindungen der Formel I, worin R¹ eine C₁-C₄-Alkanoylgruppe, eine C₁-C₄-Alkanoylaminogruppe oder eine Benzoyl-_oder Cinnamoylgruppe, deren Phenylring gegebenenfalls durch C₁-C₄-Alkyl, C₁-C₄-Alkoxy oder Halogen substituiert sein kann, bedeutet. Verbindungen der Formel IVb, worin R⁶ und/oder R⁷ eine C₁-C₄-alkoxycarbonylgruppe enthalten, können durch Einführung eines C₁-C₄-alkoxycarbonylgruppenhaltigen Restes in die Reste R³ und/oder R⁴ entsprechender Verbindungen der Formel I erhalten werden. So können beispielsweise Verbindungen der Formel I, worin R³ und/oder R⁴ eine im Phenylring durch Hydroxy substituierte Phenyl- oder Phenyl-C₁-C₄-alkylgruppe bedeuten, mit einem C₁-C₄-alkylester einer C₁-C₄-Halogencarbonsäure umgesetzt werden, um die Hydroxygruppe in eine C₁-C₄-alkoxycarbonylalkoxygruppe zu überführen. Die Umsetzung kann unter zur Phenoletherbildung üblichen Bedingungen, beispielsweise in Gegenwart einer starken Base wie Natriumhydrid in einem unter den Reaktionsbedingungen inerten Lösungsmittel, z.B. Dimethylformamid, erfolgen. Verbindungen der Formel IVb, worin R^{1III} eine N-formylsubstituierte Amino-oder C₁-C₄-alkylaminogruppe bedeutet, können aus entsprechenden Verbindungen der Formel I, worin R¹ eine Amino- oder C₁-C₄-alkylaminogruppe bedeutet, erhalten werden, indem diese auf an sich bekannte Weise formyliert werden.

Verbindungen der allgemeinen Formel IVc
(s. Formel IVc)
worin R^{1VIII}, R², R⁵, R⁶, R⁷, A, B und Q'' obige Bedeutung besitzen, können erhalten werden, indem man Verbindungen der allgemeinen Formel XXVII
(s. Formel XXVII)
worin R^{1VIII}, R², R⁶, R⁷, A, Q'' und X' obige Bedeutung besitzen, mit Verbindungen der Formel IIIb umsetzt.

Verbindungen der Formel XXVII können erhalten werden, indem man Verbindungen der Formel IX in einer Friedel-Crafts-Reaktion mit Verbindungen der Formel XXVI acyliert.

Verbindungen der allgemeinen Formel V sind neue Verbindungen, welche wertvolle Zwischenprodukte zur Herstellung von pharmakologisch aktiven Verbindungen, beispielsweise den Verbindungen der Formel I darstellen. Sie können erhalten werden, indem man aus Verbindungen der allgemeinen Formel XXIV
(s. Formel XXIV)
worin R^{1IV}, R², R^{3'}, R^{4'}, Z und A obige Bedeutung besitzen und R¹¹ eine Aminoschutzgruppe bedeutet, die Schutzgruppe auf an sich bekannte Weise abspaltet. Als Aminoschutzgruppen R¹¹ eignen sich an sich bekannte hydrolytisch oder hydrogenolytisch abspaltbare Schutzgruppen wie beispielsweise C₁-C₄-Alkanoyl, Formyl oder Benzyl. Verbindungen der Formel XXIV können analog den zur Herstellung von Verbindungen der Formel I gemäß Verfahren a) oder b) beschriebenen Methoden erhalten werden, indem man Verbindungen der allgemeinen Formel XXV
(s. Formel XXV)
worin R¹¹ obige Bedeutung besitzt, mit Verbindungen der Formel II umsetzt oder mit Säuren der Formel X oder deren reaktionsfähigen Säurederivaten umsetzt und das Reaktionsprodukt reduziert. Die Umsetzung von Verbindungen der Formel II mit den Verbindungen der Formel XXV kann nach an sich zur Aminoalkylierung üblichen Methoden erfolgen und kann beispielsweise unter den für die Umsetzung von Verbindungen der Formel II mit Verbindungen der Formel III angegebenen Reaktionsbedingungen durchgeführt werden. Sofern R^{1IV} eine mit einer Schutzgruppe zu versehende Aminogruppe darstellt, wird als Schutzgruppe R¹¹ eine andersartige Schutzgruppe gewählt, welche sich unter Bedingungen abspalten läßt, unter denen die Schutzgruppe des Restes R^{1IV} erhalten bleibt.

Die Verbindungen der Formel I und ihre pharmakologisch akzeptablen Säureadditionssalze zeichnen sich durch interessante pharmakologische Eigenschaften aus und besitzen antiinflammatorische und antiallergische Wirkungen. Insbesondere zeigen die Verbindungen ein zur Behandlung von asthmatischen Beschwerden günstiges Wirkungsprofil mit geringer Toxizität und guter Verträglichkeit.

Asthma ist ein chronisches inflammatorisches Lungenleiden, welches durch episodisch auftretende reversible Behinderungen der Atemwege charakterisiert ist. Es wird allgemein angenommen, daß die Auslösung von asthmatischen Beschwerden und Anfällen von einem als Mastzelle bekannten parenchymalen und interstitialen Zelltyp ausgeht. Diese Mastzellen enthalten vorgebildete Entzündungsmediatoren und Spasmogene, insbesondere Histamin. Sie sind auch befähigt, eine Reihe von von Membranlipiden abstammenden Mediatoren neu zu synthetisieren. Mastzellen wirken auch im Verein mit einer Vielzahl von Begleitzellen, welche alle fähig sind, inflammatorische und pro-inflammatorische Mediatoren zu synthetisieren.

Solange keine allergieauslösenden Bedingungen vorliegen, befinden sich die Mastzellen in quasi unbeteiligter Wartestellung. Der Schlüssel zu allergischen Reaktionen liegt in der Anwesenheit hoher Konzentrationen von zirkulierenden IgE-Antikörpern. Wenn diese Antikörper an ein entsprechendes Antigen gebunden werden, aktivieren sie beides, sowohl die Degranulation und Freisetzung der vorgeformten Mediatoren als auch die Neusynthese anderer Mediatoren.

Da Asthma ein inflammatorisches obstruktives Lungenleiden ist, stützt sich die Therapie auf im wesentlichen zwei Wege: Erleichterung der symptomatischen Beschwerden durch Verabreichung von Bronchodilatoren wie β-Sympathicomimetica, Xanthin-Derivate und Anticholinergica; Verabreichung von antiinflammatorischen Wirkstoffen wie Dinatriumcromoglicinat und Steroiden; und gegen spezifische Mediatoren wie z. B. Histamin zielgerichtete Therapien. Eine Behandlung zur Linderung der symptomatischen Beschwerden ist bei etwa 50 % der Asthmatiker angemessen, trägt jedoch nichts dazu bei, die Ursachen, das ist die Entzündung, zu lindern. Antiinflammatorische Wirkstoffe können die Entzündung eindämmen, besitzen jedoch oft unerwünschte Nebenwirkungen und werden oft gleichzeitig mit Bronchodilatoren verabreicht. Auf einen spezifischen Mediator zielgerichtete Therapien sind allein völlig unzureichend, da es eine Vielzahl von Mediatoren gibt.

Die erfindungsgemäßen Substanzen zeichnen sich dadurch aus, daß sie antiinflammatorisch wirksam sind und zielgerichtet gegen einen oder mehrere der drei Mediatortypen Histamin, Leukotriene und Blutplättchenaggregationsfaktor, welche nicht nur bei akuten Bronchospasmen sondern auch bei der Aufrechterhaltung der chronischen Entzündung beteiligt sind, wirken oder auch über mediatorenspezifische Rezeptoren gegen die betreffenden Targetzellen wirksam sind.

Die antiinflammatorischen und antiallgergischen Eigenschaften der Verbindungen lassen sich in pharmakologischen Standardtestmethoden in vitro und in vivo nachweisen.

### Beschreibung der Testmethoden

### 1. Bestimmung der Hemmung der passiven cutanen Anaphylaxie (P.C.A.) und der durch Histamin induzierten anaphylaktoiden cutanen Reaktion.

Die P.C.A.-Reaktion wird nach den von Goose et al (J.N. Immunology 16 (1969), 749) und von Martin et al (Arch. Pharmacol. 316 (1981), 186) beschriebenen Methoden durchgeführt.

Das in dem Test verwendete IgE-reiche Ovoalbumin-Antiserum wird in immunisierten Brown-Norway-Ratten gewonnen. Hierzu wird den Ratten zur Immunisierung eine i.p.-Injektion einer Mischung von 100 pg Ovoalbumin mit einer Bordetella-pertussis-Suspension (Vaxicoq^{R}, Hersteller: Institut Merieux, enthaltend 5 x 10⁹ Organismen und 1,25 mg Al(OH)₃) gegeben. Nach 20 Tagen erhalten die Tiere eine weitere i.p.-Injektion einer Lösung von 10 pg Ovoalbumin in 0,5 ml physiologischer Kochsalzlösung zur Reimmunisierung. Nach weiteren vier Tagen werden die Tiere entblutet und das Blut zentrifugiert. Das so erhaltene Antiserum wird bis zum Gebrauch bei -20 °C gelagert.

Die Bestimmung der Hemmung der passiven cutanen Anaphylaxie und der durch Histamin induzierten anaphylaktoiden cutanen Reaktion wird wie folgt durchgeführt:

Zur passiven Sensibilisierung gegen Ovoalbumin werden Sprague-Dawley-Ratten mit einem Körpergewicht von 150-180 g 50 µl einer 1:75-Verdünnung des IgE-reichen Ovoalbumin-Antiserum in physiologischer Natriumchlorid-Lösung intradermal an einer Flanke injiziert.

24 Stunden nach der Sensibilisierung wird den Ratten zur Auslösung einer passiven cutanen Anaphylaxie eine Lösung von 8,25 mg/kg Ovoalbumin und 26,4 mg/kg eines blauen Farbstoffes (Evans's Blue) nach Martin et al. i.v. appliziert. Der Ovoalbumin-Reiz bewirkt eine lokale anaphylaktische Reaktion an der Stelle, an welcher das Antiserum injiziert worden war.

Zur Bestimmung der histamininduzierten anaphylaktoiden Hautreaktion werden den Tieren an der der Antiserumapplikation gegenüberliegenden Flanke direkt vor der iv-Injektion der das Ovoalbumin und den blauen Farbstoff enthaltenden Lösung 50 µl einer 0,8 mg/ml Histamin enthaltenden physiologischen Natriumchloridlösung intradermal injiziert.

Am Untersuchungstage werden die Testsubstanzen in destilliertem Wasser, welches 1 Vol.-% Dimethylformamid und 1 Vol.-% Tween^{R}20 (= Polyoxyäthylen(20)sorbitanmonolaurat) enthält, gelöst. Eine Stunde vor der reizauslösenden Ovoalbumin-Applikation erhält jedes Tier 2 x 10⁻⁵ Mol/kg Testsubstanz jeweils in 0,5 ml Lösung oral appliziert. Eine Kontrollgruppe erhält zum Vergleich nur das Lösungsmittel.

Die durch die reizauslösende i.v.-Ovoalbumininjektion hervorgerufenen ödematösen anaphylactischen (P.C.A.) und anaphylactoiden (histamininduziert) Reaktionen, welche sich durch Ödembildung, Schwellung und Exsudation von blauem Farbstoff zeigen, werden 30 Minuten nach der Reizung durch die iv-Ovoalbumin-Injektion ausgewertet. Dies geschieht durch visuelle Bestimmung des Ausmaßes des Austritts von blauem Farbstoff an den Stellen der Ödembildung. Mit Hilfe von Vergleichsskalen wird die durch die Testsubstanzen hervorgerufene prozentuale Hemmung der anaphylaktischen und anaphylactoiden Reaktionen im Vergleich zu den Reaktionen der nicht mit Testsubstanz behandelten Kontrolltiere bestimmt.

Die nach den vorstehenden Testmethoden mit Verbindungen der Formel I erhaltenen Ergebnisse werden in der nachfolgenden Tabelle A wiedergegeben. Die für die Verbindungen der Formel I angegebenen Beispielsnummern beziehen sich auf die nachfolgenden Herstellungsbeispiele.

**Tabelle A**

| Test-subst. Bsp: Nr. | Hemmwirkung gegenüber cutanen anaphylactischen und anaphylactoiden Reaktionen in der Ratte % Hemmung bei Dosis von 2 x 10⁻⁵ Mol/kg p.o. | |
|---|---|---|
| | passive cutane Anaphylaxie (P.C.A.) | Histamin-induzierte anaphylactoide Reaktionen |
| 1 | 98 | 85 |
| 3 | 100 | 93 |
| 4 | 55 | 55 |
| 7 | 75 | 35 |
| 9 | 90 | 80 |
| 10 | 83 | 75 |
| 11 | 100 | 93 |
| 12 | 25 | 25 |
| 13 | 83 | 68 |
| 14 | 65 | 25 |
| 15 | 85 | 65 |
| 16 | 85 | 73 |
| 17 | 45 | 25 |
| 18 | 90 | 80 |
| 19 | 95 | 95 |
| 20 | 100 | 95 |
| 21 | 95 | 90 |
| 23 | 100 | 95 |
| 24 | 55 | 60 |
| 25 | 100 | 100 |
| 26 | 95 | 90 |
| 27 | 100 | 95 |
| 28a | 100 | 90 |
| 28b | 100 | 85 |
| 73 | 100 | 95 |
| 76 | 100 | 100 |
| 33 | 100 | 93 |
| 35 | 75 | 58 |
| 37 | 60 | 58 |
| 38 | 63 | 60 |
| 39 | 85 | 65 |
| 40 | 70 | 43 |
| 42 | 50 | 45 |
| 46 | 35 | 25 |
| 47 | 50 | 20 |
| 48 | 25 | 35 |
| 50 | 60 | 50 |
| 54 | 93 | 73 |
| 55 | 80 | 50 |
| 56 | 80 | 50 |
| 57 | 100 | 95 |
| 59 | 95 | 95 |
| 60 | 100 | 95 |
| 61 | 100 | 100 |
| 62 | 100 | 95 |
| 63 | 100 | 95 |
| 65 | 100 | 95 |
| 66 | 80 | 70 |
| 67 | 100 | 95 |
| 77 | 60 | 30 |
| 81 | 86 | 82 |
| 90 | 81 | 77 |
| 91 | 86 | 87 |

### 2. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder stark toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis in der nachstehenden Tabelle B angegeben.

**Tabelle B**

| Testsubstanz Beispiel Nr. | Minimale toxische Dosis mg/kg Maus p.o. |
|---|---|
| 1 | 100 |
| 3 | 300 |
| 11 | 100 |
| 13 | 100 |
| 14 | 300 |
| 35 | 300 |
| 38 | 300 |
| 39 | 300 |
| 40 | 300 |
| 42 | 100 |
| 54 | 300 |
| 55 | 300 |
| 56 | 300 |
| 57 | 300 |

### 3. Untersuchung der auf Histamin-(H₁)-Rezeptor Antagonismus beruhenden Antihistamin-(H₁)-Wirkung in vitro.

Zur Untersuchung des Histamin-(H₁)-Rezeptor-Antagonismus der Substanzen wird deren Hemmwirkung auf durch Histamin induzierte Kontraktionen der glatten Muskulatur am isolierten Organ in vitro bestimmt. Hierzu eignen sich isolierte Organstreifen aus dem Ileum. Sie reagieren in einem Organbad aus physiologischer Salzlösung auf Zugabe von Histamin mit Kontraktion. Durch Zugabe der erfindungsgemäßen Verbindungen wird diese durch Histaminzugabe induzierte Kontraktion der glatten Muskulatur der Ileumstreifen gemindert. Das Ausmaß der Rückbildung der Kontraktion ist ein Indiz für die Antihistamin-(H₁)-Wirksamkeit der Verbindungen. Die Untersuchung wird der ursprünglich von Magnus (Pflügers Arch. 102, 123 (1904)) beschriebenen Methode analog durchgeführt.

Versuchsbeschreibung für die Bestimmung der Hemmwirkung auf die durch eine 5 x 10⁻⁶-molare Histaminkonzentration induzierte Kontraktion am isolierten glatten Muskel des Meerschweinchen-Ileums.

Für den Versuch werden 1,5 cm lange Segmente des Ileums von Dunkin Hartley-Meerschweinchen mit einem Körpergewicht von 300 - 500 g eingesetzt.

Jeder Streifen wird in ein Organbad aus 10 ml physiologischer Salzlösung nach Krebs-Henseleit gegeben und in einer zur isotonischen Messung von Längenänderungen des Ileum-Streifens üblichen Apparatur (automatisiertes Celaster-Meßgerät) so befestigt, daß das Gewebe unter 1 g Spannung steht. Das Bad wird bei einem pH-Wert von 7,4 gehalten und mit einer Mischung aus 5 % CO₂ und 95 % O₂ begast. Nach einer Äquilibrierungsphase wird eine isotonische Kontraktion des Gewebes hervorgerufen, indem Histamin in einer Endkonzentration von 5 x 10⁻⁶ Mol/l zugegeben und nach einer Kontaktzeit von 30 Sekunden wieder ausgewaschen wird. Nur solche Gewebe, bei welchen drei reproduzierbare Kontraktionen in 10-Minuten-Abständen erhalten werden, werden in dem nachfolgenden Test verwendet. Dann werden die Testsubstanzen in einer Endkonzentration von 10⁻⁶ Mol/l zugegeben, und nach 30 Sekunden Kontaktzeit wird wiederum Histamin bis zu einer Konzentration von 5 x 10⁻⁶ Mol/l zugegeben. Die auftretenden Kontraktionen werden über 30 Sekunden gemessen. Anschließend wird das Gewebe über einen Zeitraum von 10 Minuten mehrfach gewaschen. Dann wird nochmals durch Histaminzugabe ein Kontraktionsreiz ausgelöst. Die auftretenden Kontraktionen werden wiederum über 30 Sekunden gemessen. Der Unterschied zwischen der Amplitude der durch Histaminzugabe allein erhaltenen Kontraktion und der Amplitude der in Gegenwart der Testsubstanz erhaltenen Kontraktion wird bestimmt und als % Hemmung berechnet.

Die nachfolgende Tabelle C gibt die mit den Testsubstanzen nach der vorstehend beschriebenen Methode erhaltenen Ergebnisse wieder. In der Tabelle werden die Hemmwirkungen auf die 30 Sekunden nach Applikation der Testsubstanz durch Histamin induzierten Kontraktionen und auf die durch die 10 Minuten später erfolgte Histamingabe induzierten Kontraktionen angegeben.

**Tabelle C**

| Test-subst. Bsp. Nr. | in vitro (H₁)-Rezeptorantagonismus % Hemmwirkung auf histamin-induzierte Kontraktionen des Ileums bei Histaminkonzentration 5 x 10⁻⁶ Mol/l und Testsubstanzkonzentration 10⁻⁶ Mol/l | |
|---|---|---|
| | nach 30 sec. | nach 10 min. |
| 1 | 42 | 88 |
| 3 | 15 | 57 |
| 9 | 10 | 78 |
| 10 | 47 | 92 |
| 11 | 21 | 84 |
| 13 | 33 | 63 |
| 15 | 29 | 78 |
| 16 | 42 | 55 |
| 18 | 29 | 72 |
| 19 | 26 | 83 |
| 20 | 27 | 77 |
| 21 | 9 | 56 |
| 23 | 7 | 38 |
| 24 | 81 | 94 |
| 25 | 10 | 77 |
| 26 | 15 | 42 |
| 27 | 33 | 92 |
| 28a | 25 | 24 |
| 28b | 6 | 14 |
| 29 | 7 | 30 |
| 76 | 3 | 67 |
| 33 | 45 | 96 |
| 35 | 25 | 78 |
| 37 | 24 | 61 |
| 42 | 52 | 54 |
| 43 | 11 | 23 |
| 48 | 35 | 48 |
| 50 | 24 | 50 |
| 54 | 8 | 94 |
| 57 | 20 | 80 |
| 60 | 9 | 37 |
| 62 | 13 | 72 |
| 63 | 17 | 67 |
| 65 | 21 | 48 |
| 66 | 10 | 71 |
| 67 | 59 | 93 |
| 80 | 15 | 63 |
| 90 | 19 | 88 |
| 91 | 16 | 83 |
| 78 | 12 | 92 |
| 31 | 31 | 89 |
| 83 | 12 | 52 |
| 95 | 10 | 71 |

### 4. Bestimmung der Anti-P.A.F.-Wirkung in vitro.

P.A.F. (= Platelet Activating Factor = Blutplättchenaggregationsfaktor) ist ein phosphorlipidischer Mediator, welcher eine Vielzahl von Wirkungen besitzt. Die Aktivierung der Blutplättchenaggregation führt zur Induzierung langanhaltender Bronchokontraktionen und Überreaktivität der Luftwege.

In diesem Test wird nach der von Mikashima et al. beschriebenen Methode (Jap. J. Pharmacol. 44 (1987) 387-391) die Wirkung der Testsubstanzen auf eine durch Zugabe von P.A.F. induzierte Blutplättchenaggregation in einer aus Kaninchenblut gewonnenen Blutplättchensuspension untersucht.

Es wird eine Suspension von aus Kaninchenblut stammenden Blutplättchen verwendet, welche 4 x 10⁹ Blutplättchen/ml in einer auf pH 7,4 eingestellten modifizierten Tyrode-Pufferlösung (= Tyrode-Lösung* mit Zusatz von 1,3 mMol/l CaCl₂ und 2,5 g/l Gelatine) enthält. Die Blutplättchen werden aus 10 ml Blutproben von jeweils drei Kaninchen (Neuseelandhybriden, Körpergewicht 3 - 4 kg) erhalten. Hierzu werden die Blutproben mit Äthylendiamintetraessigsäure behandelt und nach der Methode von Artley et al. (Brit. J. Haematol. 19 (1970), 7-17) gewaschen. Sodann wird durch Zentrifugieren (20 Minuten bei 400 x g) zunächst ein blutplättchenreiches Plasma abgetrennt. Durch nochmaliges Zentrifugieren für 15 Minuten bei 1400 x g werden die Blutplättchen von dem Plasma getrennt. Nach dem Zentrifugieren werden die als Sediment verbleibenden Blutplättchen in einer Tyrode-Pufferlösung (jedoch ohne Calzium) resuspendiert. Sodann werden 0,4 mMol Lysinacetylsalicylat zugegeben, und nach 15 Minuten werden die Blutplättchen nochmals sedimentiert. Das Sediment wird in der vorgenannten modifizierten Tyrodepufferlösung resuspendiert, und die Anzahl der Blutplättchen in der erhaltenen Suspension wird auf den gewünschten Gehalt eingestellt.
* Tyrodelösung = wäßrige Lösung enthaltend pro Liter 136,9 mMol NaCl, 2,68 mMol KCl, 2,31 mMol CaCl₂, 1,0 mMol MgCl₂, 11,9 mMol NaHCO₃, 1,45 mMol NaH₂PO₄ und 5,55 mMol Glucose.

Es wird eine 40 x 10⁻⁹-molare P.A.F.-Lösung als Reagenz eingesetzt. Diese Lösung wird aus einer 1,8 x 10⁻³-molaren Vorratslösung in Chloroform hergestellt. Hierzu wird eine 10 pl Probe der Vorratslösung zur Trockne eingedampft und wieder in 180 µl der modifizierten Tyrodelösung, welcher 0,25 % von Lipiden befreites Rinderserum-Albumin zugesetzt worden war, gelöst. Daraus werden sodann 10⁻⁵-molare Arbeitslösungen hergestellt und gefroren aufbewahrt. Für Tests werden Proben dieser Lösungen entsprechend verdünnt.

Zur Testdurchführung werden in einer mit einem kleinen Magnetrührer versehenen Aggregationsröhre zu 330 µl der modifizierten Tyrode-Pufferlösung unter Rühren (1000 UpM) 50 µl der Blutplättchensuspension und 10 µl einer 40 x 10⁻⁵-molaren Lösung der zu untersuchenden Verbindung gegeben. Dies entspricht einer Endkonzentration an Testsubstanz von 10⁻⁵ Mol/l. Nach 90 Sekunden Vorinkubationszeit werden 10 µl der P.A.F.-Zubereitung hinzugefügt. Während 4-5 Minuten wird die in den Aggregationsröhrchen auftretende Aggregation mit Hilfe eines computerisierten Aggregometers gemessen.

Die in den nur Blutplättchensuspension enthaltenden Teströhren auftretende Aggregation wird als 0 % gewertet, während die in Blutplättchensuspension und P.A.F.-Zubereitung enthaltenden Teströhrchen auftretende Aggregation als 100 % gewertet wird. Die bei durch Zusatz der Testsubstanzen verursachter Hemmung der durch P.A.F. induzierten Blutplättchenaggregationsverstärkung noch auftretende Aggregation wird gemessen und daraus die erfolgte Aggregationshemmung in % berechnet.

Die nach der vorstehenden Methode mit den Verbindungen der Formel I erhaltenen Ergebnisse werden in der nachfolgenden Tabelle D wiedergegeben.

**Tabelle D**

| Test subst. Bsp. Nr. | Anti-P.A.F.-Wirkung in vitro. % Hemmung der P.A.F.-induzierten Aggregation von Blutplättchen aus Kaninchenblut bei einer Testsubstanzkonzentration von 10⁻⁵ Mol/l |
|---|---|
| 1 | 97 |
| 3 | 100 |
| 4 | 58 |
| 7 | 100 |
| 8 | 100 |
| 9 | 73 |
| 10 | 94 |
| 11 | 89 |
| 12 | 100 |
| 13 | 93 |
| 14 | 86 |
| 15 | 85 |
| 16 | 73 |
| 19 | 83 |
| 20 | 17 |
| 21 | 91 |
| 23 | 100 |
| 24 | 80 |
| 25 | 43 |
| 26 | 89 |
| 27 | 83 |
| 73 | 99 |
| 76 | 99 |
| 33 | 71 |
| 35 | 56 |
| 37 | 28 |
| 40 | 100 |
| 46 | 91 |
| 47 | 95 |
| 54 | 75 |
| 55 | 40 |
| 56 | 42 |
| 57 | 86 |
| 59 | 89 |
| 60 | 44 |
| 61 | 83 |
| 62 | 70 |
| 63 | 98 |
| 65 | 100 |
| 66 | 100 |
| 67 | 76 |
| 77 | 100 |
| 80 | 72 |
| 85 | 100 |
| 86 | 100 |
| 89 | 100 |
| 90 | 64 |
| 91 | 58 |
| 79 | 71 |
| 78 | 98 |
| 31 | 95 |
| 32 | 100 |
| 83 | 100 |
| 87 | 100 |
| 93 | 100 |
| 96 | 98 |
| 97 | 99 |

### 5. In-vitro-Bestimmung der Cyclooxygenase-Hemmung und der 5-Lipoxygenase-Hemmung.

In Zellmembranen enthaltene Arachidonsäure wird nach Aktivierung der Zelle in zweierlei Weise metabolisiert. Unter Einwirkung des Enzyms 5-Lipoxygenase (= 5-LO) werden Leukotriene, unter anderem das Leukotrien C₄, und unter Einwirkung des Enzyms Cyclooxygenase (= CO) Prostanoide gebildet. In in-vitro-Systemen werden diese Metaboliten aus der Zelle sekretiert.

Zur Untersuchung der cyclooxygenasehemmenden und der 5-lipoxygenasehemmenden Eigenschaften wird die Hemmwirkung der Testsubstanzen auf die Biosynthese der Arachidonsäurederivate Leukotrien C₄ (= LTC₄) und 6-Keto-prostaglandin F₁α (= 6-Keto-PGF₁α) an peritonealen Macrophagenzellen der Maus in vitro bestimmt. Hierzu werden die Gehalte an LTC₄ und 6-Keto-PGF₁α in einem Kulturmedium von peritonalen Macrophagenzellen der Maus nach Zymosan-Stimulation wie von Scott et al (J. Exp. Med. 152 (1980), 324-335) und von Fradin et al (Prostaglandins, 33 (1987), 579-589) beschrieben bestimmt.

Eine peritoneale Zellen von männlichen 8 - 10 Wochen alten Mäusen enthaltende Zellsuspension wird auf an sich bekannte Weise gewonnen. Als Zellkulturmedium wird eine unter der Bezeichnung RPMI 1640 im Handel befindliche Lösung (Hersteller Fa. Gibco) verwendet, der Heparin (10 internationale Einheiten/ml) und Antibiotica nach der Rezeptur von Bonney et al. (Biochem. J. 176 (1978) 422-433) zugesetzt werden. Die Zellsuspension wird auf eine Zellkonzentration von 10⁶ Zellen pro ml eingestellt und gleichmäßig auf 24 1-ml-Titerzellen (Wells) enthaltende Titerplatten verteilt. Diese werden zwei Stunden in einem feucht gehaltenen, mit mit 7 % CO₂ angereicherter Luft beschickten Inkubator gehalten. Anschließend werden nicht an den Titerzellwänden festhaftende Zellen durch Waschen entfernt. Die verbleibenden an den Wänden anhaftenden Macrophagenzellen werden ca. 12 Stunden in einem Suspensionsmedium, welchem 0,1 % Rinderserumalbumin (BSA = Bovine Serum Albumin) zugesetzt wird, inkubiert. Sodann wird das Suspensionsmedium ersetzt durch eine Salzlösung nach Hanks (= Hanks' Balanced Salt Solution = HBSS) mit 10 mM Hepes (= Hydroxyäthylpiperazinoäthansulfonsäure), welcher eine 0,1 %-ige Lösung der Testsubstanzen in wäßrigem, 1 %-igem Dimethylformamid oder nur das Lösungsmittel zugesetzt worden war. 15 Minuten später wird der Arachidonsäuremetabolismus durch Zugabe von 10 Partikeln Zymosan (= Glykoproteinmischung, isoliert aus Zellwänden von Bierhefe, Saccharomyces cerevisiae, Hersteller Sigma Chemical Co., München) pro Titerzelle stimuliert. Nach 2 Stunden werden Proben der jeweils überstehenden Flüssigkeit auf ihren Gehalt an 6-Keto-PGF₁α und LTC₄ mittels eines enzymatischen Immunassay (= EIA) untersucht. Dieser EIA wird nach der Methode von Pradelles et al. (Analytical Chem. 57 (1985), 1170-1173) durchgeführt. Die Bestimmung von LTC₄ und die Bestimmung von 6-Keto-PGF₁α werden jeweils im Vergleich mit einer Vergleichsskala an geeigneten Verdünnungen der Proben (für die LTC₄-Bestimmung 1:50 bis 1:250 und für die 6-Keto-PGF₁α-Bestimmung 1:250 bis 1:1250) durchgeführt. Zur Bestimmung der Hemmwirkung einer 10⁻⁵-molaren Konzentration der Verbindungen wird die Menge an Bezugseicosanoiden bestimmt und daraus die Hemmmwirkung in % Hemmung verglichen mit den Zymosan-Kontrollmessungen berechnet. Die mit diesem Test erhaltenen Ergebnisse werden in der nachfolgenden Tabelle E wiedergegeben.

**Tabelle E**

| Test Subst. Bsp. Nr. | In vitro % Hemmwirkung in Zymosan-stimulierten peritonealen Macrophagenzellen der Maus bei einer Konzentration von 10⁻⁵ Mol/l auf die Freisetzung von | |
|---|---|---|
| | 6-Keto-PGF₁α | LTC₄ |
| 1 | 23 | 24 |
| 3 | 20 | 37 |
| 4 | 20 | 44 |
| 7 | 49 | 56 |
| 8 | 74 | 99 |
| 9 | 19 | 62 |
| 12 | 53 | 97 |
| 14 | 25 | 55 |
| 16 | 18 | 17 |
| 19 | 5 | 55 |
| 20 | 42 | 0 |
| 21 | 64 | 95 |
| 23 | 43 | 94 |
| 25 | 24 | 69 |
| 26 | 68 | 89 |
| 27 | 10 | 61 |
| 73 | 29 | 90 |
| 76 | 67 | 75 |
| 33 | 21 | 4 |
| 35 | 51 | 75 |
| 37 | 32 | 13 |
| 39 | 8 | 60 |
| 42 | 76 | 15 |
| 43 | 57 | 24 |
| 47 | 69 | |
| 55 | 41 | 45 |
| 59 | 43 | 80 |
| 61 | 65 | 98 |
| 62 | 64 | 94 |
| 63 | 38 | 98 |
| 65 | 52 | 89 |
| 66 | 72 | 33 |
| 67 | 36 | 66 |
| 77 | 42 | 74 |
| 82 | 58 | 9 |
| 85 | 52 | 62 |
| 81 | 16 | 29 |
| 84 | 46 | 20 |
| 89 | 32 | 51 |
| 90 | 56 | 66 |
| 91 | 54 | 81 |
| 79 | 52 | 71 |
| 83 | 29 | 34 |
| 80 | 12 | 39 |
| 87 | 20 | 23 |

Aufgrund ihrer vorstehend beschriebenen Wirkungen sind die Verbindungen der Formel I als antiinflammatorisch und antiallergisch wirksame Arzneimittel für größere Säugetiere, insbesondere Menschen geeignet zur Behandlung von entzündlichen und allergischen Erkrankungen. Die erfindungsgemäßen oral wirksamen Verbindungen können in mehrfacher Hinsicht wirken, da sie gegen mehrere der Hauptmediatoren, welche an entzündlichen Vorgängen und asthmatischen Beschwerden beteiligt sind, wirksam sind.

Aufgrund dieses Wirkungsprofils ist davon auszugehen, daß die erfindungsgemäßen Verbindungen im Rahmen der Behandlung von allergisch bedingten und nicht allergisch bedingten Asthmabeschwerden nicht nur die mit asthmatischen Erkrankunkungen verbundenen symptomatischen Beschwerden lindern, sondern auch die damit verbundene Entzündung mindern können. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 10 bis 250 mg pro Einzeldosis.

Als Heilmittel können die Verbindungen der Formel I mit üblichen pharmazeutischen Hilfsstoffen in galenischen Zubereitungen, wie z.B. Tabletten, Kapseln, Suppositorien oder Lösungen, enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssigen Paraffinen und unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Struktur der neuen Substanzen wurde durch spektroskopische Untersuchungen, insbesondere durch eine Analyse der IR- und NMR-Spektren, sowie durch Elementaranalyse gesichert. Die Reinheit der Zwischenprodukte wurde dünnschichtchromatographisch überwacht.

### Beispiel 1:

### 5,6-Dihydro-2-methyl-1-{2-[4-(4-methylpyridin-2-yl)-piperazin-1-yl]-ethyl}-4H-pyrrolo[3,2,1-ij]chinolin.

A) Zu einer Mischung aus 333 ml 12 N Salzsäure und 800 g Eis wurden 266,4 g 1,2,3,4-Tetrahydrochinolin gegeben. Zu dem Gemisch wurde langsam, über 2 Stunden verteilt, eine Lösung von 165 g Natriumnitrit in 500 ml Wasser zugefügt, wobei die Temperatur unter 5 °C gehalten wurde. Anschließend ließ man das Reaktionsgemisch sich innerhalb 1 Stunde auf Raumtemperatur erwärmen. Sodann wurde das Reaktionsgemisch zweimal mit je 500 ml Toluol extrahiert. Die organische Phase wurde abgetrennt, viermal mit je 300 ml Wasser gewaschen, getrocknet und eingedampft. Als Rückstand wurden 269 g rohes 1,2,3,4-Tetrahydro-1-nitrosochinolin als bräunliches Öl erhalten.
B) Zu einem Liter auf ca. 5 °C abgekühltem Tetrahydrofuran wurden langsam 50,5 g Lithiumaluminiumhydrid gegeben, wobei eine Temperatur von zwischen 5 und 10 °C eingehalten wurde. Anschließend ließ man das Gemisch sich auf eine Temperatur von ungefähr 15 °C erwärmen. Über einen Zeitraum von 4 Stunden verteilt wurde eine Lösung von 108 g des vorstehend erhaltenen 1,2,3,4-Tetrahydro-1-nitrosochinolins in 500 ml Tetrahydrofuran zu dem Reaktionsgemisch gegeben, wobei eine Temperatur von zwischen 15 und 20 °C eingehalten wurde. Anschließend wurde das Reaktionsgemisch noch 1 ½ Stunden bei Raumtemperatur gehalten. Zur Aufarbeitung wurde das Reaktionsgemisch sodann auf 5 °C abgekühlt und es wurden zur Hydrolyse zunächst eine Mischung aus 50 ml Wasser und 50 ml Tetrahydrofuran, sodann 50 ml 15-%ige wäßrige Natronlauge und weitere 50 ml Wasser zugegeben. Sodann wurde von dem gebildeten Niederschlag abfiltriert und dieser nochmals mit Dichlormethan nachgewaschen. Die vereinigten Filtrate wurden eingeengt und der verbleibende Rückstand wurde in Dichlormethan gelöst. Die erhaltene Lösung wurde mit Wasser gewaschen, getrocknet und eingedampft. Es wurden 85,1 g rohes 1-Amino-1,2,3,4-tetrahydrochinolin als bräunliches Öl erhalten.
C) 85 g des vorstehend erhaltenen Produktes wurden in einem Gemisch aus 99,2 g Lävulinsäureethylester (= 3-Acetylpropionsäureethylester), 852 ml Essigsäure und 52,5 ml 12 N Salzsäure 1 Stunde auf eine Temperatur von 80 °C unter Rückfluß erhitzt. Sodann wurden das Reaktionsgemisch auf 50 °C abgekühlt, organische Lösungsmittel weitgehend abgedampft und 200 ml Wasser zugefügt. Das wäßrige Reaktionsgemisch wurde mit gesättigter Natriumbicarbonatlösung neutralisiert und mit Dichlormethan extrahiert. Die Dichlormethanphase wurde abgetrennt, mit Wasser gewaschen, über Natriumsulfat getrocknet, filtriert und eingedampft. Als Rückstand wurden 159,9 g eines teerigen bräunlichen Rohproduktes erhalten. Dieses wurde in 2 % Toluol enthaltendem Ethanol gelöst und chromatographisch über eine kleine Kieselgelsäule unter Verwendung von Toluol/Ethanol 98:2 als Elutionsmittel gereinigt. Das von der Säule ablaufende Eluat wurde eingedampft und der verbleibende Rückstand, welcher neben 5,6-Dihydro-2-methyl-4H-pyrrolo [3,2,1-ij]chinolin-1-essigsäureethylester auch Beimengungen der entsprechenden Säure enthielt, wurde in 200 ml Dichlormethan gelöst. Die Lösung wurde zur Entfernung der sauren Anteile mit 10-%iger wäßriger Natriumhydroxidlösung gewaschen. Anschließend wurde mit Wasser neutral gewaschen, die organische Phase abgetrennt, über Natriumsulfat getrocknet und eingedampft. Es wurden 118,8 g rohes 5,6-Dihydro-2-methyl-4-pyrrolo[3,2,1-ij]chinolin-1-essigsäureethylester als bräunliches Öl erhalten.
D) In 500 ml auf ca. 5 °C abgekühltes Tetrahydrofuran wurden 17,4 g Lithiumaluminiumhydrid gegeben. Dann ließ man die Temperatur auf ca. 15 °C ansteigen und fügte langsam eine Lösung von 59 g des vorstehend erhaltenen Esters in 500 ml Tetrahydrofuran über einen Zeitraum von 3 Stunden verteilt zu, wobei die Temperatur unter 22 °C gehalten wurde. Anschließend ließ man das Reaktionsgemisch noch 1 Stunde bei Raumtemperatur reagieren. Zur Aufarbeitung wurde das Reaktionsgemisch auf ca. 5 °C abgekühlt und es wurden zur Hydrolyse zunächst eine Mischung aus 17 ml Wasser und 17 ml Tetrahydrofuran, sodann 17 ml 15-%ige wäßrige Natronlauge und nochmals 17 ml Wasser zugegeben. Anschließend wurde von dem gebildeten Niederschlag abfiltriert und dieser nochmals mit Dichlormethan nachgewaschen. Die organischen Filtrate wurden eingeengt, der Rückstand in Dichlormethan aufgenommen und die Dichlormethanphase mit Wasser gewaschen, getrocknet und eingedampft. Es wurden 44,3 g 5,6-Dihydro-1-(2-hydroxyethyl)-2-methyl-4H-pyrrolo[3,2,1-ij]chinolin als gelbliches Pulver erhalten.
E) Eine Lösung von 44 g des vorstehend erhaltenen Produktes in 250 ml Chloroform wurde auf eine Temperatur von 10 bis 15 °C abgekühlt. Sodann wurde langsam eine Lösung von 41,5 g Phosphortribromid in 85 ml Chloroform zugegeben und das Reaktionsgemisch wurde 1 Stunde unter Rückfluß erhitzt. Zur Aufarbeitung wurde das Reaktionsgemisch durch Eingeben in eine Eis/Wasser-Mischung hydrolysiert. Sodann wurde mit Chloroform extrahiert. Die organische Phase wurde abgetrennt, mit 100 ml 10-%iger wäßriger Natriumbicarbonatlösung und anschließend mit Wasser gewaschen, getrocknet und eingeengt. Als Rückstand wurden 62,8 g eines bräunlichen Öles erhalten. Dieses wurde aus absolutem Alkohol umkristallisiert. Hierbei wurden 33,7 g 1-(2-Bromethyl)-5,6-dihydro-2-methyl-4H-pyrrolo[3,2,1-ij]chinolin als cremeweißes Pulver erhalten.
F) 10 g des vorstehend erhaltenen Produktes, 7,64 g 1-(4-Methylpyridin-2-yl)-piperazin, 0,6 g Kaliumjodid und 10,1 ml Triethylamin wurden in 100 ml Dimethylformamid während 1 ½ Stunden unter Rückfluß auf 90 °C erhitzt. Zur Aufarbeitung wurde das Reaktionsgemisch eingedampft, der verbleibende Rückstand in Wasser aufgenommen und mit Dichlormethan extrahiert. Die Dichlormethanphase wurde abgetrennt, mit 10-%iger wäßriger Natriumhydroxidlösung gewaschen und anschließend mit Wasser neutral gewaschen und eingeengt. Als Rückstand verblieben 14,6 g der rohen Titelverbindung als bräunliches Öl. Dieses wurde chromatographisch über eine kleine Kieselgelsäule gereinigt unter Verwendung von anfangs Toluol und später Toluol/Ethanol 95:5 als Elutionsmittel. Es wurden 9,7 g festes 5,6-Dihydro-2-methyl-1-{2-[4-(4-methylpyridin-2-yl)-piperazin-1-yl]-ethyl}-4H-pyrrolo[3,2,1-ij]chinolin erhalten.

Zur Überführung in ihr Trihydrochlorid wurde die vorstehend erhaltene Titelbase in Isopropanol gelöst. Der isopropanolischen Lösung der Titelverbindung wurde isopropanolische 2,3 N Salzsäure zugesetzt, wobei das Trihydrochlorid der Titelverbindung auskristallisierte. Das erhaltene 5,6-Dihydro-2-methyl-1-{2-[4-(4-methylpyridin-2-yl)-piperazin-1-yl]-ethyl}-4H-pyrrolo [3,2,1-ij]chinolin-trihydrochlorid hatte einen Schmelzpunkt von 254 °C.

### Beispiel 2:

### 5,6-Dihydro-2-methyl-1-{2-[4-(4-fluorbenzoyl)-piperidin-1-yl]-ethyl}-4H-pyrrolo[3,2,1-ij]chinolin.

A) 100 g Piperidin-4-carbonsäure wurden in 400 ml Acetanhydrid 4 Stunden unter Rückfluß und Rühren auf eine Temperatur von 135 °C erhitzt. Anschließend ließ man auf Raumtemperatur abkühlen. Zur Aufarbeitung wurde das Reaktionsgemisch zur Entfernung von Acetanhydrid eingedampft, wobei gegen Ende des Eindampfens mehrmals Toluol zugesetzt wurde. Nach dem Eindampfen verblieb ein beigefarbenes festes Rohprodukt als Rückstand. Dieses wurde in 300 ml eines Gemisches aus Diisopropylether und Dichlormethan suspendiert, auf eine Temperatur von 0 °C abgekühlt und filtriert. Es wurden 65 g rohe 1-Acetylpiperidin-4-carbonsäure mit einem Schmelzpunkt von 180 bis 182 °C erhalten. Das Filtrat wurde in der Hitze mit 200 ml Wasser verdünnt, wobei weitere 1-Acetylpiperidin-4-carbonsäure als Niederschlag ausfiel und abfiltriert wurde. Hierbei wurden nochmals 42,91 g der Säure erhalten, so daß die Gesamtausbeute an 1-Acetylpiperidin-4-carbonsäure 107,9 g betrug.
B) 40 g der vorstehend erhaltenen Säure wurden in 200 ml Sulfonylchlorid unter Rückfluß erhitzt. Nach 15 Minuten trat eine Braunfärbung ein, die Lösung wurde abgekühlt und noch 2 Stunden bei Raumtemperatur reagieren gelassen. Anschließend wurde das Sulfonylchlorid abgedampft und der verbleibende braun-rote Feststoff wurde zunächst mit Toluol und anschließend mit Petrolether gespült und unter vermindertem Druck getrocknet. Es wurden 49 g rohes 1-Acetylpiperidin-4-carbonsäurechlorid erhalten.
C) 26 g Aluminiumchlorid wurden in 50 ml Fluorbenzol gegeben. Zu der Mischung wurden 19 g des vorstehend erhaltenen Säurechlorids in kleinen Portionen zugegeben. Anschließend wurde das Reaktionsgemisch für 3 Stunden auf Rückflußtemperatur des Fluorbenzols erhitzt. Sodann wurde die Reaktion durch Zugabe von Eis unterbrochen, das Reaktionsgemisch mit Chloroform extrahiert, die Chloroformphase abgetrennt, mit Wasser gewaschen und eingeengt. Es wurden 18,33 g rohes 1-Acetyl-4-(4-fluorbenzoyl)-piperidin erhalten.
D) 18 g des vorstehend erhaltenen Produktes wurden in einer Mischung aus 120 ml 12 N Salzsäure und 80 ml Wasser 3 Stunden am Rückfluß erhitzt. Anschließend wurde etwas Dichlormethan zugegeben, und das Reaktionsgemisch wurde unter Kühlung im Eisbad durch Zugabe von Natriumhydroxidlösung alkalisch gestellt. Dann wurde mit Dichlormethan extrahiert, die Dichlormethanphase mit Wasser gewaschen, getrocknet und eingeengt. Es wurden 15,68 g Rohprodukt erhalten. Dieses wurde in Toluol gelöst, sodann wurden 25 ml von mit Chlorwasserstoff gesättigtem Isopropanol zugegeben. Der ausgefallene beigefarbene Niederschlag wurde abfiltriert und getrocknet. Es wurden 11,31 g 4-(4-Fluorbenzoyl)-piperidinhydrochlorid als graufarbenes Pulver erhalten.
E) 5,78 g des vorstehenden Produktes, 6 g 1-(2-Bromethyl)-5,6-dihydro-2-methyl-4H-pyrrolo[3,2,1-ij]chinolin und 9,0 ml Triethylamin wurden in 50 ml Toluol am Rückfluß erhitzt, wobei sich allmählich ein cremeweißer Niederschlag bildete. Nach 8 Stunden wurden nochmals 2 ml Triethylamin zugegeben und später auch einige Kristalle feinzerriebenes Kaliumjodid. Insgesamt wurde das Reaktionsgemisch 32 Stunden erhitzt. Zur Aufarbeitung wurde das Reaktionsgemisch mit 500 ml Dichlormethan verdünnt, mit Wasser gewaschen, getrocknet, und zur Trockne eingedampft. Es wurden 9,83 g Rohprodukt erhalten. Aus diesem wurden nach chromatographischer Reinigung über Kieselgel 4,64 g rohe Titelverbindung erhalten. Nach Umkristallisation aus Isopropanol wurden 3,96 g 5,6-Dihydro-2-methyl-1-{2-[4-(4-fluorbenzoyl)-piperidin-1-yl]-ethyl}-4H-pyrrolo[3,2,1-ij]chinolin mit einem Schmelzpunkt von 124 - 125 °C erhalten.

### Beispiel 3:

### 5,6-Dihydro-2-methyl-4-phenyl-{2-[4-(4-methylpyridin-2-yl)-piperazin-1-yl]-ethyl}-4H-pyrrolo [3,2,1-ij]chinolin.

A) 30 g Chinolin wurden in 100 ml Tetrahydrofuran gelöst. Die Lösung wurde auf 2 °C abgekühlt, sodann wurden 19,6 g Phenyllithium in Form einer 2 M Lösung in einem Gemisch aus Cyclohexan/Diethylether 70:30 über eine Dauer von 2 Stunden verteilt zugegeben, wobei die Temperatur zwischen 0 und 2 °C gehalten wurde. Anschließend wurde das Reaktionsgemisch sich auf Raumtemperatur erwärmen lassen. Zur Aufarbeitung wurde das Reaktionsgemisch in Wasser gegeben und anschließend mit Diethylether extrahiert. Die Etherphase wurde mit Wasser neutral gewaschen, getrocknet und eingeengt. Es wurden 50 g Rohprodukt erhalten, welches durch Chromatograhpie über eine Kieselgelsäule unter Verwendung von Toluol als Elutionsmittel gereinigt wurde. Es wurden 20,3 g 1,2-Dihydro-2-phenylchinolin mit einem Schmelzpunkt von 75 °C erhalten.
B) 2 g des vorstehend erhaltenen Produktes wurden in 40 ml Ethanol unter Rühren am Rückfluß erhitzt. Dabei wurden portionsweise über 2 Stunden verteilt insgesamt 4 g metallisches Natrium zu dem Reaktionsgemisch gegeben. Zur Aufarbeitung wurde das Reaktionsgemisch in 200 ml Eiswasser gegeben. Anschließend wurde mit Ether extrahiert, die Etherphase mit Wasser neutral gewaschen, getrocknet und eingeengt. Als Rückstand verblieben 1,6 g rohes öliges 1,2,3,4-Tetrahydro-2-phenylchinolin.
C) 30 g des vorstehend erhaltenen Produktes wurden mit 11,38 g Natriumnitrit analog Beispiel 1A) umgesetzt. Das Reaktionsgemisch wurde wie in Beispiel 1A) beschrieben aufgearbeitet. Es wurden 32,5 g öliges rohes 1,2,3,4-Tetrahydro-1-nitroso-2-phenylchinolin erhalten.
D) 32 g des vorstehend erhaltenen Produktes wurden in Tetrahydrofuran mit 15,25 g Lithiumaluminiumhydrid nach der in Beispiel 1B beschriebenen Methode reduziert. Das Reaktionsgemisch wurde wie in Beispiel 1B) beschrieben aufgearbeitet. Es wurden 28,2 g rohes 1-Amino-1,2,3,4-tetrahydro-2-phenylchinolin als orangefarbenes Öl erhalten.
E) 28 g des vorstehend erhaltenen Produktes wurden mit 21,6 g Lävulinsäureethylester in einem Gemisch aus 187,5 ml Essigsäure und 12,5 ml 12 N Salzsäure 2 Stunden unter Rückfluß auf eine Temperatur von 80 °C erhitzt. Zur Aufarbeitung wurden das Reaktionsgemisch abgekühlt, organische Lösungsmittel abgedampft, der Rückstand in 100 ml Wasser aufgenommen, die wäßrige Phase durch Zugabe von Natriumbicarbonat neutralisiert und dann zweimal mit je 100 ml Toluol extrahiert. Die Toluolphase wurde mit Wasser gewaschen, getrocknet und eingeengt. Es verblieben 36,6 g öliges Rohprodukt. Dieses wurde zur weiteren Reinigung über eine Kieselgelsäule unter Verwendung von Toluol/Ethanol 9:1 als Elutionsmittel chromatographiert. Hierbei wurden 26,6 g eines bräunlichen Öls erhalten, welches durch nochmalige Chromatographie über eine Kiesegelsäule unter Verwendung von Toluol/Ethanol 9.8:0,2 als Elutionsmittel weiter aufgetrennt wurde. Es wurden 10 g reines 5,6-Dihydro-2-methyl-4-phenyl-4H-pyrrolo[3,2,1-ij]chinolin-1-essigsäureethylester erhalten. Außerdem wurden noch 12,8 g einer Mischung dieses Esters mit der entsprechenden Säure erhalten. Dieses Gemisch wurde zur vollständigen Veresterung in Ethanol unter Zusatz von Schwefelsäure 2 ½ Stunden am Rückfluß erhitzt. Zur Aufarbeitung wurde das Ethanol aus dem Reaktionsgemisch abgedampft, dann wurde Wasser zugesetzt und mit Ethylacetat extrahiert. Die organische Phase wurde mit Wasser gewaschen, getrocknet und eingeengt. Der verbleibende Rückstand bestand aus reinem 5,6-Dihydro-2-methyl-4-phenyl-4H-pyrrolo [3,2,1-ij]chinolin-1-essigsäureethylester.
F) 19,2 g des vorstehend erhaltenen Produktes wurden in Tetrahydrofuran mit insgesamt 9,5 g Lithiumaluminiumhydrid nach der in Beispiel 1D) beschriebenen Methode reduziert, wobei das Reaktiongemisch jedoch 3 Stunden am Rückfluß erhitzt wurde. Zur Aufarbeitung wurde das Reaktionsgemisch abgekühlt, und es wurden zur Hydrolyse zunächst 9,5 ml Wasser, dann 9,5 ml 15-%ige wäßrige Natronlauge und nochmals 9,5 ml Wasser zugesetzt. Dann wurde von dem gebildeten Niederschlag abfiltriert und der Filterrückstand nochmals mit Ether gewaschen. Die Filtrate wurden eingeengt. Der verbleibende Rückstand wurde in Ether aufgenommen, die organische Phase mit Wasser gewaschen, getrocknet und eingedampft. Es wurden 13,7 g rohes 5,6-Dihydro-1-(2-hydroxyethyl)-2-methyl-4-phenyl-4H-pyrrolo[3,2,1-ij]chinolin als gelbes Öl erhalten.
G) 13,6 g des vorstehend erhaltenen Alkoholes wurden in 80 ml Chloroform mit 9,37 g Phosphortribromid nach der in Beispiel 1E) beschriebenen Methode umgesetzt und das Reaktionsgemisch wurde wie in Beispiel 1E) beschrieben aufgearbeitet. Es wurden 16,4 g 1-(2-Bromethyl)-5,6-dihydro-2-methyl-4-phenyl-4H-pyrrolo[3,2,1-ij]chinolin als Öl erhalten, welches anschließend kristallisierte.
H) 8 g des vorstehend erhaltenen Produktes, 4,8 g 1-(4-Methylpyridin-2-yl)-piperazin und 6,3 ml Triethylamin wurden in 60 ml Dimethylformamid 12 Stunden am Rückfluß erhitzt. Zur Aufarbeitung wurden dem Reaktionsgemisch 100 ml 20-%ige wäßrige Salzsäure zugesetzt. Dann wurde das Reaktionsgemisch durch Zugabe Natriumbikarbonat neutralisiert und mit Dichlormethan extrahiert. Die organische Phase wurde mit Wasser neutral gewaschen, getrocknet und eingedampft. Es wurden 9 g rohe ölige Titelverbindung erhalten. Zur Überführung in ihr Hydrochlorid wurde die Titelverbindung in 20 ml Isopropylalkohol gelöst und zu der Lösung wurden 19 ml einer 2,1-molaren isopropanolischen Salzsäurelösung gegeben. Der gebildete Niederschlag wurde abfiltriert und aus absolutem Alkohol und wenig Wasser umkristallisiert. Es wurden 5,4 g 5,6-Dihydro-2-methyl-4-phenyl-1-{2-[4-(4-methylpyridin-2-yl)-piperazin-1-yl]-ethyl}-4H-pyrrolo[3,2,1-ij]chinolin·2,2 HCl·0,3 H₂O mit einem Schmelzpunkt von 216 °C erhalten.

### Beispiel 4:

### 5,6-Dihydro-4-n-heptyl-2-methyl-1-{2-[4-(4-methylpyridin-2-yl)-piperazin-1-yl]-ethyl}-4H-pyrrolo[3,2,1-ij]chinolin.

A) Zu einer Mischung aus 70 ml einer 1,6 M Lösung von n-Butyllithium in Hexan und 40 ml Diethylether wurde langsam eine Lösung von 25 g 2-Methylchinolin in 40 ml Ether gegeben. Das Reaktionsgemisch wurde bei Raumtemperatur gerührt, anschließend wurde auf ca. 5 °C abgekühlt und langsam eine Lösung von 28,9 g n-Hexylbromid in 10 ml Ether zugegeben. Dann wurde das Reaktionsgemisch auf Raumtemperatur erwärmt und 2 Stunden bei Raumtemperatur gerührt. Zur Aufarbeitung wurde das Reaktionsgemisch in 500 ml Wasser gegeben und mit Ether extrahiert. Die organische Phase wurde mit leicht angesäuertem Wasser gewaschen, getrocknet und eingedampft. Das erhaltene Rohprodukt wurde chromatographisch über eine Kieselgelsäule unter Verwendung von Dichlormethan als Elutionsmittel gereinigt. Es wurden 31 g öliges rohes 2-n-Heptylchinolin erhalten.
B) 31 g des vorstehend erhaltenen Produktes wurden in 300 ml Essigsäure gelöst. Zu der Lösung wurden allmählich über 20 Minuten verteilt 18 g Natriumcyanoborhydrid gegeben. Die Temperatur stieg dabei bis zu ca. 28 °C an. Das Reaktionsgemisch wurde weitere 12 Stunden bei dieser Temperatur gerührt. Zur Aufarbeitung wurden dem Reaktionsgemisch 800 ml 10 N wäßrige Natriumhydroxidlösung unter Kühlung durch Zugabe von Eis zugegeben. Anschließend wurde das Reaktionsgemisch mit Ethylacetat extrahiert. Die organische Phase wurde mit Wasser neutral gewaschen, getrocknet und eingedampft. Als Rückstand verblieben 25,5 g rohes öliges 2-n-Heptyl-1,2,3,4-tetrahydrochinolin.
C) 25,5 g des vorstehend erhaltenen Produktes wurden mit 9,2 g Natriumnitrit in wäßrigem salzsaurem Medium analog Beispiel 1A) umgesetzt. Das Reaktionsgemisch wurde wie in Beispiel 1A) beschrieben aufgearbeitet. Es wurden 25,1 g rohes, öliges 2-n-Heptyl-l-nitroso-1,2,3,4-tetrahydrochinolin erhalten.
D) 25 g des vorstehend erhaltenen Produktes wurden in Tetrahydrofuran mit 7,3 g Lithiumaluminiumhydrid wie in Beispiel 1B) beschrieben reduziert. Das Reaktionsgemisch wurde wie in Beispiel 1B) beschrieben aufgearbeitet. Es wurden 18,5 g rohes, öliges 1-Amino-2-n-heptyl-1,2,3,4-tetrahydrochinolin erhalten.
E) 18,5 g des vorstehenden Produktes wurden mit 13 g Lävulinsäureethylester in einem Gemisch aus 120 ml Eisessig und 7,2 ml 12 N Salzsäure 3 Stunden am Rückfluß auf 80 °C erhitzt. Zur Aufarbeitung wurde das Reaktionsgemisch abgekühlt, dann wurden organische Lösungsmittel abgedampft. Sodann wurden dem Reaktionsgemisch wäßrige Natriumhydroxidlösung und Ethylacetat zugegeben. Die organische Phase wurde abgetrennt, mit Wasser neutral gewaschen, getrocknet und eingeengt. Als Rückstand wurden 20 g öliges Rohprodukt erhalten. Dieser rohe Ester wurde in 100 ml Ethanol gelöst. Zu der Lösung wurde eine Lösung von 8 g Kaliumhydroxid in 100 ml Ethanol gegeben und das Reaktionsgemisch wurde zur Verseifung des Esters 2 Stunden am Rückfluß erhitzt. Anschließend wurde das Ethanol abgedampft, der Rückstand in Wasser gelöst, die wäßrige Phase mit Diethylether gewaschen, angesäuert und mit Essigsäureethylester extrahiert. Die Ethylacetatphase wurde mit Wasser gewaschen, getrocknet und eingedampft. Die als öliger Rückstand verbleibende rohe Säure wurde in 100 ml Ethanol unter Zugabe einiger Tropfen Schwefelsäure zur Wiederveresterung 2 Stunden am Rückfluß erhitzt. Anschließend wurde der überschüssige Alkohol abgedampft, der Rückstand in Wasser aufgenommen und mit Ethylacetat extrahiert. Die organische Phase wurde gewaschen, getrocknet und eingeengt. Es wurden 3,2 g 4-n-Heptyl-5,6-dihydro-2-methyl-4H-pyrrolo[3,2,1-ij]chinolin-1-essigsäureethylester erhalten.
F) 3,2 g des vorstehend erhaltenen Esters wurden mit 1 g Lithiumaluminiumhydrid in Tetrahydrofuran nach der in Beispiel 1D) beschriebenen Methode reduziert. Anschließend wurde das Reaktionsgemisch wie in Beispiel 1D) beschrieben aufgearbeitet. Es wurden 2,6 g rohes 4-n-Heptyl-5,6-dihydro-1-(2-hydroxyethyl)-2-methyl-4H-pyrrolo[3,2,1-ij]chinolin als Öl erhalten, welches langsam kristallisierte.
G) 2,6 g des vorstehend erhaltenen Produktes wurden mit 1,68 g Phosphortribromid in Chloroform nach der in Beispiel 1E) beschriebenen Methode umgesetzt. Das Reaktionsgemisch wurde wie in Beispiel 1E) beschrieben aufgearbeitet. Es wurden 2,7 g Rohprodukt erhalten. Dieses wurde durch Chromatographie über eine Kieselgelsäule unter Verwendung von Dichlormethan als Elutionsmittel gereinigt. Hierbei wurden 2 g 1-(2-Bromethyl)-4-n-heptyl-5,6-dihydro-2-methyl-4H-pyrrolo[3,2,1-ij]chinolin erhalten.
H) 2 g des vorstehend erhaltenen Produktes wurden mit 1,2 g 1-(4-Methylpyridin-2-yl)piperazin und 1,07 g Triethylamin in 25 ml Dimethylformamid 6 Stunden am Rückfluß erhitzt. Anschließend wurde das Reaktionsgemisch wie in Beispiel 1F) beschrieben aufgearbeitet. Es wurden 2 g Rohprodukt erhalten. Dieses wurde durch Chromatographie über eine Kiesel-gelsäule unter Verwendung von Dichlormethan als Elutionsmittel gereinigt. Es wurden 0,6 g 4-n-Heptyl-5,6-dihydro-2-methyl-1-{2-[4-(4-methylpyridin-2-yl)-piperazin-1-yl]-ethyl}-4H-pyrrolo[3,2,1-ij]chinolin erhalten. Dieses wurde nach der in Beispiel 1F) beschriebenen Methode in das Dihydrochlorid überführt. Nach Einengen der isopropanolischen Lösung wurden 0,45 g meringue-farbenes 4-n-Heptyl-5,6-dihydro-2-methyl-1-{2-[4-(4-methylpyridin-2-yl)-piperazin-1-yl]-ethyl}-4H-pyrrolo[3,2,1-ij]chinolin-dihydrochlorid mit einem Schmelzpunkt von 150 °C erhalten.

### Beispiel 5:

### 5,6-Dihydro-8-methoxy-2-methyl-1-{2-[4-(4-methylpyridin-2-yl)-piperazin-1-yl]-ethyl}-4H-pyrrolo [3,2,1-ij]chinolin.

A) 51 g 6-Methoxychinolin wurden in 500 ml Essigsäure gelöst. Zu der Lösung wurden tropfenweise 63,4 ml (= 58,37 g) Diboran/Pyridin-Komplex zugegeben, wobei kein Temperaturanstieg erfolgte. Das Reaktionsgemisch wurde insgesamt ca. 30 Stunden bei Raumtemperatur gerührt, wobei nach 7 Stunden und nach 27 Stunden nochmal je 32 ml Diboran/Pyridin-Komplex zugegeben wurden. Zur Aufarbeitung wurden dem Reaktionsgemisch unter Kühlung 750 ml wäßrige Natriumhydroxidlösung und 500 ml Wasser zugegeben und anschließend wurde zweimal mit je 500 ml Toluol extrahiert. Die organische Phase wurde eingeengt. Zur Zerstörung von restlichem Boran-Komplex wurde der Rückstand in 250 ml wäßriger 6 N Salzsäure aufgenommen und 12 Stunden in der Salzsäurelösung belassen. Anschließend wurden 250 ml 10-%ige wäßrige Natriumhydroxidlösung unter Abkühlung zugegeben, um das Reaktionsgemisch alkalisch zu stellen. Dann wurde mit Toluol extrahiert, die organische Phase abgetrennt, mit Wasser und Natriumchloridlösung neutral gewaschen, getrocknet und eingedampft. Es verblieben 36,6 g rohes 1,2,3,4-Tetrahydro-6-methoxychinolin als gelbliches Öl.
B) 36 g der vorstehend erhaltenen Verbindung wurden mit 18,2 g Natriumnitrit in wäßriger Salzsäurelösung nach der in Beispiel 1A) beschriebenen Methode umgesetzt und das Reaktionsgemisch wurde wie in Beispiel 1A) beschrieben aufgearbeitet. Es wurden 29,5 g rohes 6-Methoxy-1,2,3,4-tetrahydro-1-nitrosochinolin als rotes Öl erhalten.
C) 29,5 g des vorstehend erhaltenen Produktes wurden mit 11,65 g Lithiumaluminiumhydrid in Tetrahydrofuran nach der in Beispiel 1B) beschriebenen Methode reduziert. Das Reaktionsgemisch wurde wie in Beispiel 1B) beschrieben aufgearbeitet. Es wurden 26 g rohes 1-Amino-1,2,3,4-tetrahydro-6-methoxychinolin als bräunlich rotes Öl erhalten.
D) 26 g des vorstehend erhaltenen Produktes wurden mit 25,75 g Lävulinsäureethylester in einem Gemisch aus 220 ml Essigsäure und 13,3 ml 12 N Salzsäure 1 Stunde unter Rückfluß auf 80 °C erhitzt. Zur Aufarbeitung wurde das Reaktionsgemisch zur Entfernung organischer Lösungsmittel eingeengt und dann mit Wasser und Dichlormethan versetzt. Die organische Phase wurde abgetrennt und mit Wasser neutral gewaschen. Dann wurde die organische Phase zur Entfernung von Säure mit einer Mischung aus 10 ml konzentrierter Natronlauge und 200 ml Wasser gewaschen. Anschließend wurde die organische Phase mit Wasser neutral gewaschen, getrocknet und eingeengt. Als Rückstand verblieben 33,2 g dunkles, öliges, teilweise kristallisierendes Rohprodukt. Dieses wurde durch Chromatographie über eine kleine Kieselgelsäule unter Verwendung von Dichlormethan als Eluierungsmittel gereinigt. Hierbei wurden 22 g eines rot-orangen Öles erhalten, welches aus Hexan umkristallisiert wurde. Hierbei wurden 13,9 g 5,6-Dihydro-8-methoxy-2-methyl-4H-pyrrolo[3,2,1-ij]chinolin-1-essigsäureethylester als beigefarbenes Pulver erhalten.
E) 13,4 g des vorstehend erhaltenen Produktes wurden mit 3,4 g Lithiumaluminiumhydrid in Tetrahydrofuran nach der in Beispiel 1D) beschriebenen Methode reduziert. Das Reaktionsgemisch wurde wie in Beispiel 1D) beschrieben aufgearbeitet. Es wurden 10,7 g 1-(2-Hydroxyethyl)-8-methoxy-2-methyl-5,6-dihydro-4H-pyrrolo [3,2,1-ij]chinolin als hellgelbes Pulver erhalten.
F) 10,5 g des vorstehend erhaltenen Produktes wurden mit 11,6 g Phosphortribromid in Chloroform nach dem in Beispiel 1E) beschriebenen Verfahren umgesetzt. Das Reaktionsgemisch wurde wie in Beispiel 1E) beschrieben aufgearbeitet. Es wurden 13,1 g 1-(2-Bromethyl)-8-methoxy-2-methyl-5,6-dihydro-4H-pyrrolo [3,2,1-ij]chinolin als graues Pulver erhalten.
G) 13 g des vorstehend erhaltenen Produktes wurden mit 8,97 g 1-(4-Methylpyridin-2-yl)-piperazin, 11,8 ml Triethylamin und 0,7 g Kaliumjodid in 180 ml Dimethylformamid 3 Stunden unter Rückfluß auf eine Temperatur von 80 - 85 °C erhitzt. Das Reaktionsgemisch wurde wie in Beispiel 1F) beschrieben aufgearbeitet. Es wurden 10,1 g hellbeiges 8-Methoxy-2-methyl-1-{2-[4-(4-methylpyridin-2-yl)-piperazin-1-yl]-ethyl}-5,6-dihydro-4H-pyrrolo[3,2,1-ij]chinolin mit einem Schmelzpunkt von 104 °C erhalten.

### Beispiel 6:

### 8-Hydroxy-2-methyl-1-{2-[4-(4-methylpyridin-2-yl)-piperazin-1-yl]-ethyl}-5,6-dihydro-4H-pyrrolo[3,2,1-ij]chinolin.

7,9 g 8-Methoxy-2-methyl-1-{2-[4-(4-methylpyridin-2-yl)-piperazin-1-yl]-ethyl}-5,6-dihydro-4H-pyrrolo[3,2,1-ij]chinolin (Herstellung siehe Beispiel 5) wurden in 110 ml Dichlormethan gelöst. Die Lösung wurde auf -10 °C abgekühlt. Dann wurde eine Lösung von 24,42 g Bortribromid in 35 ml Dichlormethan zugegeben, wobei die Temperatur zwischen -10 und 0 °C gehalten wurde. Das Reaktionsgemisch wurde 30 Minuten bei ca. 0 °C reagieren gelassen. Zur Aufarbeitung wurde das Reaktionsgemisch auf eine Mischung aus Eis und gesättigter wäßriger Natriumbicarbonatlösung gegeben. Die organische Phase wurde von der alkalischen wäßrigen Phase abgetrennt, neutral gewaschen, getrocknet und eingedampft. Als Rückstand verblieben 9,3 g hellgraues, festes Rohprodukt, welches durch Chromatographie über eine kleine Kieselgelsäule gereinigt wurde. Es wurden 4,4 g cremefarbenes 8-Hydroxy-2-methyl-1-{2-[4-(4-methylpyridin-2-yl)-piperazin-1-yl]-ethyl}-5,6-dihydro-4H-pyrrolo[3,2,1-ij]chinolin mit einem Schmelzpunkt von 110 °C erhalten.

### Beispiel 7:

### 2-(4-Methoxyphenyl)-1-{2-[4-(4-methylpyridin-2-yl)-piperazin-1-yl]-ethyl}-5,6-dihydro-4H-pyrrolo[3,2,1-ij]chinolin.

A) 21 g 3-(4-Methoxybenzoyl)-propionsäure wurden in 350 ml Ethanol nach Zusatz von 7,3 ml konzentrierter Schwefelsäure 5 ¼ Stunden am Rückfluß erhitzt. Zur Aufarbeitung wurde das Reaktionsgemisch eingeengt, der verbleibende Rückstand in 100 ml Wasser aufgenommen und mit Dichlormethan extrahiert. Die organische Phase wurde abgetrennt, mit 50 ml gesättigter, wäßriger Natriumbicarbonatlösung gewaschen, anschließend mit Wasser neutral gewaschen, getrocknet und eingeengt. Es wurden 23,3 g roher 3-(4-Methoxybenzoyl)propionsäureethylester als gelbliches, kristallisierendes Öl erhalten.
B) 11 g 1-Amino-1,2,3,4-tetrahydrochinolin (Herstellung siehe Beispiel 1B) und 21,04 g des vorstehend erhaltenen Esters wurden in einem Gemisch aus 110 ml Essigsäure und 6,80 ml 12 N Salzsäure 3 Stunden unter Rückfluß erhitzt. Zur Aufarbeitung wurde das Reaktionsgemisch eingeengt. Es wurden 37,15 g eines roten Öles erhalten. Dieses wurde in Wasser aufgenommen und die wäßrige Phase mit Dichlormethan extrahiert. Die organische Phase wurde abgetrennt, mit 20 ml 10-%iger wäßriger Natriumhydroxidlösung gewaschen, anschließend mit Wasser gewaschen, getrocknet und eingedampft. Es wurden 25,5 g eines rohen öligen Gemisches aus 2-(4-Methoxyphenyl)-5,6-dihydro-4H-pyrrolo[3,2,1-ij]chinolin-1-essigsäure und deren Ethylester erhalten. Das Gemisch wurde in Isopropylether gegeben. Hierbei kristallisierten 6,4 g der Säure als ockerfarbenes Pulver aus und wurden abfiltriert. Das Filtrat wurde durch Säulenchromatographie an Kieselgel unter Verwendung von Toluol aufgetrennt. Dabei wurden 3,6 g des Esters und 1,3 g der Säure erhalten. Die insgesamt 7,7 g der Säure wurden mit Ethanol verestert, wobei 8 g des Esters erhalten wurden.
C) 11 g des vorstehend erhaltenen Esters wurden mit 2,39 g Lithiumaluminiumhydrid in Tetrahydrofuran wie in Beispiel 1D) beschrieben reduziert. Das Reaktionsgemisch wurde wie in Beispiel 1D) beschrieben aufgearbeitet. Es wurden 7,6 g rohes 1-(2-Hydroxyethyl)-2-(4-methoxyphenyl)-5,6-dihydro-4H-pyrrolo [3,2,1-ij]chinolin als bräunliches, kristallisierendes Öl erhalten.
D) 7,5 g des vorstehend erhaltenen Produktes wurden mit 4,95 g Phosphortribromid in Chloroform nach der in Beispiel 1E) beschriebenen Methode umgesetzt. Das Reaktionsgemisch wurde wie in Beispiel 1E) beschrieben aufgearbeitet. Es wurden 8 g 1-(2-Bromethyl)-2-(4-methoxyphenyl)-5,6-4H-pyrrolo [3,2,1-ij]chinolin als bräunliches Pulver erhalten.
E) 7,5 g des vorstehenden Produktes wurden mit 4,3 g 1-(4-Methylpyridin-2-yl)-piperazin, 4,10 g Triethylamin und 0,34 g Kaliumjodid in 50 ml Dimethylformamid 5 Stunden unter Rückfluß auf eine Temperatur von 85 - 90 °C erhitzt. Zur Aufarbeitung wurde das Reaktionsgemisch zur Trockene eingeengt, der verbleibende Rückstand in Dichlormethan gelöst, die Dichlormethanlösung zunächst mit Wasser, dann mit gesättigter, wäßriger Natriumbicarbonatlösung gewaschen und schließlich nochmals mit Wasser neutral gewaschen, getrocknet und eingeengt. Es wurden 10,07 g Rohprodukt erhalten, welches durch Chromatographie über eine Kieselgelsäule unter Verwendung von zunächst Toluol und anschließend Toluol/Ethanol 98:2 als Elutionsmittel gereinigt wurde. Es wurde ein gelbliches Öl erhalten, welches aus Isopropanol kristallisiert wurde. Nach Trocknen wurden 4,0 g 2-(4-Methoxyphenyl)-1-{2-[4-(4-methylpyridin-2-yl)-piperazin-1-yl]-ethyl}-5,6-dihydro-4H-pyrrolo [3,2,1-ij]chinolin mit einem Schmelzpunkt von 120 °C erhalten.

### Beispiel 8:

### 2-(4-Hydroxyphenyl)-1-{2-[4-(4-methylpyridin-2-yl) -piperazin-1-yl]-ethyl}-5,6-dihydro-4H-pyrrolo [3,2,1-ij]chinolin.

7,5 g 2-(4-Methoxyphenyl)-1-{2-[4-(4-methylpyridin-2-yl)-piperazin-1-yl]-ethyl}-5,6-dihydro-4H-pyrrolo[3,2,1-ij]chinolin (Herstellung siehe Beispiel 7) wurden in 110 ml Essigsäure gelöst. Zu der Lösung wurden 110 ml 41-%ige wäßrige Bromwasserstoffsäure gegeben und das Reaktionsgemisch wurde 16 Stunden am Rückfluß erhitzt. Zur Aufarbeitung wurde auf Raumtemperatur abgekühlt, dann wurde das Reaktionsgemisch mit 100 ml Wasser verdünnt und durch Zugabe von 10-%iger wäßriger Natriumhydroxidlösung neutralisiert (pH = 6). Anschließend wurde mit Dichlormethan extrahiert, die organische Phase abgetrennt, getrocknet und eingeengt. Der verbleibende ölige Rückstand wurde aus Ethanol/Ether kristallisiert. Es wurden 5,2 g 2-(4-Hydroxyphenyl)-1-{2-[4-(4-methylpyridin-2-yl)-piperazin-1-yl]-ethyl}-5,6-dihydro-4H-pyrrolo[3,2,1-ij]chinolin mit einem Schmelzpunkt von 220 °C erhalten.

### Beispiel 9:

### 8-Brom-2-methyl-1-{2-[4-(4-methylpyridin-2-yl)-piperazin-1-yl]-ethyl)-5,6-dihydro-4H-pyrrolo[3,2,1-ij]chinolin.

A) 5 g 1-(2-Bromethyl)-5,6-dihydro-2-methyl-4H-pyrrolo[3,2,1-ij]chinolin (Herstellung siehe Beispiel 1E) wurden in 20 ml Essigsäure gelöst. Zu der Lösung wurde eine Lösung von 3,4 g Brom in 10 ml Essigsäure gegeben und das Reaktionsgemisch wurde 2 Stunden bei Raumtemperatur gerührt. Zur Aufarbeitung wurde das Reaktionsgemisch auf Eis gegeben, durch Zugabe von 20-%iger wässriger Natriumhydroxidlösung neutralisiert und mit Dichlormethan extrahiert. Die organische Phase wurde mit Wasser neutral gewaschen, getrocknet und eingeengt. Als Rückstand wurden 6 g öliges Rohprodukt erhalten, welches durch Chromatographie über eine Kiesegelsäule unter Verwendung von Dichlormethan als Elutionsmittel gereinigt wurde. Es wurden 3,8 g 8-Brom-1-(2-bromethyl)-2-methyl-5,6-dihydro-4H-pyrrolo[3,2,1-ij]chinolin erhalten.
B) 3,8 g des vorstehend erhaltenen Produktes wurden mit 1,85 g 1-(4-Methylpyridin-2-yl)-piperazin in Dimethylformamid unter Zusatz von 1,86 g Triethylamin nach der in Beispiel 7E) beschriebenen Methode umgesetzt. Das Reaktionsgemisch wurde wie in Beispiel 7E) beschrieben aufgearbeitet. Es wurden 1,0 g öliges, rohes 8-Brom-2-methyl-1-{2-[4-(4-methylpyridin-2-yl)-piperazin-1-yl]-ethyl}-5,6-dihydro-4H-pyrrolo[3,2,1-ij]chinolin erhalten. Dieses wurde wie in Beispiel 1F) beschrieben in sein Trihydrochlorid überführt. Es wurden 500 mg des Trihydrochlorids der Titelverbindung mit einem Schmelzpunkt von 200 °C (Zersetzung) erhalten.

### Beispiel 10:

### 2-Methyl-8-nitro-1-{2-[4-(4-methylpyridin-2-yl)-piperazin-1-yl]-ethyl}-5,6-dihydro-4H-pyrrolo [3,2,1-ij]chinolin.

A) 5 g 1-(2-Bromethyl)-2-methyl-5,6-dihydro-4H-pyrrolo[3,2,1-ij]chinolin wurden in 10 ml auf 0 °C abgekühlte konzentrierte Schwefelsäure gegeben. Das Reaktionsgemisch wurde bei einer Temperatur von ca. 0 °C gehalten und es wurde tropfenweise eine Mischung aus 1,15 ml konzentrierter Schwefelsäure und 0,84 ml Salpetersäure als nitrierendes Agens zugegeben. Das Reaktionsgemisch wurde noch 1 Stunde bei 0 °C und eine weitere Stunde bei Raumtemperatur gehalten. Dann wurde zur Aufarbeitung auf 30 g Eis gegossen und mit Dichlormethan extrahiert. Die organische Phase wurde getrocknet und eingeengt. Der verbleibende Rückstand wurde in 10 ml Ethanol erhitzt und der gebildete gelbliche Niederschlag wurde abfiltriert. Es wurden 2,26 g 1-(2-Bromethyl)-2-methyl-8-nitro-5,6-dihydro-4H-pyrrolo[3,2,1-ij]chinolin als ockerfarbenes Pulver erhalten.
B) 3,1 g des vorstehend erhaltenen Produktes wurden mit 2,04 g 1-(4-Methylpyridin-2-yl)-piperazin in Dimethylformamid unter Zusatz von 2,69 ml Triethylamin und 0,16 g Kaliumjodid nach der in Beispiel 7E) beschriebenen Methode umgesetzt. Das Reaktionsgemisch wurde wie in Beispiel 7E) beschrieben aufgearbeitet. Es wurden 1,5 g rohes 2-Methyl-8-nitro-1-{2-[4-(4-methylpyridin-2-yl)-piperazin-1-yl]-ethyl}-4H-pyrrolo[3,2,1-ij]chinolin als bräunliches Öl erhalten. Dieses wurde wie in Beispiel 3H) beschrieben in das Hydrochlorid überführt. Es wurden 1,18 g 2-Methyl-8-nitro-1-{2-[4-(4-methylpyridin-2-yl)-piperazin-1-yl]-ethyl}-5,6-dihydro-4H-pyrrolo[3,2,1-ij]chinolin·1,6 HCl·1,5 H₂O mit einem Schmelzpunkt von 230 °C erhalten.

### Beispiel 11:

### 5-Methyl-6-{2-[4-(4-methylpyridin-2-yl)-piperazin-1-yl]-ethyl}-2,3-dihydro-pyrrolo [1,2,3-de]-1,4-benzothiazin.

A) 25,3 g Lithiumaluminiumhydrid wurden in 500 ml Tetrahydrofuran suspendiert und das Reaktionsgemisch wurde im Eisbad auf eine Temperatur von 15 - 20 °C gekühlt. Dann wurde eine Lösung von 33 g 2H,4H-1,4-Benzothiazin-2-on in 400 ml Tetrahydrofuran zugegeben und das Reaktionsgemisch wurde 1 Stunde am Rückfluß erhitzt. Anschließend wurde das Reaktionsgemisch nach der in Beispiel 1B) beschriebenen Methode aufgearbeitet. Es wurden 26 g 2H-3,4-Dihydro-1,4-benzothiazin erhalten.
B) 26 g des vorstehend erhaltenen Produktes wurden nach der in Beispiel 1A) beschriebenen Methode mit Natriumnitrit in wäßriger Salzsäurelösung umgesetzt und das Reaktionsgemisch wurde wie in Beispiel 1A) beschrieben aufgearbeitet. Es wurden 31 g 2H-3,4-Dihydro-4-nitroso-1,4-benzothiazin erhalten.
C) 31 g des vorstehend erhaltenen Nitrosoproduktes wurden mit 19,6 g Lithiumaluminiumhydrid in Tetrahydrofuran nach der in Beispiel 1B) beschriebenen Methode reduziert. Das Reaktionsgemisch wurde wie in Beispiel 1B) beschrieben aufgearbeitet. Es wurden 25,4 g öliges 4-Amino-2H-3,4-dihydro-1,4-benzothiazin erhalten.
D) 25,4 g des vorstehend erhaltenen Produktes wurden mit 26,3 g Lävulinsäureethylester nach der in Beispiel 1C) beschriebenen Methode umgesetzt. Das Reaktionsgemisch wurde wie in Beispiel 1C) beschrieben aufgearbeitet. Es wurden 9,7 g 5-Methyl-2,3-dihydro-pyrrolo[1,2,3-de]-1,4-benzothiazin-6-essigsäureethylester erhalten.
E) 4 g Lithiumaluminiumhydrid wurden in 150 ml Tetrahydrofuran suspendiert. Zu der Suspension wurde eine Lösung von 9,7 g des vorstehend erhaltenen Esters in 200 ml Tetrahydrofuran gegeben und das Reaktionsgemisch wurde 1 Stunde am Rückfluß erhitzt. Anschließend wurde das Reaktionsgemisch wie in Beispiel 1D) beschrieben aufgearbeitet. Es wurden 7,1 g 6-(2-Hydroxyethyl)-5-methyl-2,3-dihydro-pyrrolo[1,2,3-de]-1,4-benzothiazin erhalten.
F) 7 g des vorstehend erhaltenen Produktes wurden mit 6,09 g Phosphortribromid in Chloroform nach der in Beispiel 1E) beschriebenen Methode umgesetzt. Das Reaktionsgemisch wurde wie in Beispiel 1E) beschrieben aufgearbeitet. Es wurden 7,8 g 6-(2-Bromethyl)-5-methyl-2,3-dihydro-pyrrolo[1,2,3-de]-1,4-benzothiazin mit einem Schmelzpunkt von 94 °C erhalten.
G) 3,5 g des vorstehend erhaltenen Produktes wurden mit 2,32 g 1-(4-Methylpyridin-2-yl)-piperazin und 3,3 ml Triethylamin in 100 ml Toluol 14 Stunden am Rückfluß erhitzt, wobei nach 10 Stunden nochmals 1,5 ml Triethylamin zugefügt wurden.

Zur Aufarbeitung wurde das Reaktionsgemisch durch Zugabe von 200 ml wässriger Natriumbicarbonatlösung alkalisiert und mit Dichlormethan extrahiert. Das Dichlormethanextrakt wurde mit Wasser neutral gewaschen, getrocknet und eingedampft. Es wurden 1,4 g rohe Titelverbindung als Öl erhalten. Zur Überführung in das Hydrochlorid wurde die erhaltene rohe Titelbase in 50 ml Aceton gelöst und die Lösung wurde mit 6,3 ml 2,2 N isopropanolischer Salzsäurelösung versetzt. Das ausgefallene Hydrochlorid der Titelverbindung wurde abfiltriert und aus einem Isopropylalkohol/ Methanol-Gemisch umkristallisiert. Es wurden 0,9 g 5-Methyl-6-{2-[4-(4-methylpyridin-2-yl)-piperazin-1-yl]-ethyl}-2,3-dihydro-pyrrolo[1,2,3-de]-1,4-benzothiazin·2 HCl mit einem Schmelzpunkt von 240 °C erhalten.

### Beispiel 12:

### 2-[4-(4-Hydroxybutyloxy)-phenyl]-1-{2-[4-(4-methylpyridin-2-yl)-piperazin-1-yl]-ethyl}-5,6-dihydro-4H-pyrrolo[3,2,1-ij]chinolin.

A) In 50 ml trocknes Dimethylformamid wurden unter Stickstoffatmosphäre 0,25 g Natriumhydrid gegeben und die Lösung wurde auf eine Temperatur von 80 °C erhitzt. Dann wurde eine Lösung von 4 g 2-(4-Hydroxyphenyl)-1-{2-[4-(4-methylpyridin-2-yl)-piperazin-1-yl]-ethyl}-5,6-dihydro-4H-pyrrolo[3,2,1-ij]chinolin (Herstellung siehe Beispiel 8) in 75 ml Dimethylformamid zugefügt. Das Reaktionsgemisch wurde 10 Minuten gerührt, wobei es eine grünliche Färbung annahm. Sodann wurde eine Lösung von 1,6 g 4-Brombuttersäureethylester in 40 ml Dimethylformamid zugegeben und das Reaktionsgemisch wurde 30 Minuten lang auf 80 °C erhitzt. Zur Aufarbeitung wurde dem Reaktionsgemisch Wasser zugesetzt und dann wurde mit Dichlormethan extrahiert. Die organische Phase wurde abgetrennt, gewaschen, getrocknet und eingeengt. Der verbleibende Rückstand wurde chromatographisch über eine Kieselgelsäule gereinigt, wobei als Elutionsmittel Toluol verwendet wurde, dem bis zu 2 % ansteigende Mengen Ethanol zugesetzt wurden. Es wurden 1 g öliges 4-[4-[1-{2-[4-(4-Methylpyridin-2-yl)-piperazin-1-yl]-ethyl}-5,6-dihydro-4H-pyrrolo [3,2,1-ij]chinolin-2-yl]-phenyloxy]-buttersäureethylester erhalten.
B) Zu einer Lösung von 0,1 g Lithiumaluminiumhydrid in trocknem Tetrahydrofuran wurden 0,7 g des vorstehend erhaltenen Esters mit Hilfe einer Pipette unter Kühlung im Eisbad zugetropft, wobei die Reaktionsmischung sofort aufschäumte. Zur Aufarbeitung wurden dem Reaktionsgemisch zur Hydrolyse zunächst einige Tropfen Wasser, dann einige Tropfen 10-%ige Natriumhydroxydlösung und 15 ml Tetrahydrofuran zugesetzt. Sodann wurde über Zeolith filtriert, das Filtrat getrocknet und eingeengt. Es wurden 0,6 g Rohprodukt erhalten, welches durch Chromatographie über eine Kieselgelsäule unter Verwendung von zunächst Toluol und dann Toluol/Ethanol 99:1 als Elutionsmittel gereinigt wurde. Es wurden 250 mg 2-[4-(4-Hydroxybutyloxy)-phenyl]-1-{2-[4-(4-methylpyridin-2-yl)-piperazin-1-yl]-ethyl}-5,6-dihydro-4H-pyrrolo[3,2,1-ij]chinolin erhalten.

Zur Überführung in ihr Dihydrochlorid wurde die Titelbase in Isopropanol gelöst. Zu der Lösung wurde isopropanolische Salzsäure und anschließend Ether gegeben. Der gebildete Niederschlag wurde abfiltriert. Es wurden 250 mg 2-[4-(4-Hydroxybutyloxy)-phenyl]-1-{2-[4-(4-methylpyridin-2-yl)-piperazin-1-yl]-ethyl}-5,6-dihydro-4H-pyrrolo[3,2,1-ij]chinolin·2 HCl·2 H₂O mit einem Schmelzpunkt von 150 °C erhalten.

### Beispiel 13:

### 5,6-Dihydro-1-{2-[4-(4-methylpyridin-2-yl)-piperazin-1-yl]-ethyl}-4H-pyrrolo[3,2,1-ij]chinolin.

A) 17 g 1-Amino-1,2,3,4-tetrahydrochinolin (Herstellung siehe Beispiel 1B) und 20 g 2-Ketoglutarsäure wurden in einer Mischung aus 160 ml Essigsäure und 11,4 ml konzentrierter Salzsäure 2 Stunden am Rückfluß auf eine Temperatur von 80 °C erhitzt. Zur Aufarbeitung wurde der verbleibende feste Rückstand in Wasser aufgenommen und mit Essigsäureethylester extrahiert. Die organische Phase wurde abgetrennt und mit 20-%iger wäßriger Natriumhydroxidlösung extrahiert. Die alkalische (pH = 9) wäßrige Phase wurde abgetrennt und durch Zugabe von wäßriger 10-%iger Salzsäurelösung auf pH = 4 angesäuert, wobei sich ein Niederschlag bildete. Dieser wurde mit Essigsäureethylester extrahiert. Die organische Phase wurde eingedampft. Es wurden 10,6 g rohe 5,6-Dihydro-4H-pyrrolo [3,2,1-ij]chinolin-1-essigsäure als fester Rückstand erhalten.
B) 2,7 g der vorstehend erhaltenen Säure wurden in 150 ml Dichlormethan gelöst. Zu der Lösung wurden 3,2 g Carbonyldiimidazol gegeben, und das Reaktiongemisch wurde 1 Stunde am Rückfluß erhitzt. Dann wurden 2,63 g 1-(4-Methylpyridin-2-yl)-piperazin zugegeben und das Reaktionsgemisch weitere 3 Stunden am Rückfluß erhitzt. Zur Aufarbeitung wurde das Reaktionsgemisch mit 100 ml 10-%iger wäßriger Salzsäurelösung und zweimal mit Wasser gewaschen und anschließend mit 50 ml 10-%iger wäßriger Natriumhydroxidlösung gewaschen und mit Wasser bis zur Neutralität gewaschen. Dann wurde die organische Phase eingeengt. Es wurden 3,7 g rohes 5,6-Dihydro-1-{2-[4-(4-methylpyridin-2-yl)-piperazin-1-yl]-2-oxoethyl}-4H-pyrrolo [3,2,1-ij]chinolin als öliger Rückstand erhalten.
C) 0,8 g Lithiumaluminiumhydrid wurden in 30 ml wasserfreiem Tetrahydrofuran suspendiert. Zu der Suspension wurde eine Lösung von 3,7 g der vorstehend erhaltenen Amid-Verbindung in 50 ml Tetrahydrofuran zugegeben. Das Reaktionsgemisch wurde 1 Stunde am Rückfluß erhitzt. Zur Aufarbeitung wurde das Reaktionsgemisch abgekühlt und es wurden zur Hydrolyse 2 ml einer Mischung aus gleichen Teilen Wasser und Tetrahydrofuran, sodann 1 ml 15-%ige wäßrige Natriumhydroxidlösung und weitere 1 ml Wasser zugegeben. Sodann wurde von dem gebildetem Niederschlag abfiltriert und dieser nochmals mit Dichlormethan nachgewaschen. Die vereinigten Filtrate wurden eingeengt und der verbleibende Rückstand wurde in Dichlormethan gelöst. Die erhaltene Lösung wurde mit Wasser gewaschen, getrocknet und eingedampft. Es wurden 3 g rohes, öliges 5,6-Dihydro-1-{2-[4-(4-methylpyridin-2-yl)-piperazin-1-yl]-ethyl}-4H-pyrrolo [3,2,1-ij]chinolin erhalten.

Zur Überführung in ihr Dihydrochlorid wurde die vorstehend erhaltene Titelbase in Isopropanol gelöst. Der Lösung wurde isopropanolische 2,3 N Salzsäure zugesetzt, wobei das Dihydrochlorid der Titelverbindung auskristallisierte. Das erhaltene 5,6-Dihydro-1-{2-[4-(4-methylpyridin-2-yl)-piperazin-l-yl]-ethyl}-4H-pyrrolo [3,2,1-ij]chinolin-dihydrochlorid hatte nach Umkristallisation aus Ethanol einen Schmelzpunkt von 215 °C.

### Beispiel 14:

### 2-Ethyl-5,6-dihydro-1-{2-[4-(4-methylpyridin-2-yl)-piperazin-1-yl]-1-hydroxyethyl}-4H-pyrrolo[3,2,1-ij]chinolin.

A) 8,2 g 1-Amino-1,2,3,4-tetrahydrochinolin wurden in 20 ml Isopropanol gelöst. Zu der Lösung wurden 5,6 g (= 5,7 ml) Butan-2,3-dion gegeben und das Reaktionsgemisch wurde 2 Stunden am Rückfluß erhitzt. Sodann wurden 20 ml 2,5 molare isopropanolische Salzsäurelösung zugegeben und das Reaktionsgemisch weitere 5 Stunden am Rückfluß erhitzt. Zur Aufarbeitung wurde das Isopropanol abgedampft und der Rückstand mit Dichlormethan extrahiert. Die Dichlormethanphase wurde mit Wasser gewaschen, getrocknet und eingeengt. Es wurden 6 g öliges Rohprodukt erhalten, welches durch Chromatographie über eine kleine Kieselgelsäule unter Verwendung von Dichlormethan als Elutionsmittel gereinigt wurde. Hierbei wurden aus der ersten Eluatfraktion 2,4 g festes 2-Acetyl-5,6-dihydro-4H-pyrrolo [3,2,1-ij]chinolin erhalten.
B) 1 g des vorstehend erhaltenen Produktes wurden mit 1,4 ml Hydrazin (98-%ig) und 1 g Kaliumhydroxid in 14 ml Diethylenglycol 2 Stunden unter Rückfluß auf eine Temperatur von 170 °C erhitzt. Sodann wurden Wasser und Hydrazin abgedampft, wobei die Temperatur auf 190 °C anstieg, und das Reaktionsgemisch wurde weitere 2 Stunden bei dieser Temperatur gehalten. Zur Aufarbeitung wurde auf 100 °C abgekühlt und das Reaktionsgemisch auf Eis gegeben und mit Ethylacetat extrahiert. Der Ethylacetatextrakt wurde mit Wasser gewaschen, getrocknet und eingedampft. Es wurden 0,57 g rohes öliges 2-Ethyl-5,6-dihydro-4H-pyrrolo[3,2,1-ij]chinolin erhalten.
C) 1,63 g Oxalylchlorid wurden in 10 ml Diethylether gelöst. Zu der Lösung wurde eine Lösung von 2 g des vorstehend erhaltenen Produktes in 20 ml Diethylether bei einer Temperatur von 5 °C gegeben. Dann wurde das Reaktionsgemisch 3 Stunden am Rückfluß erhitzt. Nach Abkühlen auf 5 °C wurde eine Lösung von 2,3 g 1-(4-Methylpyridin-2-yl)-piperazin in 15 ml Tetrahydrofuran zugegeben und das Reaktionsgemisch wurde eine Stunde am Rückfluß erhitzt. Zur Aufarbeitung wurden 100 ml Wasser zugegeben und das Gemisch mit Dichlormethan extrahiert. Der Dichlormethanextrakt wurde getrocknet und eingeengt. Es wurden 3,3 g öliges Rohprodukt erhalten, welches durch Chromatographie über eine kleine Kieselgelsäule unter Verwendung von Dichlormethan/Ethanol als Elutionsmittel gereinigt wurde. Hierbei wurden 3 g 2-Ethyl-5,6-dihydro-1-{2-[4-(4-methylpyridin-2-yl)-piperazin-1-yl]-1,2-dioxoethyl}-4H-pyrrolo[3,2,1-ij]chinolin erhalten.
D) 0,3 g Lithiumaluminiumhydrid wurden in 20 ml Tetrahydrofuran suspendiert. Zu der Suspension wurde eine Lösung von 1 g des vorstehend erhaltenen Produktes in 20 ml Tetrahydrofuran gegeben. Dann wurde das Reaktionsgemisch 4 Stunden am Rückfluß erhitzt. Zur Aufarbeitung wurde das Reaktionsgemisch hydrolysiert, indem nacheinander 1 ml eines Tetrahydrofuran/Wasser-Gemisches, 0,5 ml einer 15%-igen wäßrigen Natriumhydroxidlösung und 0,5 ml Wasser zugegeben wurden. Dann wurde das Gemisch filtriert, das Filtrat wurde eingeengt, und der Rückstand wurde mit Dichlormethan extrahiert. Der Dichlormethanextrakt wurde mit Wasser gewaschen, getrocknet und eingeengt. Es wurden 800 mg 2-Ethyl-5,6-dihydro-1-{2-[4-(4-methylpyridin-2-yl)-piperazin-1-yl]-1-hydroxyethyl}-4H-pyrrolo[3,2,1-ij]chinolin mit einem Schmelzpunkt von 60 °C erhalten.

### Beispiel 15:

### 2-Ethyl-5,6-dihydro-1-{2-[4-(4-methylpyridin-2-yl)-piperazin-1-yl]-ethyl}-4H-pyrrolo [3,2,1-ij]chinolin.

0,32 g Lithiumaluminiumhydrid wurden in 20 ml Tetrahydrofuran suspendiert. Zu der Suspension wurde eine Lösung von 0,7 g 2-Ethyl-5,6-dihydro-1-{2-[4-(4-methyl-pyridin-2-yl)-piperazin-1-yl]-1,2-dioxoethyl}-4H-pyrrolo-[3,2,1,-ij]chinolin (Herstellung siehe Beispiel 14 C) in 20 ml Tetrahydrofuran gegeben. Das Reaktionsgemisch wurde 9 Stunden am Rückfluß erhitzt und anschließend wie in Beispiel 14 D) beschrieben aufgearbeitet. Es wurden 600 mg öliges Rohprodukt erhalten. Dieses wurde durch Chromatographie über eine Kieselgelsäule unter Verwendung von Toluol/ Ethanol 95 : 5 als Elutionsmittel gereinigt. Es wurden 300 mg 2-Ethyl-5,6-dihydro-1-{2-[4-(4-methylpyridin-2-yl)-piperazin-1-yl]-ethyl}-4H-pyrrolo[3,2,1-ij]chinolin als Öl erhalten.

### Beispiel 16:

### 5,6-Dihydro-2-hydroxymethyl-1-{2-[4-(4-methylpyridin-2-yl)-piperazin-1-yl]-ethyl}-4H-pyrrolo[3,2,1-ij]chinolin.

A) 30 g 2-Ketoglutarsäure wurden in 500 ml Ethanol unter Zusatz von 3 ml Schwefelsäure 3 Stunden am Rückfluß gekocht. Anschließend wurde das Ethanol abgedampft, der Rückstand mit Dichlormethan extrahiert, die Dichlormethanphase mit Wasser gewaschen, getrocknet und eingeengt. Es wurden 37 g öliger 2-Ketoglutarsäurediethylester erhalten.
B) 5,5 g 1-Amino-1,2,3,4-tetrahydrochinolin und 8,7 g 2-Ketoglutarsäurediethylester wurden in einem Gemisch aus 100 ml Essigsäure und 2 ml 12 N Salzsäure 1 Stunde am Rückfluß erhitzt. Zur Aufarbeitung wurde die Essigsäure abgedampft, sodann wurde dem Reaktionsgemisch 10-%ige wäßrige Natriumhydroxidlösung bis zur Erreichung von pH = 10 zugegeben, das Gemisch in 100 ml Wasser gegeben und mit Dichlormethan extrahiert. Die organische Phase wurde mit Wasser gewaschen, über Natriumsulfat getrocknet und eingeengt. Es wurden 12 g öliges Rohprodukt erhalten. Dieses wurde mittels Säulenchromatographie an Kieselgel unter leicht erhöhtem Druck (Flashchromatographie) unter Verwendung von Toluol/Ethanol 95:5 als Elutionsmittel gereinigt. Es wurden 9,7 g 2-Ethoxycarbonyl-5,6-dihydro-4H-pyrrolo [3,2,1-ij]chinolin-1-essigsäureethylester erhalten.
C) Zur Hydrolyse seiner Essigsäureethylestergruppe wurden 9,5 g des vorstehend erhaltenen Produktes in 50 ml Ethanol gelöst. Zu der Lösung wurden 28 ml einer ethanolischen Lösung von Kaliumhydroxid (enthaltend 3 g Kaliumhydroxid in 50 ml Ethanol) gegeben und das Reaktionsgemisch wurde 2 Stunden auf 60 °C erhitzt. Zur Aufarbeitung wurde das Ethanol abgedampft, der verbleibende Rückstand in Wasser gelöst und dreimal mit Diethylether gewaschen. Dann wurde die wäßrige Phase mit Salzsäure auf pH-Wert 3 angesäuert und mit Ethylacetat extrahiert. Die Ethylacetatphase wurde abgetrennt, dreimal mit Wasser gewaschen, getrocknet und eingeengt. Es wurden 3 g öliger Rückstand erhalten, dieser wurde durch Chromatographie an Kieselgel unter Verwendung von Dichlormethan/Ethanol 9:1 als Elutionsmittel gereinigt. Es wurden 1,7 g 2-Ethoxycarbonyl-5,6-dihydro-4H-pyrrolo[3,2,1-ij]chinolin-1-essigsäure als Öl erhalten, welches anschließend kristallisierte.
D) 1,7 g der vorstehend erhaltenen Säure wurden mit 1,3 g 1-(4-Methylpyridin-2-yl)-piperazin in Dichlormethan in Gegenwart von 1,54 g Carbonyldiimidazol wie in Beispiel 13B) beschrieben umgesetzt. Das Reaktionsgemisch wurde wie in Beispiel 13B) beschrieben aufgearbeitet. Es wurden 1,3 g öliges 2-Ethoxycarbonyl-5,6-dihydro-1-{2-[4-(4-methylpyri-din-2-yl)-piperazin-1-yl]-2-oxoethyl}-4H-pyrrolo[3,2,1-ij]chinolin erhalten.
E) 0,2 g Lithiumaluminiumhydrid wurden in 50 ml Tetrahydrofuran suspendiert. Zu der Suspension wurde eine Lösung von 1,3 g des vorstehend erhaltenen Produktes in 25 ml Tetrahydrofuran gegeben. Das Reaktionsgemisch wurde 1 Stunde bei Raumtemperatur reagieren gelassen. Anschließend wurde wie in Beispiel 13C) beschrieben aufgearbeitet und das erhaltene Rohprodukt durch Chromatographie an Kieselgel gereinigt. Es wurden 0,8 g 5,6-Dihydro-2-hydroxymethyl-1-{2-[4-(4-methylpyridin-2-yl)-piperazin-1-yl]-ethyl}-4H-pyrrolo[3,2,1-ij]chinolin erhalten.

Die erhaltene Titelbase wurde in Ethanol gelöst und zu der Lösung wurde eine Lösung von 0,5 g Fumarsäure in Ethanol zugegeben. Nach Einengen der Lösung wurden 800 mg 5,6-Dihydro-2-hydroxymethyl-1-{2-[4-(4-methylpyridin-2-yl)-piperazin-1-yl]-ethyl}-4H-pyrrolo[3,2,1-ij]chinolin-difumarat·1,5 H₂O mit einem Schmelzpunkt von 100 °C (Zersetzung) erhalten.

### Beispiel 17:

### 5,6-Dihydro-2-methyl-1-{3-[4-(4-methylpyridin-2-yl)-piperazin-1-yl]-propyl}-4H-pyrrolo[3,2,1-ij]chinolin.

A) Zu einer Lösung von 14 g 1-(2-Bromethyl)-5,6-dihydro-2-methyl-4H-pyrrolo[3,2,1-ij]chinolin in 100 ml Toluol wurde eine Lösung von 2,96 g Natriumcyanid in 60 ml Wasser gegeben und das Reaktionsgemisch wurde während 4 Stunden am Rückfluß erhitzt. Zur Aufarbeitung wurde das Reaktionsgemisch zur Trockne eingeengt, der Rückstand mit Wasser gewaschen und mit Dichlormethan extrahiert. Die Dichlormethanphase wurde abgetrennt, getrocknet und eingedampft. Es wurden 10,5 g rohes 1-(3-Cyanopropyl)-5,6-dihydro-2-methyl-4H-pyrrolo [3,2,1-ij]chinolin als bräunliches Öl erhalten.
B) 10,5 g des vorstehend erhaltenen Produktes wurden in 50 ml Essigsäure gelöst. Dann wurden 50 ml Wasser und 50 ml Schwefelsäure zugesetzt und das Reaktionsgemisch wurde 45 Minuten am Rückfluß erhitzt. Zur Aufarbeitung wurden unter Kühlung im Eisbad Wasser und konzentrierte Natriumhydroxidlösung zugesetzt. Dann wurde das Reaktionsgemisch mit Dichlormethan extrahiert, die Dichlormethanphase abgetrennt, getrocknet und eingeengt. Der Rückstand wurde in 200 ml 10-%iger Natriumhydroxidlösung aufgenommen und wiederum mit Dichlormethan extrahiert. Die wäßrige Phase wurde abgetrennt, mit verdünnter Salzsäure auf pH 4-5 angesäuert und nochmals mit Dichlormethan extrahiert. Die vereinigten Dichlormethanphasen wurden gewaschen, getrocknet und eingeengt und das als Rückstand verbleibende Rohprodukt wurde durch Säulenchromatographie über Kieselgel unter Verwendung von Toluol/Ethanol 95:5 als Elutionsmittel gereinigt. Es wurden 3 g 3-(5,6-Dihydro-2-methyl-4H-pyrrolo[3,2,1-ij]chinolin-1-yl)-propionsäure als Öl erhalten, welches kristallisierte.
C) 2,8 g der vorstehend erhaltenen Säure wurden mit 2,98 g Carbonyldiimidazol in 50 ml Dimethylformamid unter Stickstoffatmosphäre 2 Stunden auf 40 °C erhitzt. Dann wurden 2,45 g 1-(4-Methylpyridin-2-yl)-piperazin zugegeben und das Reaktionsgemisch wurde weitere 7 Stunden auf 40 °C erhitzt. Zur Aufarbeitung wurde das Dimethylformamid abgedampft, der Rückstand wurde in 100 ml Dichlormethan aufgenommen. Die Dichlormethanlösung wurde nacheinander mit 50 ml 10-%iger wäßriger Salzsäurelösung, 50 ml Wasser und 50 ml 10-%iger wäßriger Natriumhydroxidlösung gewaschen und anschließend mit Wasser neutral gewaschen, getrocknet und eingeengt. Es wurden 4,3 g eines orangefarbenen Öles als Rückstand erhalten. Dieses wurde in wenig Toluol heiß gelöst und zu der Lösung wurde tropfenweise Hexan bis zum Ausfallen eines Niederschlages gegeben. Der Niederschlag wurde abfiltriert und getrocknet. Es wurden 2,2 g 5,6-Dihydro-2-methyl-1-{3-[4-(4-methylpyridin-2-yl)-piperazin-1-yl]-3-oxopropyl}-4H-pyrrolo[3,2,1-ij]chinolin als dunkelrosa Pulver erhalten.
D) 1,5 g des vorstehend erhaltenen Produktes wurden mit 0,83 g Lithiumaluminiumhydrid in Tetrahydrofuran nach der in Beispiel 13C) beschriebenen Methode reduziert. Das Reaktionsgemisch wurde wie in Beispiel 13C) beschrieben aufgearbeitet. Es wurden 1,35 g rohes 5,6-Dihydro-2-methyl-1-{3-[4-(4-methylpyridin-2-yl)-piperazin-1-yl]-propyl}-4H-pyrrolo[3,2,1-ij]chinolin als gelbes Öl erhalten. Zur weiteren Reinigung wurde diese rohe Titelverbindung nochmals in Dichlormethan aufgenommen und die Dichlormethanlösung mit wäßriger Natriumbicarbonatlösung und anschließend mit Wasser gewaschen, getrocknet und eingeengt. Es wurden 0,7 g gereinigte Titelverbindung erhalten.

Die Titelbase wurde wie in Beispiel 3H) beschrieben in ihr Hydrochlorid überführt und kristallisierte als 5,6-Dihydro-2-methyl-1-{3-[4-(4-methylpyridin-2-yl)-piperazin-1-yl]-propyl}-4H-pyrrolo[3,2,1-ij]chinolin-dihydrochlorid·2,5 H₂O, Schmelzpunkt 175 °C.

### Beispiel 18:

### 5,6-Dihydro-4-hydroxymethyl-2-methyl-1-{2-[4-(4-methylpyridin2-yl)-piperazin-1-yl]-ethyl}-4H-pyrrolo[3,2,1-ij]chinolin.

A) 29,89 g Chinolin-2-carbonsäure wurden in 450 ml Ethanol unter Zusatz von 3,5 ml Schwefelsäure 5 ½ Stunden am Rückfluß erhitzt. Zur Aufarbeitung wurde das Reaktionsgemisch eingedampft, der Rückstand in Wasser aufgenommen und mit Dichlormethan extrahiert. Die organische Phase wurde mit wäßriger Natriumbicarbonatlösung gewaschen und anschließend mit Wasser neutral gewaschen, getrocknet und eingedampft. Es wurden 32,23 g roher Chinolin-2-carbonsäureethylester als grünes Öl erhalten.
B) 32 g des vorstehend erhaltenen Produktes wurden mit 20 g Natriumcyanoborhydrid in Essigsäure wie in Beispiel 4B) beschrieben reduziert. Das Reaktionsgemisch wurde wie in Beispiel 4B) beschrieben aufgearbeitet. Es wurden 22,56 g rohes 1,2,3,4-Tetrahydro-chinolin-2-carbonsäureethylester als gelboranges Öl erhalten.
C) 21 g des vorstehend erhaltenen Produktes wurden in ein Gemisch aus 17 ml 12 N Salzsäure und 50 g Eis gegeben und mit einer Lösung 8,47 g Natriumnitrit in 20 ml Wasser analog Beispiel 1A) umgesetzt. Das Reaktionsgemisch wurde wie in Beispiel 1A) beschrieben aufgearbeitet. Es wurden 21,31 g 1-Nitroso-1,2,3,4-tetrahydro-chinolin-2-carbonsäureethylester als bräunliches Öl erhalten.
D) 21 g des vorstehend erhaltenen Produktes wurden in Tetrahydrofuran mit 10,21 g Lithiumaluminiumhydrid wie in Beispiel 1B) beschrieben reduziert. Das Reaktionsgemisch wurde wie in Beispiel 1B) beschrieben aufgearbeitet. Es wurden 12,76 g 2-(1-Amino-1,2,3,4-tetrahydro-chinolin-2-yl)-ethanol als bräunlich oranges Öl erhalten.
E) 22 g des vorstehend hergestellten Produktes wurden mit 21,3 g Lävulinsäureethylester in einem Gemisch aus 183 ml Essigsäure und 11 ml 12 N Salzsäure 2 Stunden am Rückfluß auf 80 °C erhitzt. Zur Aufarbeitung wurde das Reaktionsgemisch eingedampft, der Rückstand in Wasser aufgenommen und mit Dichlormethan extrahiert. Die organische Phase wurde abgetrennt, mit 10-%iger wäßriger Natriumhydroxidlösung gewaschen und anschließend mit Wasser neutral gewaschen, getrocknet und eingeengt. Als Rückstand verblieben 34,42 g eines bräunlichen Öles. Dieses wurde durch Chromatographie über eine kleine Kieselgelsäule unter Verwendung von reinem Toluol als Elutionsmittel gereinigt. Es wurden 28,51 g roher 5,6-Dihydro-4-hydroxymethyl-2-methyl-4H-pyrrolo [3,2,1-ij]chinolin-1-essigsäureethylester erhalten.
F) 7,5 g des vorstehend erhaltenen Produktes wurden in einem Gemisch aus 50 ml Wasser und 10 ml Ethylalkohol mit 3 g Natriumhydroxid während 4 Stunden am Rückfluß erhitzt. Zur Aufarbeitung wurden dem Reaktionsgemisch 30 ml 20-%ige wäßrige Salzsäure zugesetzt und das Reaktionsgemisch wurde zweimal mit Dichlormethan extrahiert. Die vereinigten Dichlormethanphasen wurden mit Wasser gewaschen, getrocknet und eingeengt. Es wurden 4,5 g rohe 5,6-Dihydro-4-hydroxymethyl-2-methyl-4H-pyrrolo [3,2,1-ij]chinolin-1-essigsäure erhalten.
G) 0,5 g der vorstehend erhaltenen Säure wurden mit 0,4 g 1-(4-Methylpyridin-2-yl)-piperazin in Dichlormethan in Gegenwart von 0,38 g Carbonyldiimidazol wie in Beispiel 13B) beschrieben umgesetzt. Das Reaktionsgemisch wurde wie in Beispiel 13B) beschrieben aufgearbeitet. Es wurden 200 mg öliges 5,6-Dihydro-4-hydroxymethyl-2-methyl-1-{2-[4-(4-methylpyridin-2-yl)-piperazin-1-yl]-2-oxoethyl}-4H-pyrrolo [3,2,1-ij]chinolin erhalten.
H) 0,36 g Lithiumaluminiumhydrid wurden in 20 ml wasserfreiem Tetrahydrofuran suspendiert. Zu der Suspension wurde eine Lösung von 2 g des vorstehend hergestellten Produktes in 20 ml Tetrahydrofuran tropfenweise zugegeben. Anschließend wurde das Reaktionsgemisch 1 Stunde bei Raumtemperatur gerührt. Das Reaktionsgemisch wurde wie in Beispiel 13B) beschrieben aufgearbeitet. Es wurden 1,8 g rohes, öliges 5,6-Dihydro-4-hydroxymethyl-2-methyl-1-{2-[4-(4-methylpyridin-2-yl)-piperazin-1-yl]-ethyl}-4H-pyrrolo[3,2,1-ij]chinolin erhalten.

Zur Überführung in ihr Fumarat wurden 1 g der vorstehend erhaltenen Titelbase in wenig Ethanol gelöst und die Lösung wurde mit einer Lösung von 0,6 g Fumarsäure in Ethanol versetzt. Das Reaktionsgemisch wurde zur Trockene eingedampft und der Rückstand wurde in Diethylether aufgenommen, der gebildete Niederschlag abfiltriert und getrocknet. Es wurden 0,5 g 5,6-Dihydro-4-hydroxymethyl-2-methyl-1-{2-[4-(4-methylpyridin-2-yl)-piperazin-1-yl]-ethyl}-4H-pyrrolo [3,2,1-ij]chinolin-1,5-fumarat mit einem Schmelzpunkt von 110 °C erhalten.

### Beispiel 19:

### 5,6-Dihydro-8-(1-hydroxy-2-methylpropyl)-2-methyl-1-{2-[4-(4-methylpyridin-2-yl)-piperazin-1-yl]-ethyl}-4H-pyrrolo[3,2,1-ij]chinolin.

A) 63 g Aluminiumtrichlorid wurden in 150 ml Chloroform bei einer Temperatur von 10 °C suspendiert. Zu der Suspension wurde ein Gemisch aus 50 g 5,6-Dihydro-2-methyl-4H-pyrrolo[3,2,1-ij]chinolin-1-essigsäureethylester und 26,3 g Isobuttersäurechlorid in 150 ml Chloroform zugegeben, wobei die Temperatur auf 10 °C gehalten wurde. Das Reaktionsgemisch wurde 1 Stunde bei Raumtemperatur gerührt, anschließend 4 Stunden am Rückfluß erhitzt und weitere 12 Stunden bei Raumtemperatur gehalten. Sodann wurden nochmals 10 g Aluminiumchlorid zugesetzt und es wurde eine weitere Stunde am Rückfluß erhitzt. Zur Aufarbeitung wurde das Reaktionsgemisch auf Eis gegossen und mit Dichlormethan extrahiert. Die organische Phase wurde mit verdünnter wäßriger Natriumhydroxidlösung gewaschen und anschließend mit Wasser neutral gewaschen, getrocknet und eingeengt. Es wurden 80 g Rohprodukt erhalten, welches durch Chromatographie über eine Kieselgelsäule unter Verwendung von Dichlormethan als Elutionsmittel gereinigt wurde. Es wurden 42,5 g 5,6-Dihydro-8-(2-methyl-1-oxopropyl)-2-methyl-4H-pyrrolo[3,2,1-ij]chinolin-1-essigsäureethylester erhalten.
B) 37,5 g des vorstehend erhaltenen Esters wurden in einer Mischung aus 300 ml Ethanol und 60 ml Wasser mit 13,7 g Natriumhydroxid 1 Stunde am Rückfluß erhitzt. Zur Aufarbeitung wurde der Alkohol aus dem Reaktionsgemisch abgedampft, der sich bildende Niederschlag in Wasser aufgenommen und die wäßrige Phase dreimal mit Dichlormethan gewaschen. Dann wurde die wäßrige Phase durch Zusatz von verdünnter Salzsäure auf einen pH-Wert von 2 angesäuert. Der gebildete Niederschlag wurde in Dichlormethan gelöst, die Dichlormethanphase wurde gewaschen, getrocknet und eingeengt. Es wurden 40 g festes Rohprodukt erhalten, welches aus Toluol kristallisiert wurde. Es wurden 22 g 5,6-Dihydro-8-(2-methyl-1-oxopropyl)-2-methyl-4H-pyrrolo[3,2,1-ij]chinolin-1-essigsäure mit einem Schmelzpunkt von 162 °C erhalten.
C) 22 g der vorstehend erhaltenen Säure wurden mit 14,4g 1-(4-Methylpyridin-2-yl)-piperazin in Dichlormethan in Gegenwart von 17 g Carbonyldiimidazol wie in Beispiel 13B) beschrieben umgesetzt. Das Reaktionsgemisch wurde wie in Beispiel 13B) beschrieben aufgearbeitet. Es wurden 34 g öliges 5,6-Dihydro-8-(2-methyl-1-oxopropyl)-2-methyl-1-{2-[4-(4-methylpyridin-2-yl)-piperazin-1-yl]-2-oxoethyl}-4H-pyrrolo[3,2,1-ij]chinolin erhalten, welches aus Cyclohexan kristallisiert wurde, Schmelzpunkt 120 °C.

7 g Lithiumaluminiumhydrid wurden in 200 ml Tetrahydrofuran suspendiert. Zu der Suspension wurde eine Lösung von 34 g des vorstehend erhaltenen Produktes in 100 ml Tetrahydrofuran bei einer Temperatur von 15 - 20 °C zugegeben. Das Reaktionsgemisch wurde 1 Stunde bei Raumtemperatur gerührt und anschließend 1 Stunde am Rückfluß erhitzt. Anschließend wurde wie in Beispiel 13C) beschrieben aufgearbeitet. Es wurden 30,5 g öliges Rohprodukt erhalten, welches aus Diisopropylether umkristallisiert wurde. Es wurden 22 g 5,6-Dihydro-8-(2-methyl-1-hydroxypropyl)-2-methyl-1-{2-[4-(4-methylpyridin-2-yl)-piperazin-1-yl]-ethyl}-4H-pyrrolo [3,2,1-ij]chinolin mit einem Schmelzpunkt von 140 °C erhalten.

0,6 g der vorstehend erhaltenen Titelbase wurden wie in Beispiel 18H) beschrieben in das entsprechende 1,2-Fumarat mit einem Schmelzpunkt von 130 °C überführt.

### Beispiel 20:

### 8-Amino-5,6-dihydro-2-methyl-1-{2-[4-(4-methylpyridin-2-yl)-piperazin-1-yl]-ethyl}-4H-pyrrolo [3,2,1-ij]chinolin.

A) 20 g 5, 6-Dihydro-2-methyl-4H-pyrrolo[3,2,1-ij]chinolin-1-essigsäure (hergestellt durch Hydrolyse ihres nach Beispiel 1C) erhaltenen Ethylesters) wurden vorsichtig zu 50 ml auf 0 °C abgekühlter Schwefelsäure gegeben. Dann wurde vorsichtig die Nitriersäure (= Mischung aus 5,56 ml konzentrierter Schwefelsäure und 4,04 ml konzentrierter Salpetersäure) zugefügt. Das Reaktionsgemisch wurde 1 Stunde bei einer Temperatur von 0 °C und 2 Stunden bei Raumtemperatur reagieren gelassen. Zur Aufarbeitung wurde das Reaktionsgemisch auf 200 g Eis gegeben, wobei sich ein Niederschlag bildete. Das Reaktionsgemisch wurde mit Dichlormethan extrahiert, wobei ein Teil des gebildeten Niederschlages in der Dichlormethanphase gelöst wurde. Die Dichlormethanphase wurde abgetrennt, der restliche in der wäßrigen Phase verbliebene Niederschlag wurde abfiltriert, mit Wasser gewaschen und getrocknet. Es wurden 7,54 g 5,6-Dihydro-2-methyl-8-nitro-4H-pyrrolo[3,2,1-ij]chinolin-1-essigsäure als grüngelbes Pulver erhalten. Die Dichlormethanphase wurde mit Wasser gewaschen, getrocknet und eingeengt. Der verbleibende Rückstand, welcher weitere Säure neben öligen Verunreinigungen enthielt, wurde zur Entfernung der öligen Verunreinigungen mit ca. 25 ml Ethanol behandelt und filtriert. So wurden weitere 3,23 g der Säure erhalten, so daß die Gesamtausbeute 10,8 g betrug.
B) 27,4 g der vorstehend hergestellten Säure wurden in 500 ml Dimethylformamid gelöst. Zu der Lösung wurden 25 g Carbonyldiimidazol gegeben und das Reaktionsgemisch wurde 2 Stunden auf 50 °C erwärmt. Anschließend wurden 21,3 g 1-(4-Methylpyridin-2-yl)-piperazin zugegeben, und das Reaktionsgemisch wurde weitere 4 Stunden auf 50 °C erhitzt. Zur Aufarbeitung wurde das Dimethylformamid aus dem Reaktionsgemisch abgedampft, der Rückstand wurde in 400 ml Dichlormethan gelöst und die Lösung wurde zunächst mit 250 ml 10-%iger wäßriger Salzsäurelösung, dann mit 250 ml Wasser und dann mit 250 ml 10-%iger wäßriger Natriumhydroxidlösung gewaschen und schließlich mit Wasser neutral gewaschen, getrocknet und eingeengt. Das als Rückstand verbleibende Rohprodukt wurde aus Ethanol kristallisiert. Es wurden 28 g 5,6-Dihydro-2-methyl-8-nitro-1-{2-[4-(4-methylpyridin-2-yl)-piperazin-1-yl]-2-oxoethyl}-4H-pyrrolo[3,2,1-ij]chinolin als ockerfarbenes Pulver erhalten.
C) 3 g des vorstehend erhaltenen Produktes wurden in ein Gemisch aus 200 ml Ethanol und 100 ml Methanol gegeben. Dann wurden 0,5 g eines Palladium/Kohle-Katalysators (10 % Palladium auf Kohle) zugefügt und das Reaktionsgemisch wurde bei einer Temperatur von 50 °C mit einem Wasserstoffdruck von 3 - 4 bar während 7 Stunden hydriert. Anschließend wurde vom Katalysator abfiltriert und zur Trockene eingedampft. Es wurden 3,3 g rohes 8-Amino-5,6-dihydro-2-methyl-1-{2-[4-(4-methylpyridin-2-yl)-piperazin-1-yl]-2-oxoethyl}-4H-pyrrolo[3,2,1-ij]chinolin als dunkelbraunes Pulver erhalten.
   Die Nitroverbindung kann statt durch katalytische Hydrierung auch durch Umsetzen mit Natriumborhydrid in Gegenwart eines Palladium/Kohle-Katalysators in Tetrahydrofuran reduziert werden.
D) 0,47 g Lithiumaluminiumhydrid wurden in 80 ml auf eine Temperatur von 0 - 5 °C gekühltes Tetrahydrofuran gegeben. Das Reaktionsgemisch wurde sich auf Raumptemperatur erwärmen gelassen. Dann wurde eine Lösung von 2,5 g des vorstehend erhaltenen Produktes in Tetrahydrofuran zugegeben. Das Reaktionsgemisch wurde 1 Stunde bei Raumtemperatur reagieren gelassen. Anschließend wurde wie in Beispiel 13C) beschrieben aufgearbeitet und das erhaltene Rohprodukt wurde durch Chromatographie über eine Kieselgelsäule unter Verwendung von Toluol/Ethanol 9:1 als Elutionsmittel gereinigt. Es wurden 0,8 g 8-Amino-5,6-dihydro-2-methyl-1-{2-[4-(4-methylpyridin-2-yl)-piperazin-1-yl]-ethyl}-4H-pyrrolo-[3,2,1-ij]chinolin als bräunlichrotes Öl erhalten.

Die vorstehend erhaltene Titelbase wurde in Isopropanol gelöst und die Lösung wurde mit 3,5-molarer isopropanolischer Salzsäurelösung versetzt. Es wurden 0,8 g 8-Amino-5,6-dihydro-2-methyl-1-{2-[4-(4-methylpyridin-2-yl)-piperazin-1-yl]-ethyl}-4H-pyrrolo[3,2,1-ij]chinolin·2,8 HCl·2 H₂O als graues Pulver mit einem Schmelzpunkt von 250 °C (Zersetzung) erhalten.

### Beispiel 21:

### 5,6-Dihydro-2-methyl-8-(2-methylpropyl)-1-{2-[4-(4-methylpyridin-2-yl)-piperazin-1-yl]-ethyl}-4H-pyrrolo[3,2,1-ij]chinolin.

A) 3,2 g 5,6-Dihydro-8-(2-methyl-1-oxypropyl)-2-methyl-4H-pyrrolo [3,2,1-ij] chinolin-1-essigsäureethylester (Herstellung siehe Beispiel 19A) wurden in 40 ml Diethylenglycol gelöst. Zu der Lösung wurden 2,6 g Kaliumhydroxid gegeben und das Reaktionsgemisch wurde zum Lösen des Kaliumhydroxid 1 Stunde auf 80 °C erhitzt. Dann wurden 3,2 ml Hydrazin (98-%ig) zugegeben und das Reaktionsgemisch wurde 2 Stunden auf eine Temperatur von 140 - 150 °C erhitzt. Sodann wurden Wasser und Hydrazin abgedampft, wobei die Temperatur auf 210 °C anstieg, und das Reaktionsgemisch wurde weitere 3 Stunden bei dieser Temperatur gehalten. Das Reaktionsgemisch wurde wie in Beispiel 14B) beschrieben aufgearbeitet. Es wurden 3 g feste 5,6-Dihydro-2-methyl-8-(2-methylpropyl)-4H-pyrrolo[3,2,1-ij]chinolin-1-essigsäure erhalten.
B) 2,9 g der vorstehend erhaltenen Säure wurden in Dichlormethan mit 1,98 g 1-(4-Methylpyridin-2-yl)-piperazin in Gegenwart von 2,4 g Carbonyldiimidazol wie in Beispiel 13B) beschrieben umgesetzt. Das Reaktionsgemisch wurde wie in Beispiel 13B) beschrieben aufgearbeitet. Es wurden 3,2 g 5,6-Dihydro-2-methyl-8-(2-methylpropyl)-1-{2-[4-(4-methylpyridin-2-yl)-piperazin-1-yl]-2-oxoethyl}-4H-pyrrolo[3,2,1-ij]chinolin erhalten.
C) 0,6 g Lithiumaluminiumhydrid wurden in 20 ml Tetrahydrofuran bei einer Temperatur von 0 - 5 °C suspendiert. Zu der Suspension wurde eine Lösung von 3,2 g des vorstehend erhaltenen Produktes in 10 ml Tetrahydrofuran zugegeben. Das Reaktionsgemisch wurde 1 Stunde bei Raumtemperatur gerührt und anschließend 2 Stunden unter Rückfluß erhitzt. Anschließend wurde wie in Beispiel 13C) beschrieben aufgearbeitet. Es wurden 2,7 g öliges 5,6-Dihydro-2-methyl-8-(2-methylpropyl)-1-{2-[4-(4-methylpyridin-2-yl)-piperazin-1-yl]-ethyl}-4H-pyrrolo[3,2,1-ij]chinolin erhalten.

Die Titelbase wurde wie in Beispiel 1H) beschrieben in ihr Dihydrochlorid überführt. Dieses wurde aus Isopropanol/Ethanol umkristallisiert. Es wurde 1 g des Dihydrochlorides der Titelverbindung mit einem Schmelzpunkt von 228 °C erhalten.

### Beispiel 22:

### 5,6-Dihydro-2-methyl-1-{2-[4-(4-methylpyridin-2-yl)-piperazin-1-yl]-ethyl}-4H-pyrrolo[3,2,1-ij]chinolin.

A) 20 g 1- (2-Bromethyl) -5, 6-dihydro-2-methyl-4H-pyrrolo-[3,2,1-ij]chinolin, 10,15 g 1-Acetylpiperazin, 1,2 g Kaliumjodid und 20,2 ml Triethylamin wurden in 300 ml Dimethylformamid während 5 Stunden auf eine Temperatur von 80 - 85 °C erhitzt. Zur Aufarbeitung wurde das Reaktionsgemisch eingeengt und der Rückstand in Dichlormethan aufgenommen. Die Dichlormethanphase wurde mit 10-%iger wäßriger Natriumhydroxidlösung gewaschen, mit Wasser neutral gewaschen, getrocknet und eingeengt. Es wurden 22,3 g braunes öliges Rohprodukt erhalten. Dieses wurde durch Chromatographie über eine kleine Kieselgelsäule unter Verwendung von Dichlormethan/Ethanol 95:5 als Elutionsmittel gereinigt und aus Diisopropylether kristallisiert. Es wurden 8,3 g 5,6-Dihydro-2-methyl-1-[2-(4-acetylpiperazin-1-yl)-ethyl]-4H-pyrrolo[3,2,1-ij]chinolin erhalten.
B) 8 g des vorstehend erhaltenen Produktes wurden in 20,5 ml wäßriger 6 N Salzsäure 45 Minuten am Rückfluß erhitzt. Zur Aufarbeitung wurde das Reaktionsgemisch abgekühlt und durch Zugabe von wäßriger 10-%iger Natriumhydroxidlösung neutralisiert. Dann wurde mit Dichlormethan extrahiert, die Dichlormethanphase abgetrennt, mit Wasser neutral gewaschen, getrocknet und eingedampft. Es wurden 8,85 g Rohprodukt als braunes Öl erhalten. Dieses wurde mit Diethylether behandelt. Hierbei wurden 2 g reines und 4,5 g öliges, nur wenig verunreinigtes 5,6-Dihydro-2-methyl-1-[2-(piperazin-1-yl-ethyl]-4H-pyrrolo [3,2,1-ij]chinolin erhalten.
c) 115,5 g 2-Amino-4-methylpyridin wurden über einen Zeitraum von 20 Minuten verteilt in 553 ml 48-%ige wäßrige auf 10 - 15 °C abgekühlte Bromwasserstoffsäurelösung gegeben. Das Reaktionsgemisch wurde auf eine Temperatur von 0 - 5 °C abgekühlt und dann wurden 161 ml Brom über 1 ½ Stunden verteilt, zugefügt, wobei sich ein orangefarbener Niederschlag bildete. Dem Reaktionsgemisch wurden nochmals 100 ml 48-%ige wäßrige Bromwasserstoffsäure zugesetzt und dann wurde unter Beibehaltung einer Temperatur von 0 °C eine Lösung von 189 g Natriumnitrit in 280 ml Wasser über einen Zeitraum von 3 Stunden verteilt, zugegeben. Hierbei trat eine völlige Lösung des gebildeten Niederschlages ein. Das Reaktionsgemisch wurde weitere 12 Stunden bei Raumtemperatur stehengelassen. Dann wurden 500 g Natriumhydroxid in 700 ml Wasser über einen Zeitraum von 3 Stunden verteilt zugegeben, wobei eine Temperatur von 20 °C nicht überschritten wurde. Zur Aufarbeitung wurde das Reaktionsgemisch einmal mit 400 ml und dreimal mit je 200 ml Diethylether extrahiert. Die vereinigten Etherphasen wurden gewaschen, getrocknet und eingeengt. Es wurden 171,7 g rohes, öliges 2-Brom-4-methylpyridin erhalten.
   Zur Reinigung wurde das rohe 2-Brom-4-methylpyridin mit isopropanolischer 2,5 N Salzsäurelösung in sein Hydrochlorid überführt. Dieses wurde abfiltriert und in 600 ml Methanol suspendiert. Durch die Suspension wurde Ammoniak geleitet. Anschließend wurde das Reaktionsgemisch filtriert, eingeengt, der Rückstand in Dichlormethan aufgenommen, die Dichlormethanphase filtriert und wieder eingeengt. Es wurden 153,4 g 2-Brom-4-methylpyridin als bräunliches Öl erhalten.
D) 3 g 5,6-Dihydro-2-methyl-1[2-(piperazin-1-yl)-ethyl]-4H-pyrrolo[3,2,1-ij]chinolin, 1,45 g 2-Brom-4-methylpyridin, 0,175 g Kaliumjodid und 3 ml Triethylamin wurden in 50 ml Dimethylformamid 7 Stunden auf eine Temperatur von 85 °C erhitzt. Das Reaktionsgemisch wurde wie in Beispiel 1H) beschrieben aufgearbeitet und die erhaltene rohe Titelbase wurde in das 5,6-Dihydro-2-methyl-1-{2-[4-(4-methylpyridin-2-yl)-piperazin-1-yl]-ethyl}-4H-pyrrolo[3,2,1-ij]chinolin-trihydrochlorid mit einem Schmelzpunkt von 254 °C überführt.

### Beispiel 23:

### 4-n-Butyl-5,6-dihydro-8-(2-methyl-1-oxopropyl)-2-methyl-1-(2-[4-(4-methylpyridin-2-yl)-piperazin-1-yl]-ethyl}-4H-pyrrolo [3,2,1-ij]chinolin.

0,76 g Aluminiumchlorid wurden in 20 ml Chloroform suspendiert. Zu der Suspension wurde eine Lösung von 1 g 4-n-Butyl-5,6-dihydro-2-methyl-1-{2-[4-(4-methylpyridin-2-yl)-piperazin-l-yl]-ethyl}-4H-pyrrolo[3,2,1-ij]chinolin (siehe Beispiel Nr. 73) und 0,29 g Isobuttersäurechlorid in 10 ml Chloroform bei einer Temperatur von 5 °C gegeben. Das Reaktionsgemisch wurde 1 Stunde bei Raumtemperatur gerührt. Dann wurde das Reaktionsgemisch wie in Beispiel 19A) beschrieben aufgearbeitet. Die rohe Titelverbindung wurde durch Chromoatographie über eine Kieselgelsäule unter Verwendung von Dichlormethan/Ethanol als Elutionsmittel gereinigt. Es wurden 0,65 g der Titelverbindung erhalten. Die Titelbase wurde zur Überführung in ihr Hydrochlorid in Isopropanol gelöst und die Lösung mit isopropanolischer Salzsäure versetzt. Das Reaktionsgemisch wurde eingeengt, der Rückstand in Ether suspendiert und filtriert. Es wurden 400 mg 4-n-Butyl-5,6-dihydro-8-(2-methyl-1-oxopropyl)-2-methyl-1-{2-[4-(4-methylpyridin-2-yl)-piperazin-1-yl]-ethyl}-4H-pyrrolo[3,2,1-ij]chinolin·1,9HCl·1,2 H₂O mit einem Schmelzpunkt von 150 °C erhalten.

### Beispiel 24:

### 8-Acetoxy-5,6-dihydro-2-methyl-1-{2-[4-(4-methylpyridin-2-yl)-piperazin-1-yl]-ethyl}-4H-pyrrolo[3,2,1-ij]chinolin.

2,2 g 5,6-Dihydro-2-methyl-8-hydroxy-1-{2-[4-(4-methylpyridin-2-yl)-piperazin-1-yl]-ethyl}-4H-pyrrolo[3,2,1-ij]chinolin (Herstellung siehe Beispiel 6), 0,884 g Acetylchlorid und 0,78 ml Triethylamin wurden in 15 ml Toluol 5 Stunden am Rückfluß erhitzt. Zur Aufarbeitung wurde das Reaktionsgemisch in 50 ml 10-%ige wäßrige Natriumhydroxidlösung gegeben. Die Toluolphase wurde abgetrennt und mit Wasser neutral gewaschen und eingeengt. Es wurden 2 g Rohprodukt erhalten, welches durch Chromatographie über eine kleine Kieselgelsäule unter Verwendung von erst Dichlormethan und dann Dichlormethan/Ethanol 98:2 gereinigt wurde. Es wurden 1,8 g ölige Titelverbindung erhalten. Diese wurde wie in Beispiel 1H) beschrieben in ihr Hydrochlorid überführt. Es wurden 1,72 g 8-Acetoxy-5,6-dihydro-2-methyl-1-{2-[4-(4-methylpyridin-2-yl)-piperazin-1-yl]-ethyl}-4H-pyrrolo[3,2,1-ij]chinolin·2 HCl·2,5 H₂O mit einem Schmelzpunkt von 220 °C erhalten.

### Beispiel 25:

### 8-Benzoylamino-5,6-dihydro-2-methyl-1-{2-[4-(4-methylpyridin-2-yl)-piperazin-1-yl]-ethyl}-4H-pyrrolo[3,2,1-ij]chinolin.

2 g 8-Amino-5,6-dihydro-2-methyl-1-{2-[4-(4-methylpyridin-2-yl)-piperazin-1-yl]-ethyl}-4H-pyrrolo[3,2,1-ij]chinolin (Herstellung siehe Beispiel 20) wurden in 25 ml Chloroform gelöst. Zu der Lösung wurden 1,45 ml Triethylamin und dann tropfenweise 1,075 g Benzoylchlorid zugesetzt ohne dabei eine Temperatur von 20 °C zu überschreiten. Das Reaktionsgemisch wurde 1 Stunde bei Raumtemperatur reagieren gelassen. Zur Aufarbeitung wurde das Reaktionsgemisch mit 20 ml 10-%iger wäßriger Natriumhydroxidlösung neutral gewaschen, getrocknet und eingedampft. Das erhaltene Rohprodukt wurde durch Chromatographie über eine kleine Kieselgelsäule unter Verwendung von Toluol/Ethanol 95:5 als Elutionsmittel gereinigt. Nach Umkristallisation aus Ethanol wurden 0,6 g 8-Benzoylamino-5,6-dihydro-2-methyl-1-{2-[4-(4-methylpyridin-2-yl)-piperazin-1-yl]-ethyl}-4H-pyrrolo[3,2,1-ij]chinolin als cremefarbenes Pulver erhalten. Dieses wurde wie in Beispiel 3H) beschrieben in sein 0,5-Hydrochlorid mit einem Schmelzpunkt von 218 °C überführt.

### Beispiel 26:

### 5,6-Dihydro-2-methyl-8-(2-methylprop-1-enyl)-1-{2-[4-(4-methylpyridin-2-yl)-piperazin-1-yl]-ethyl}-4H-pyrrolo[3,2,1-ij]chinolin.

1 g 5,6-Dihydro-8-(2-methyl-1-hydroxypropyl)-2-methyl-1-{2-[4-(4-methylpyridin-2-yl)-piperazin-1-yl]-ethyl}-4H-pyrrolo[3,2,1-ij]chinolin (Herstellung siehe Beispiel 19) wurden in 40 ml Toluol gelöst. Zu der Lösung wurden 0,042 g p-Toluolsulfonsäure gegeben. Das Reaktionsgemisch wurde in einer mit Wasserabscheider versehenen Apparatur 6 Stunden am Rückfluß erhitzt.

Zur Aufarbeitung wurde das Reaktionsgemisch mit 50 ml 10-%iger wäßriger Natriumhydroxidlösung gewaschen. Dann wurde die organische Phase abgetrennt, mit Wasser neutral gewaschen, getrocknet und eingedampft. Es wurden 0,9 g ölige Titelbase erhalten. Diese wurde wie in Beispiel 1H) beschrieben in ihr Hydrochlorid überführt. Es wurden 790 mg 5,6-Dihydro-2-methyl-8-(2-methylprop-1-enyl)-1-{2-[4-(4-methylpyridin-2-yl)-piperazin-1-yl]-ethyl}-4H-pyrrolo[3,2,1-ij]chinolin·1,9 HCl mit einem Schmelzpunkt von 190 °C erhalten.

### Beispiel 27:

### 5,6-Dihydro-2-methyl-8-(2-methyl-1-oxopropyl)-1-{2-[4-(4-methylpyridin-2-yl)-piperazin-1-yl]-ethyl}-4H-pyrrolo [3,2,1-ij]chinolin.

3 g Pyridiniumchlorochromat wurden in 20 ml über Calciumchlorid getrocknetem Dichlormethan unter Stickstoffatmosphäre gelöst. Die Lösung wurde auf ca. 10 °C abgekühlt. Dann wurden 0,5 g Natriumacetat zugegeben. Dann wurde eine Lösung von 2,5 g 5,6-Dihydro-2-methyl-8-(2-methyl-1-hydroxypropyl)-1-{2-[4-(4-methylpyridin-2-yl)-piperazin-1-yl]-ethyl}-4H-pyrrolo [3,2,1-ij]chinolin (Herstellung siehe Beispiel 19) in 20 ml über Calciumchlorid getrocknetem Dichlormethan zugegeben. Das Reaktionsgemisch wurde 3 Stunden gerührt, dann wurden nochmals 0,3 g Pyridiniumchlorochromat zugegeben und weitere 3 Stunden gerührt. Zur Aufarbeitung wurden dem Reaktionsgemisch 100 ml Dichlormethan zugesetzt und filtriert. Dem Filtrat wurden 100 ml Wasser zugesetzt, wobei ein Niederschlag gebildet wurde. Von diesem wurde abfiltriert, die organische Phase wurde abgetrennt, mit Wasser gewaschen, getrocknet und eingeengt. Das erhaltene ölige Rohprodukt wurde durch Chromatographie über eine Kiesel-gelsäule unter Verwendung von Toluol/Ethanol 95:5 als Elutionsmittel gereinigt. Es wurden 1,4 g ölige Titelverbindung erhalten. Diese wurden wie in Beispiel 1H) beschrieben in ihr Hydrochlorid überführt, welches aus Isopropanol/Ethanol kristallisiert wurde. Es wurden 940 mg 5,6-Dihydro-2-methyl-8-(2-methyl-1-oxopropyl)-1-{2-[4-(4-methylpyridin-2-yl)-piperazin-1-yl]-ethyl}-4H-pyrrolo-[3,2,1-ij]chinolin-dihydrochlorid mit einem Schmelzpunkt von 248 °C erhalten.

### Beispiel 28:

### 2,3-Dihydro-5-methyl-6-{2-[4-(4-methylpyridin-2-yl)-piperazin-1-yl]-ethyl)-pyrrolo[1,2,3-de]-1,4-benzothiazin-1-oxid (= 28a) und

### 2,3-Dihydro-5-methyl-6-(2-[4-(4-methylpyridin-2-yl)-piperazin-1-yl]-ethyl}-pyrrolo[1,2,3-de]-1,4-benzothiazin-1,1-dioxyd (= 28b).

Eine Lösung von 0,84 g 2,3-Dihydro-5-methyl-6-{2-[4-(4-methylpyridin-2-yl)-piperazin-1-yl]-ethyl}-pyrrolo[1,2,3-de]-1,4-benzothiazin (erhalten aus 1 g des entsprechenden Hydrochlorides, Herstellung siehe Beispiel 11) in 20 ml Dichlormethan wurde auf -10 °C abgekühlt. Dann wurden 0,69 g m-Chlorperbenzoesäure zugesetzt. Das Reaktionsgemisch wurde 1 Stunde bei einer Temperatur von -10 °C gehalten und weitere 45 Minuten bei Raumtemperatur reagieren gelassen. Zur Aufarbeitung wurde das Reaktionsgemisch mit 10 ml gesättigter Natriumbicarbonatlösung extrahiert, zweimal mit je 10 ml Wasser gewaschen, getrocknet und eingeengt. Das als Rückstand verbleibende die beiden vorgenannten Titelverbindungen enthaltende rohe Gemisch wurde durch Chromatographie über eine kleine Kieselgelsäule aufgetrennt. Unter Verwendung eines Toluol/ Ethanol 9:1-Gemisches wurde eine Ausgangsprodukt enthaltende Fraktion gewonnen. Bei Erhöhung des Ethanolgehaltes des Elutionsmittels bis hin zu 100 % wurde eine das Monooxid enthaltende Fraktion gewonnen und durch anschließende Verwendung von Methanol als Elutionsmittel wurde eine weitere das Dioxid enthaltende Fraktion gewonnen. Aus der ethanolischen Fraktion wurden 0,24 g beigefarbenes Monooxid mit einem Schmelzpunkt von 132 °C erhalten. Aus der methanolischen Fraktion wurden 0,36 g Dioxid mit einem Schmelzpunkt von 126 °C erhalten.

### Beispiel 29:

### 5,6-Dihydro-2-methyl-8-methylamino-1-{2-[4-(4-methylpyridin-2-yl)-piperazin-1-yl]-ethyl}-4H-pyrrolo[3,2,1-ij]chinolin.

A) 44,5 g 5,6-Dihydro-2-methyl-8-nitro-4H-pyrrolo-[3,2,1-ij]-chinolin-1-essigsäure (Herstellung s. Beispiel 20 A) wurden in 450 ml Ethanol nach Zugabe von 8,63 ml Schwefelsäure 1 Stunde am Rückfluß erhitzt. Zur Aufarbeitung wurde das Ethanol abgedampft, der Rückstand in einem Gemisch aus Wasser und Dichlormethan aufgenommen, die organische Phase abgetrennt, mit Wasser neutral gewaschen, getrocknet und eingedampft. Zu dem als Rückstand verbleibenden öligen Rohprodukt wurde etwas Diethylether gegeben. Der gebildete Niederschlag wurde abfiltriert. Es wurden 32,4 g 5,6-Dihydro-2-methyl-8-nitro-4H-pyrrolo[3,2,1-ij]chinolin-1-essigsäureethylester als grünlich-ockerfarbenes Pulver erhalten.
B) 20 g des vorstehend erhaltenen Produktes wurden in einem Gemisch aus 300 ml Ethanol und 100 ml Ethylacetat gelöst. Dann wurden 3 g eines Palladium/Kohle-Katalysators (10 % Palladium auf Kohle) zugefügt und das Reaktionsgemisch wurde bei einer Temperatur von 50 °C mit einem Wasserstoffdruck von 4 bar hydriert. Anschließend wurde vom Katalysator abfiltriert und zur Trockne eingedampft. Es wurden 20 g Rohprodukt als bräunliches Öl erhalten. Dieses wurde aus Diisopropylether und Petrolether kristallisiert. Es wurden 15,2 g 5,6-Dihydro-2-methyl-8-amino-4H-pyrrolo-[3,2,1-ij]chinolin-1-essigsäureethylester als dunkelbraunes Pulver erhalten.
C) 14 g des vorstehend erhaltenen Produktes wurden mit 2,84 g Ameisensäure in 150 ml Toluol 2 Stunden am Rückfluß erhitzt. Zur Aufarbeitung wurde das Reaktionsgemisch zunächst mit 75 ml 10%-iger wäßriger Natriumhydroxidlösung, dann mit 100 ml Wasser, dann mit 75 ml 10%-iger wäßriger Salzsäure und schließlich bis zur Neutralität mit Wasser gewaschen, getrocknet und eingeengt. Als Rückstand verblieben 18 g Rohprodukt, welches aus Toluol umkristalli-siert wurde. Es wurden 11 g 5,6-Dihydro-2-methyl-8-formylamino-4H-pyrrolo[3,2,1-ij]chinolin-1-essigsäure- ethylester als beigefarbenes Pulver erhalten.
D) 11 g des vorstehend erhaltenen Produktes wurden in 100 ml Ethanol gegeben, dann wurde eine Lösung von 2,93 g Natriumhydroxid in 10 ml Wasser zugegeben, und das Reaktionsgemisch wurde 45 min. am Rückfluß erhitzt. Zur Aufarbeitung wurde das Reaktionsgemisch eingeengt, der Rückstand in Wasser aufgenommen, angesäuert und mit Dichlormethan extrahiert. Der Dichlormethanextrakt wurde getrocknet und eingeengt. Es wurden 1,73 g 5,6-Dihydro-2-methyl-8-formylamino-4H-pyrrolo[3,2,1-ij]chinolin-essigsäure erhalten.
E) 1,7 g der vorstehend erhaltenen Säure wurden in 20 ml Dichlormethan mit 1,61 g Carbonyldiimidazol 2 Stunden am Rückfluß erhitzt. Anschließend wurden 1,32 g 1-(4-Methylpyridin-2-yl)-piperazin zugegeben und das Reaktionsgemisch wurde weitere 2 Stunden am Rückfluß erhitzt. Zur Aufarbeitung wurde das Reaktionsgemisch mit Wasser gewaschen, getrocknet und eingeengt. Es wurden 3 g rohes 5,6-Dihydro-2-methyl-8-formylamino-1-{2-[4-(4-methylpyridin-2-yl)-piperazin-1-yl]-2-oxo-ethyl}-4H-pyrrolo-[3,2,1-ij]chinolin in Form gelbbräunlicher Plättchen erhalten.
F) 0,79 g Lithiumaluminiumhydrid wurden in 30 ml auf 5 - 10 °C gekühltes Tetrahydrofuran gegeben. Das Reaktionsgemisch wurde sich auf Raumtemperatur erwärmen gelassen. Dann wurde eine Lösung von 3 g des vorstehend erhaltenen Produktes in 50 ml Tetrahydrofuran zugegeben. Das Reaktionsgemisch wurde 1 Stunde am Rückfluß erhitzt. Anschließend wurde wie in Beispiel 13 C) beschrieben aufgearbeitet. Es wurden 2,6 g rohes 5,6-Dihydro-8-methylamino-2-methyl-1-{2-[4-(4-methylpyridin-2-yl)-piperazin-1-yl]-ethyl}-4H-pyrrolo[3,2,1-ij]chinolin als braunes Öl erhalten.

Zur Überführung in ihr Tartrat wurden 1 g der vorstehend erhaltenen Titelbase in wenig Isopropanol gelöst. Zu der Lösung wurde eine Lösung von 0,375 g Weinsäure in 1 ml Methanol gegeben. Der gebildete Niederschlag wurde abfiltriert. Es wurden 0,85 g 5,6-Dihydro-8-methylamino-2-methyl-1-{2-[4-(4-methylpyridin-2-yl)-piperazin-1-yl]-ethyl}-4H-pyrrolo[3,2,1-ij] chinolin-tartrat·1 H₂O mit einem Schmelzpunkt von 140 °C erhalten.

### Beispiel 30:

### 5,6-Dihydro-2-methyl-8-dimethylamino-1-{2-[4-(4-methylpyridin-2-yl)-piperazin-1-yl]-ethyl}-4H-pyrrolo[3,2,1-ij]chinolin.

A) 0,5 g 5,6-Dihydro-2-methyl-8-methylamino-1-{2-[4-(4-methylpyridin-2-yl)-piperazin-1-yl]-ethyl)-4H-pyrrolo[3,2,1-ij]chinolin (Herstellung siehe Beispiel 29) wurden mit 0,07 g Ameisensäure in 5 ml Toluol 3 Stunden am Rückfluß erhitzt. Zur Aufarbeitung wurde das Reaktionsgemisch mit Wasser gewaschen, getrocknet und eingeengt. Es wurden 0,42 g 5,6-Dihydro-2-methyl-8-(N-formyl-N-methylamino)-1-{2-[4-(4-methylpyridin-2-yl)-piperazin-1-yl]-ethyl)-4H-pyrrolo-[3,2,1-ij]chinolin erhalten.
B) 0,08 g Lithiumaluminiumhydrid wurden in 10 ml auf eine Temperatur von 0 - -5 °C gekühltes Tetrahydrofuran gegeben. Das Reaktionsgemisch wurde sich auf Raumtemperatur erwärmen gelassen. Dann wurde eine Lösung von 0,42 g des vorstehend erhaltenen Produktes in Tetrahydrofuran zugegeben. Das Reaktionsgemisch wurde 2 Stunden am Rückfluß erhitzt. Anschließend wurde wie in Beispiel 13 C) beschrieben aufgearbeitet. Es wurden 0,37 g rohes 5,6-Dihydro-2-methyl-8-dimethylamino-1-{2-[4-(4-methylpyridin-2-yl)-piperazin-1-yl]-ethyl}-4H-pyrrolo[3,2,1-ij]chinolin als braunes Öl erhalten. Diese Titelbase wurde wie in Beispiel 13 C) beschreiben in ihr Dihydrochlorid überführt. Es wurden 0,34 g 5,6-Dihydro-2-methyl-8-dimethylamino-1-{2-[4-(4-methylpyridin-2-yl)-piperazin-1-yl]-ethyl}-4H-pyrrolo-[3,2,1-ij]chinolin·2,1 HCl·0,1 H₂O als braunes Pulver mit einem Schmelzpunkt von 260 °C (Zersetzung) erhalten.

### Beispiel 31:

### 4,5,6,7-Tetrahydro-2-methyl-1-{2-[4-(4-methylpyridin-2-yl)-piperazin-1-yl]-ethyl}-pyrrolo[3,2,1-jk]benzazepin.

A) 43,86 g α-Tetralon wurden in 180 ml Ethanol gelöst. Zu der Lösung wurden eine Lösung von 104 g Hydroxylamin-hydrochlorid in 210 ml Wasser und anschließend 168 ml einer 50-%igen wäßrigen Kaliumhydroxydlösung gegeben. Die Mischung wurde im Wasserbad 15 Minuten zum Sieden erhitzt. Anschließend ließ man das Reaktionsgemisch auf Raumtemperatur abkühlen. Zur Aufarbeitung wurde das Reaktionsgemisch in kaltes Wasser gegeben und die Mischung mit Schwefelsäure angesäuert. Der gebildete dunkelgelbe Niederschlag wurde abfiltriert und aus Ethanol umkristallisiert. Es wurden 38,15 g α-Tetralonoxim als weißes Pulver mit einem Schmelzpunkt von 101 °C erhalten.
B) Zu einer auf 0 °C gekühlten Lösung von 1 g Tetralonoxim in 20 ml trockenem Dichlormethan wurden unter Stickstoffatmosphäre 30 ml DIBAH (= 1 molare Lösung von Diisobutylaluminiumhydrid in Hexan) tropfenweise zugefügt. Das Reaktionsgemisch wurde 1 Stunde bei 0 °C gehalten. Anschließend ließ man das Reaktionsgemisch sich auf Raumtemperatur erwärmen, nach 2 Stunden wurde wieder auf 0 °C gekühlt und zur Unterbrechung der Reaktion wurde mit 30 ml Dichlormethan verdünnt und unter kräftigem Rühren mit 5,2 g Natriumfluorid und anschließend 2 ml Wasser behandelt. Das Reaktionsgemisch wurde für weitere 30 Minuten bei 0 °C kräftig gerührt. Es bildete sich eine weißliche gelatinöse Masse. Zur Aufarbeitung wurde filtriert, der Filterrückstand nochmals mit Dichlormethan gewaschen und die vereinigten Filtrate eingedampft. Es wurden 0,80 g rohes 2,3,4,5-Tetrahydro-benzo[b]-1H-azepin als gelbes Öl erhalten.
C) Zu einer Mischung aus 17 ml 12 N Salzsäure, 50 g Eis und 15 g des vorstehend erhaltenen Produktes wurde langsam eine Lösung von 8,5 g Natriumnitrit in 20 ml Wasser gegeben, wobei die Temperatur unter 5 °C gehalten wurde. Das Reaktionsgemisch wurde 30 Minuten bei dieser Temperatur gehalten. Anschließend ließ man das Reaktionsgemisch sich auf Raumtemperatur erwärmen. Nach 1 Stunde wurde das Reaktionsgemisch mit Essigsäureethylester extrahiert. Die organische Phase wurde abgetrennt, mit Wasser gewaschen, getrocknet und eingedampft. Es wurde 17,88 g rohes 2,3,4,5-Tetrahydro-1-nitrosobenz[b]-1H-azepin als gelboranges Öl erhalten.
D) 17,5 g des vorstehend erhaltenen Produktes wurden wie in Beispiel 1B) beschrieben mit 7,54 g Lithiumaluminiumhydrid in Tetrahydrofuran reduziert. Das Reaktionsgemisch wurde wie in Beispiel 1B) beschrieben aufgearbeitet. Es wurden 14,6 g 1-Amino-2,3,4,5-tetrahydrobenz[b]-1H-azepin als oranges Öl erhalten.
E) 4 g des vorstehend Produktes wurden mit 4,26 g Lävulinsäureethylester in einem Gemisch aus 36,66 ml Eisessig und 2,25 ml 12 N Salzsäure 3 Stunden unter Rückfluß auf eine Temperatur von 90 °C erhitzt. Zur Aufarbeitung wurde das Reaktionsgemisch abgekühlt, das organische Lösungsmittel abgedampft, der Rückstand in Wasser aufgenommen und mit Dichlormethan extrahiert. Die Dichlormethanphase wurde abgetrennt, mit Wasser gewaschen, getrocknet und eingedampft. Es wurden 2,67 g rohes 4,5,6,7-Tetrahydro-2-methyl-pyrrolo [3,2,1-jk]benzazepin-1-essigsäureethylester als schwarzes Öl erhalten.
F) 2,67 g des vorstehenden Esters wurden in 40 ml Ethanol gelöst. Zu der Lösung wurde eine Lösung von 1,18 g Natriumhydroxid in 5 ml Wasser gegeben und das Reaktionsgemisch wurde 1 Stunde am Rückfluß (Temperatur ca. 78 °C) erhitzt. Zur Aufarbeitung wurde der Alkohol abgedampft, der Rückstand in Wasser aufgenommen, die wäßrige Phase mit Essigsäureethylester gewaschen und anschließend durch Zugabe von Salzsäure auf pH = 1 eingestellt. Die angesäuerte wäßrige Phase wurde wiederum mit Essigsäureethylester extrahiert, die organische Phase abgetrennt, mit Wasser neutral gewaschen, getrocknet und eingedampft. Es wurden 1,78 g rohes 4,5,6,7-Tetrahydro-2-methyl-pyrrolo[3,2,1-jk]benzazepin-1-essigsäure als braunes Öl erhalten.
G) 4,8 g der vorstehenden Säure wurden nach der in Beispiel 13B) beschriebenen Methode mit 4,2 g 1-(4-Methylpyridin-2-yl)piperazin umgesetzt. Das Reaktionsgemisch wurde wie in Beispiel 13B) aufgearbeitet. Es wurden 6,82 g rohes 4,5,6,7-Tetrahydro-2-methyl-1-{2-[4-(4-methylpyridin-2-yl)-piperazin-1-yl]-2-oxoethyl}-pyrrolo[3,2,1-jk)benzazepin als grünes Öl erhalten.
H) 6,5 g des vorstehend erhaltenen Produktes wurden in Tetrahydrofuran mit 1,53 g Lithiumaluminiumhydrid wie in Beispiel 13C) beschrieben reduziert. Das Reaktionsgemisch wurde wie in Beispiel 13C) beschrieben aufgearbeitet. Es wurden 5,83 g rohes 4,5,6,7-Tetrahydro-2-methyl-1-{2-[4-(4-methylpyridin-2-yl)-piperazin-1-yl]-ethyl}-pyrrolo[3,2,1-jk]benzazepin erhalten.

Die vorstehend erhaltene Titelbase wurde nach der in Beispiel 13C) beschriebenen Methode in ihr Hydrochlorid überführt. Das erhaltene 4,5,6,7-Tetrahydro-2-methyl-1-{2-[4-(4-methylpyridin-2-yl)-piperazin-1-yl]-ethyl}-pyrrolo[3,2,1-jk]benzazepindihydrochlorid•1 H₂O wurde als weißes Pulver (Schmelzpunkt > 260 °C) erhalten.

### Beispiel 32:

### 2-Benzyl-5,6-dihydro-1-{2-[4-(4-methylpyridin-2-yl)-piperazin-1-yl]-ethyl}-4H-pyrrolo[3,2,2-ij]chinolin.

A) 10 g 1-Amino-1,2,3,4-tetrahydrochinolin wurden in 100 ml Ethanol gelöst. Zu der Lösung wurden 10,87 g Phenylaceton gegeben und das Reaktionsgemisch wurde 5 Stunden am Rückfluß erhizt und anschließend 12 Stunden bei Raumtemperatur gehalten. Zur Aufarbeitung wurde das Reaktionsgemisch zur Trockene eingeengt und der Rückstand durch Chromatographie über eine Kieselgelsäule unter Verwendung von Dichlormethan als Elutionsmittel gereinigt. Es wurden 14 g rohes 1-(1-Benzyl-ethylimino)-1,2,3,4-tetrahydrochinolin als orangefarbenes Öl erhalten.
B) Eine Lösung von 13 g des vorstehend erhaltenen Produktes in 105,7 ml Dichlormethan wurde mit einer Lösung von 2,1 g Phosphorpentoxid in 70 g Methansulfonsäure vermischt. Das Gemisch wurde 2 Tage am Rückfluß erhitzt und weitere 3 Tage bei Raumtemperatur reagieren gelassen. Zur Aufarbeitung wurde das Reaktionsgemisch abgekühlt und durch Zugabe von 280 ml 2,5 N Natriumhydroxidlösung neutralisiert. Die organische Phase wurde abgetrennt, zweimal mit je 200 ml Wasser gewaschen, getrocknet und eingeengt. Der Rückstand wurde durch Chromatographie über eine Kieselgelsäule unter Verwendung von Dichlormethan als Elutionsmittel gereinigt. Es wurden 4,5 g rohes 2-Benzyl-5,6-dihydro-4H-pyrrolo[3,2,1-ij]chinolin als gelbes Öl erhalten.
C) 2 g des vorstehend erhaltenen Produktes wurden in Diethylether mit 1,12 g Oxalylchlorid und anschließend mit 2,1 g 1-(4-Methylpyridin-2-yl)-piperazin nach der in Beispiel 14C) beschriebenen Methode umgesetzt. Das Reaktionsgemisch wurde wie in Beispiel 14C) beschrieben aufgearbeitet. Es wurden 4,2 g 2-Benzyl-5, 6-dihydro-1-{2-[4-(4-methylpyridin-2-yl)-piperazin-1-yl]-1,2-dioxoethyl}-4H-pyrrolo [3,2,1-ij]chinolin erhalten.
D) 2,7 g des vorstehend erhaltenen Produktes wurden in 15 ml Tetrahydrofuran gelöst, anschließend wurden zu der Lösung unter Stickstoffatmosphäre 30 ml einer 1-molaren Lösung von Diboran in Tetrahydrofuran gegeben, wobei eine Gasentwicklung stattfand. Das Reaktionsgemisch wurde 3 Stunden am Rückfluß erhitzt. Zur Aufarbeitung und zur Zerstörung des Diborankomplexes wurde das Reaktionsgemisch zur Trockene eingedampft, der Rückstand in 30 ml 3 N Salzsäure aufgenommen und 4 Stunden bei Raumtemperatur belassen. Anschließend wurde durch Zugabe von 10 ml verdünnter Natronlauge neutralisiert und mit 100 ml Dichlormethan extrahiert. Die organische Phase wurde zweimal mit je 100 ml Wasser gewaschen, getrocknet und eingedampft. Die gleiche Behandlung wurde noch zweimal wiederholt und das erhaltene Rohprodukt wurde anschließend durch Chromatographie über eine Kieselgelsäule unter Verwendung von Toluol/Ethanol 99:1 gereinigt. Es wurden 0,9 g rohes 2-Benzyl-5,6-dihydro-1-{2-[4-(4-methylpyridin-2-yl)-piperazin-1-yl]-ethyl}-4H-pyrrolo[3,2,1-ij]chinolin als gelbes Öl erhalten.

Die vorstehend erhaltene Titelbase wurde wie in Beispiel 13C) beschrieben in ihr Hydrochlorid überführt. Es wurden 1,5 g 2-Benzyl-5,6-dihydro-1-{2-[4-(4-methylpyridin-2-yl)-piperazin-1-yl]-ethyl}-4H-pyrrolo[3,2,1-ij]chinolin•2,4 HCl•2 H₂O als hellgraues Pulver mit einem Schmelzpunkt von 186 - 208 °C erhalten.

Nach den in den vorstehenden Beispielen beschriebenen Verfahren können auch die in der nachfolgenden Tabelle 1 aufgeführten Verbindungen der Formel I hergestellt werden.

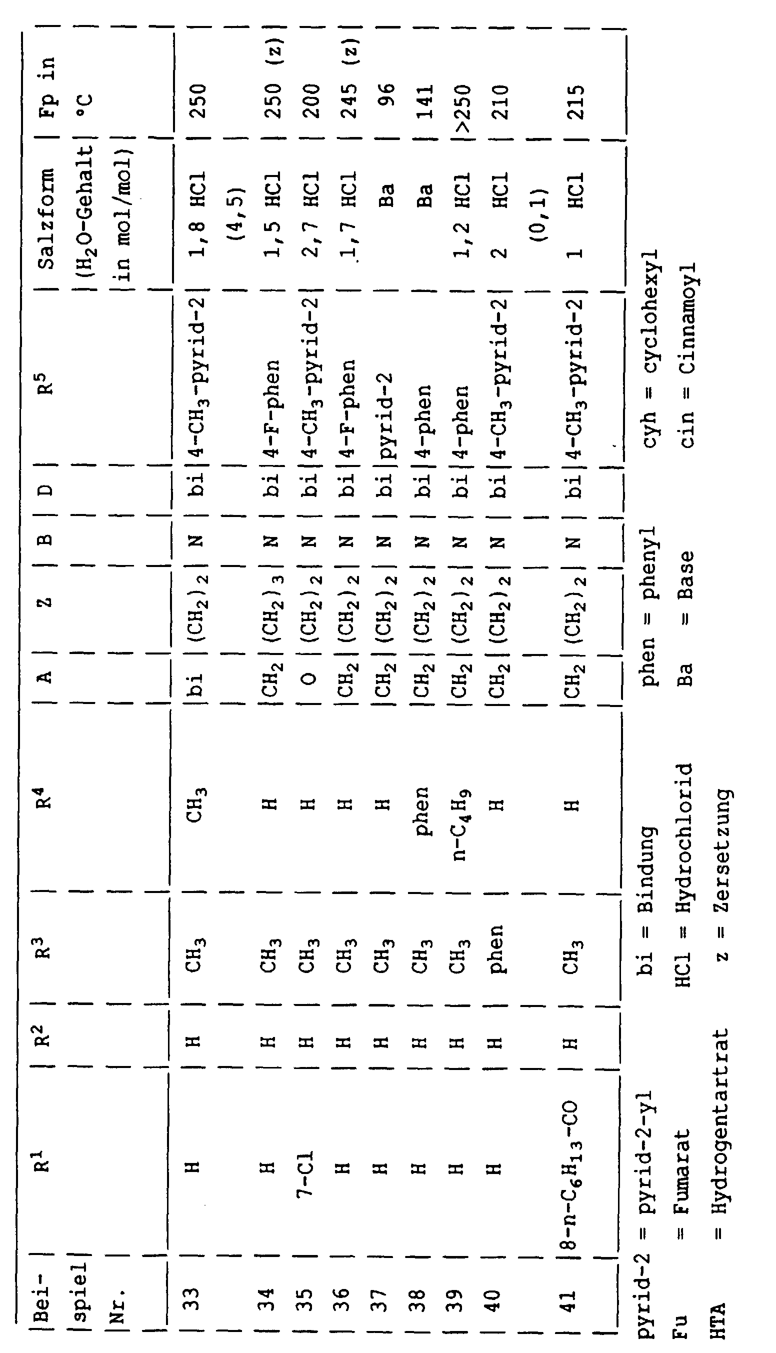

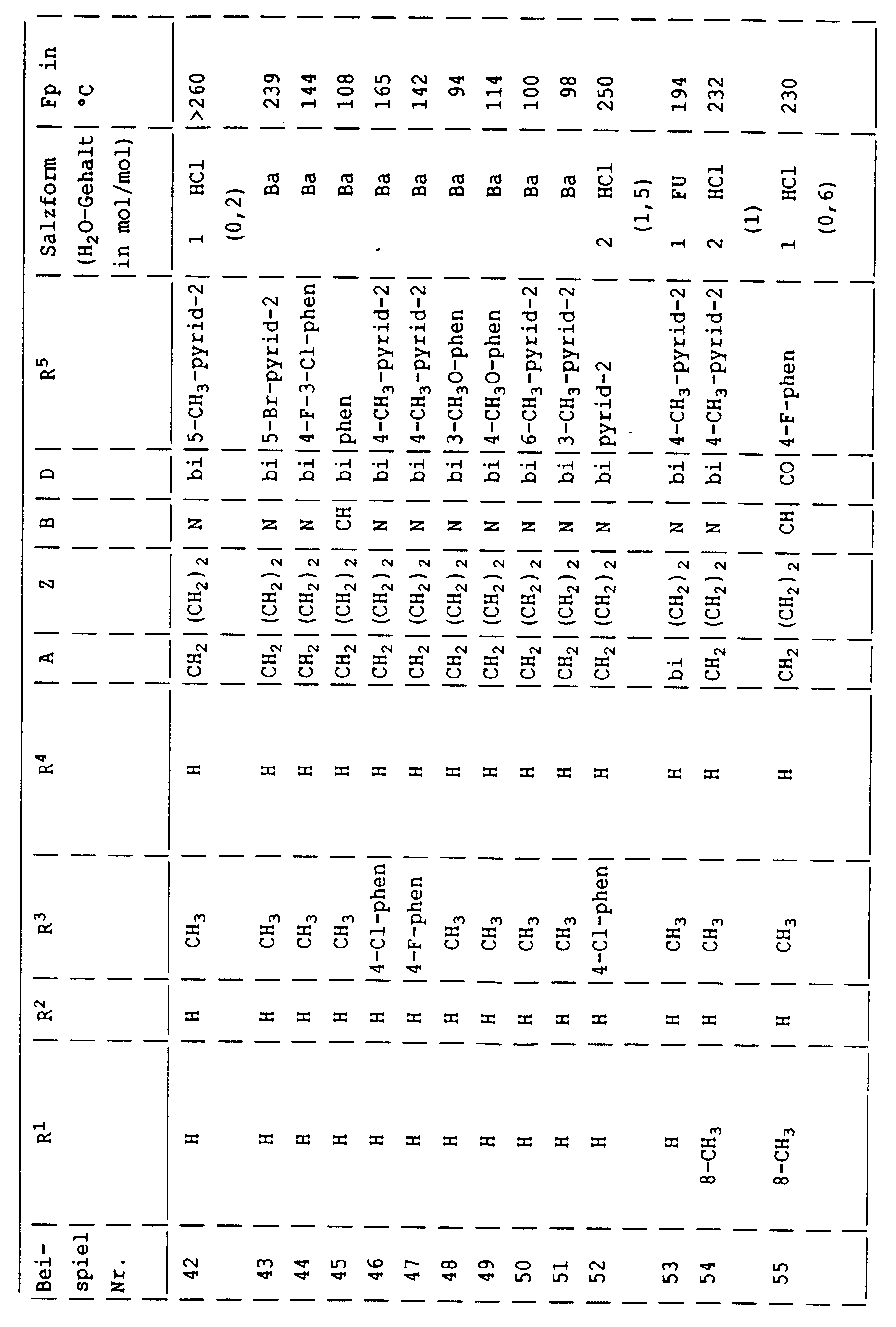

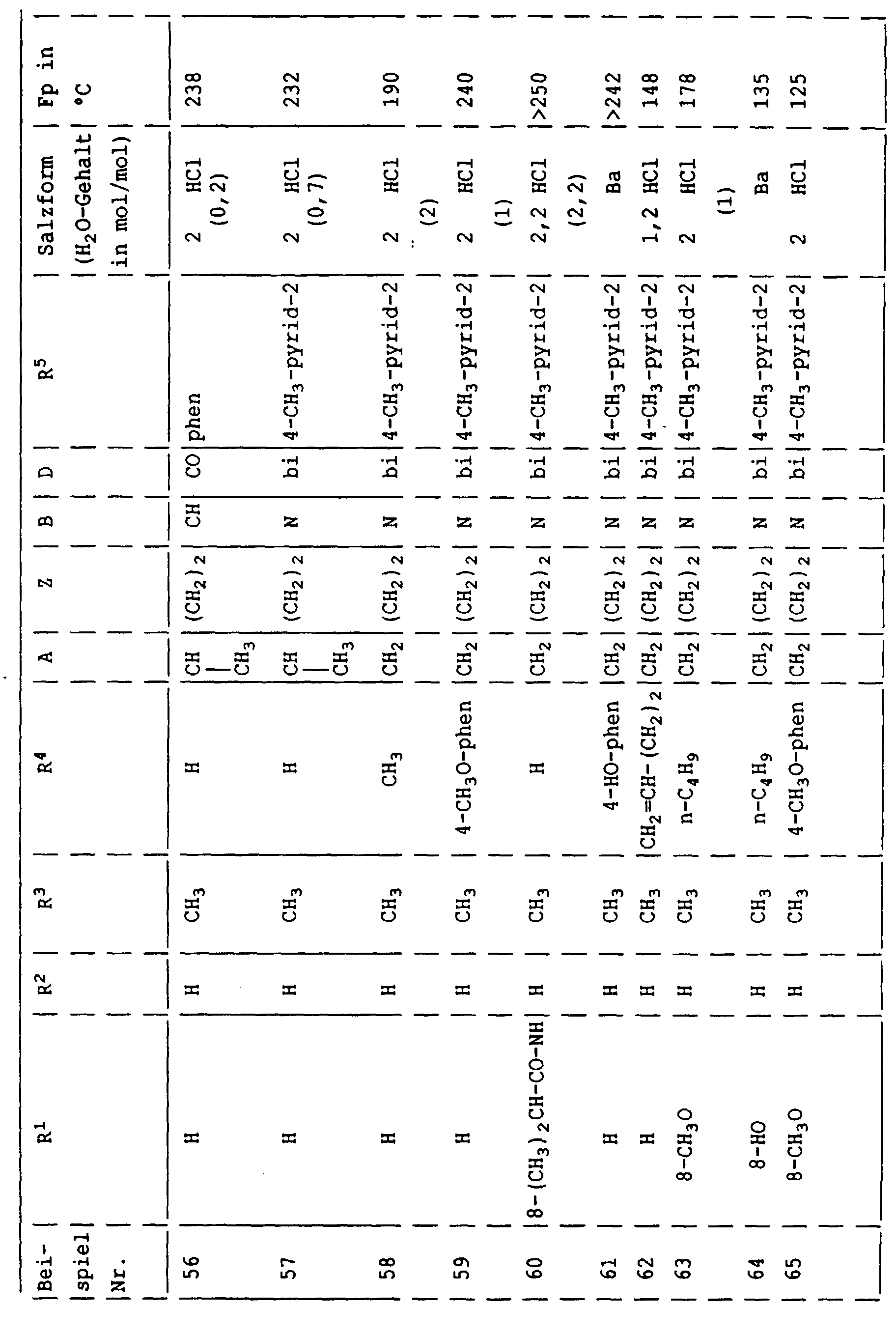

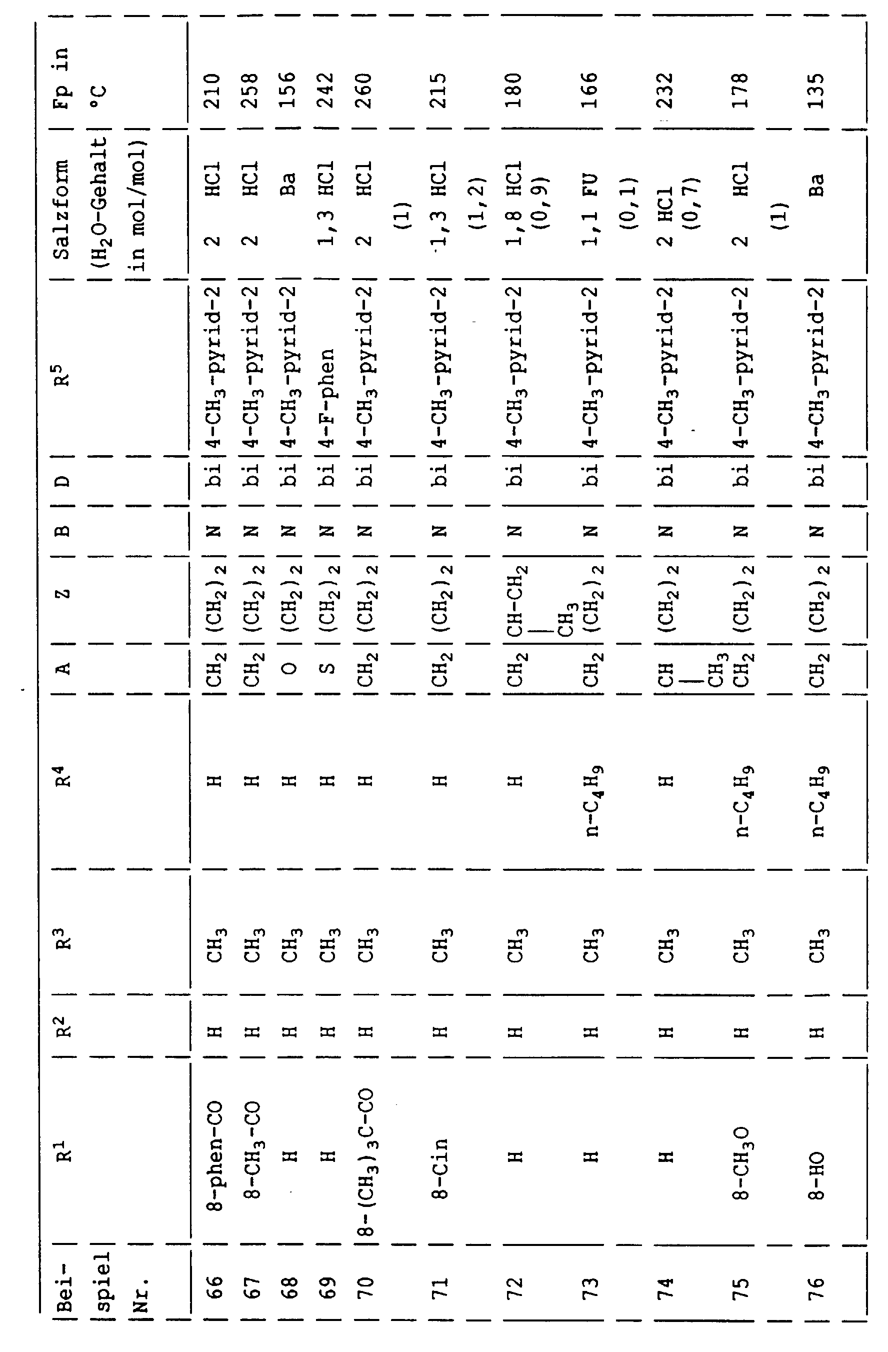

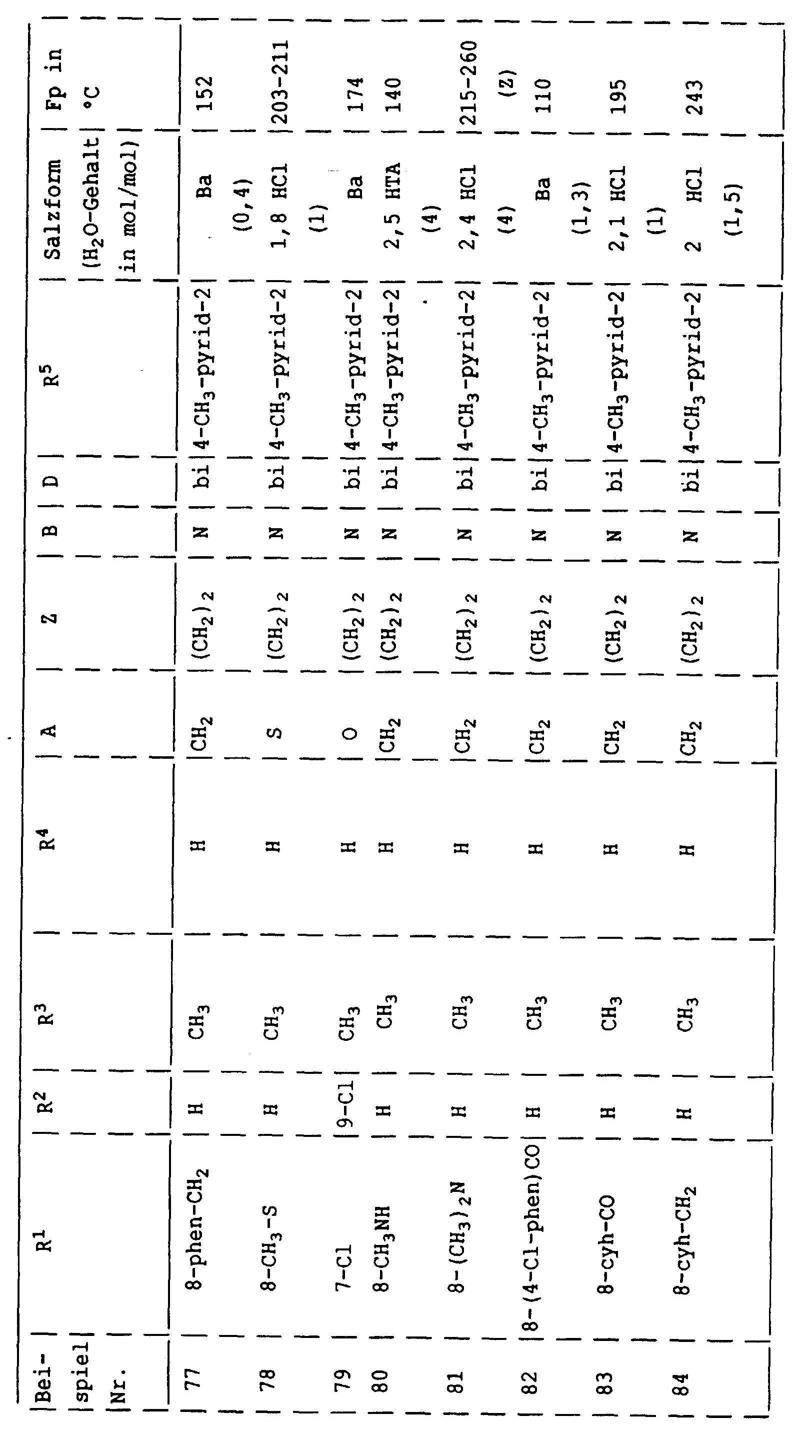

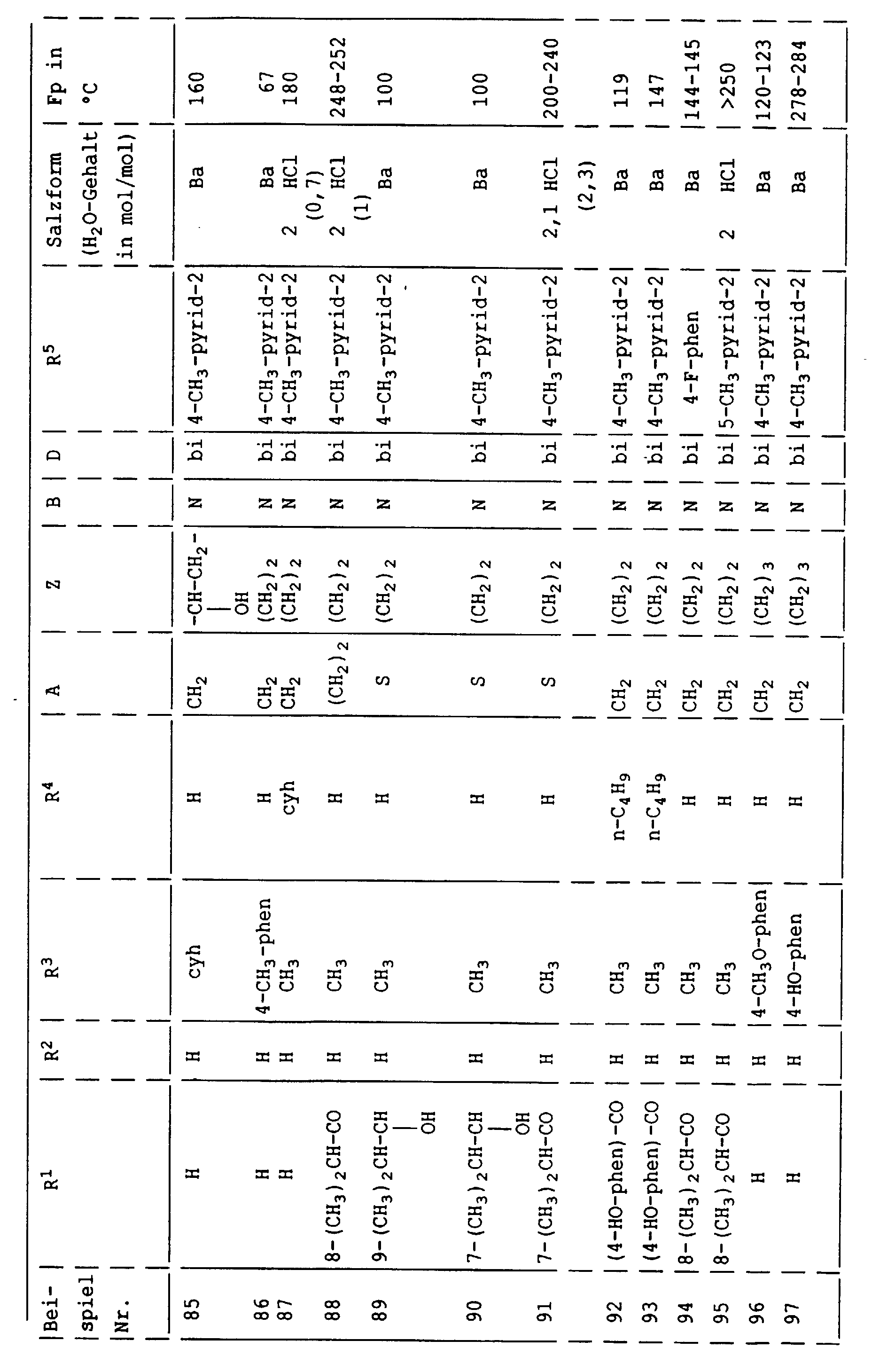

### Beispiel I:

### 5,6-Dihydro-2-methyl-1-{2-[4-(4-methylpyridin-2-yl)-piperazin-1-yl]-ethyl}-4H-pyrrolo [3,2,1-ij]chinolin enthaltende Tabletten.

Man stellte Tabletten in folgender Zusammensetzung pro Tablette her:

| | |
|---|---|
| 5,6-Dihydro-2-methyl-1-{2-[4-(4-methylpyridin-2-yl)-piperazin-1-yl]-ethyl}-4H-pyrrolo[3,2,1-ij]chinolin | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

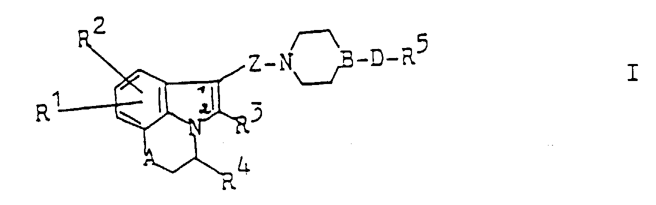

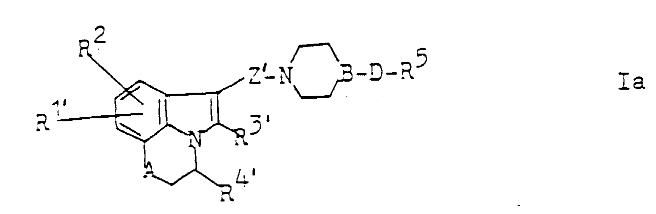

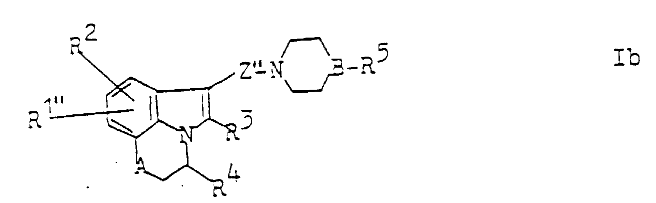

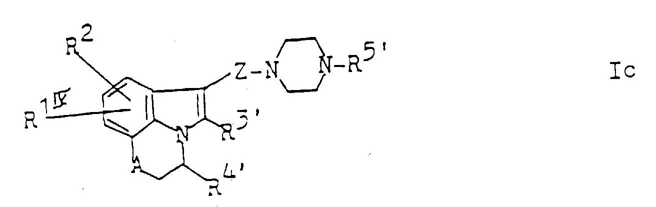

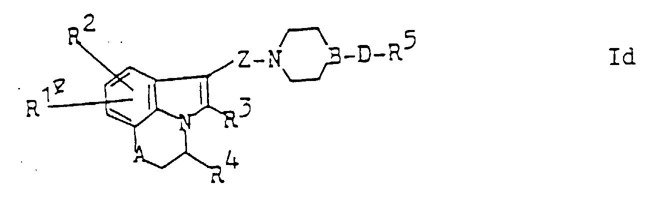

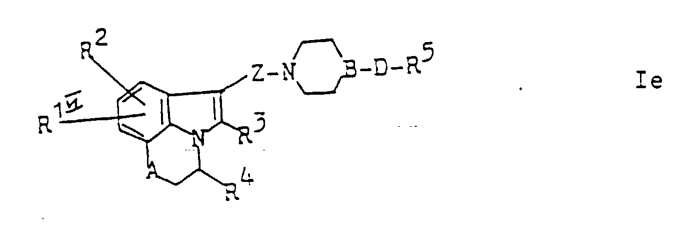

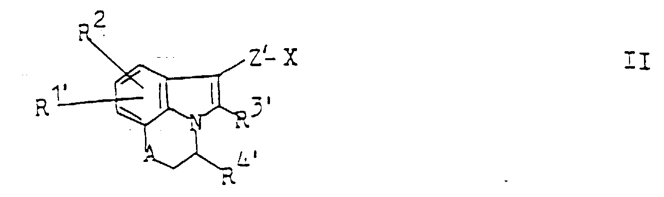

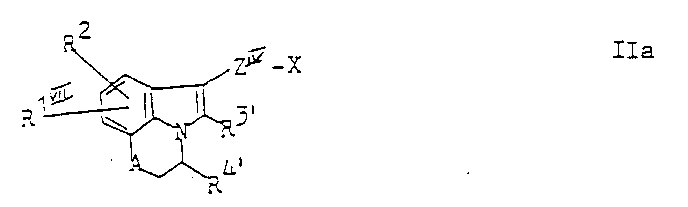

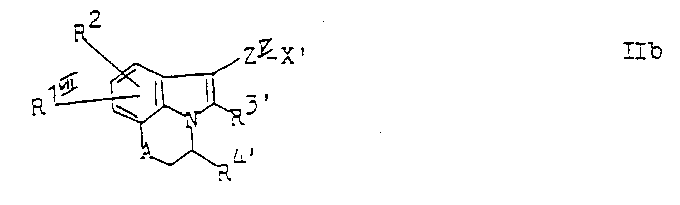

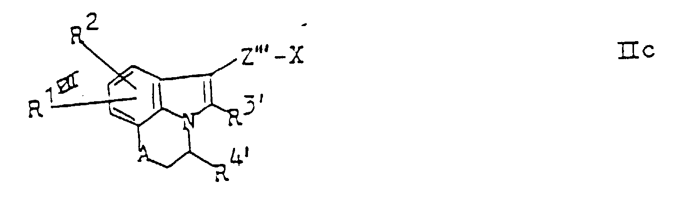

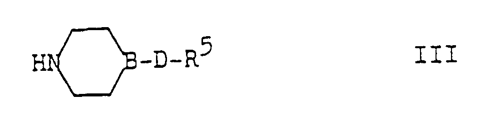

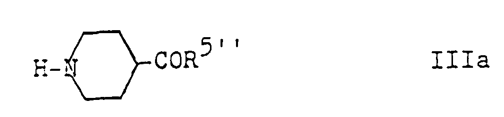

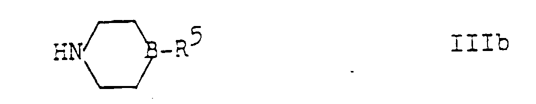

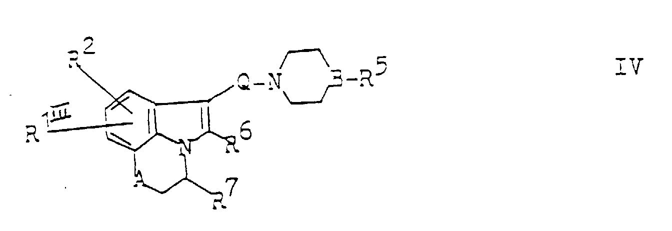

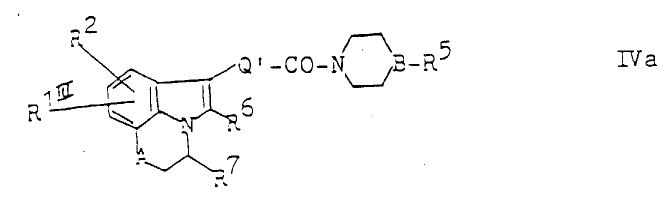

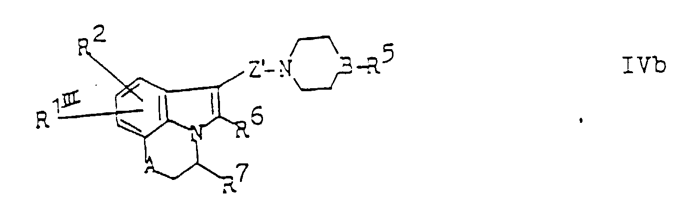

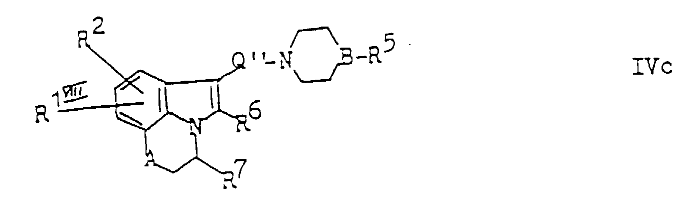

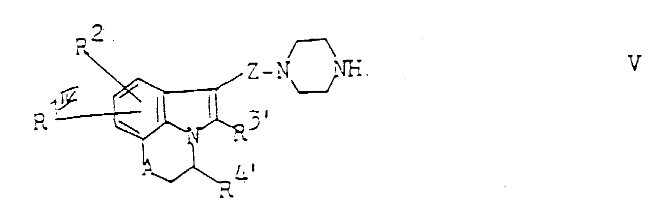

X'-R^{5'} VI

R⁸-Y VII

R⁸-OH VIIa

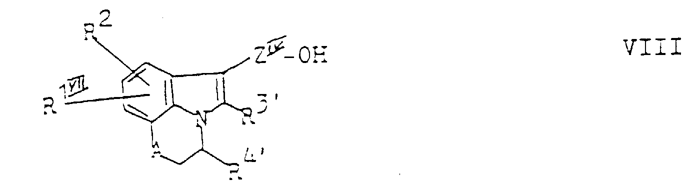

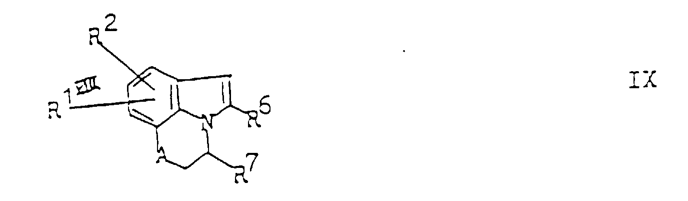

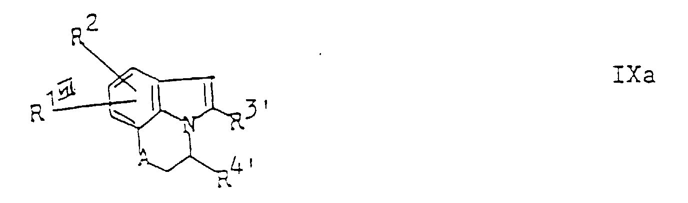

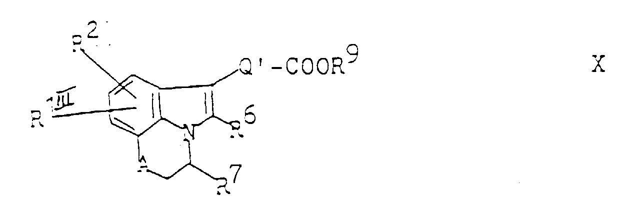

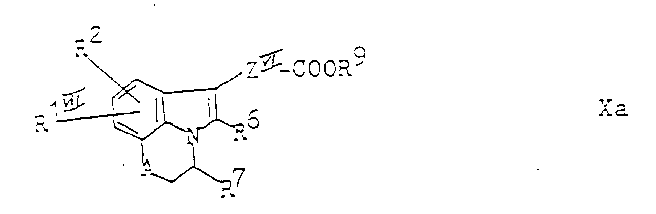

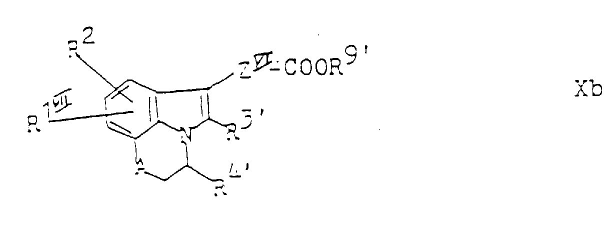

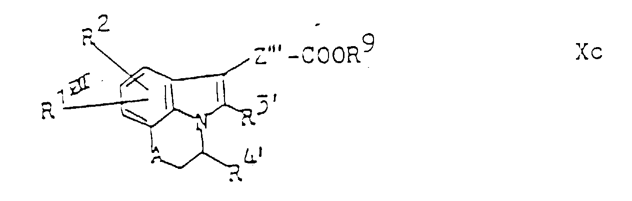

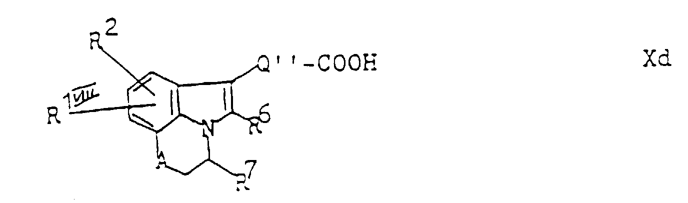

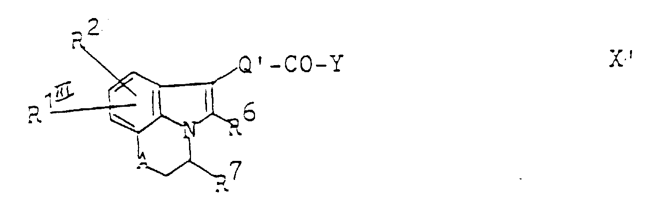

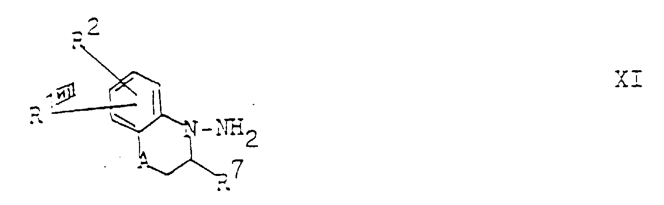

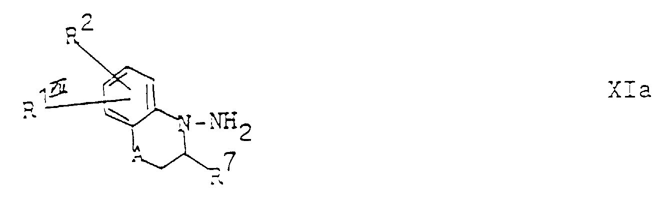

R⁹O-CO-Z^{VI}-CH₂-CO-R¹⁰ XII

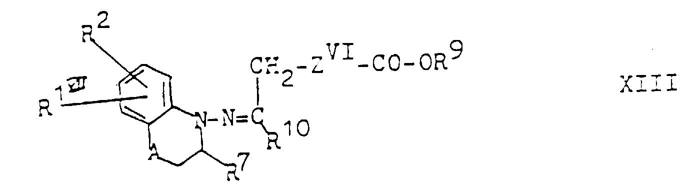

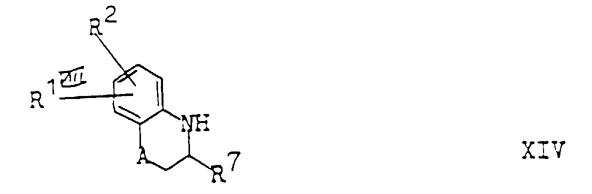

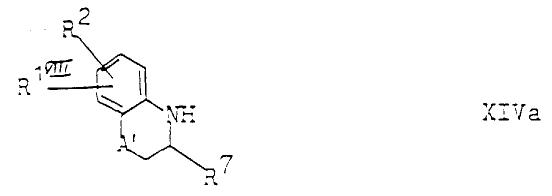

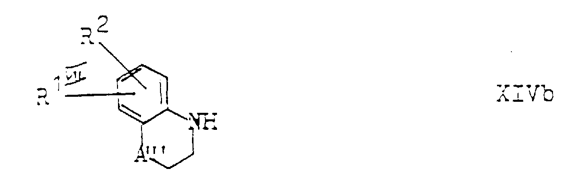

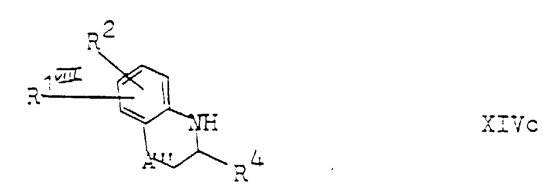

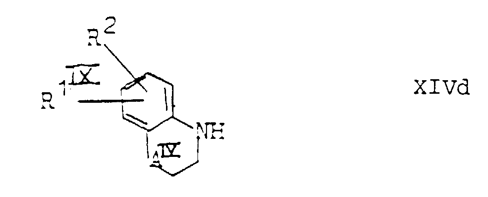

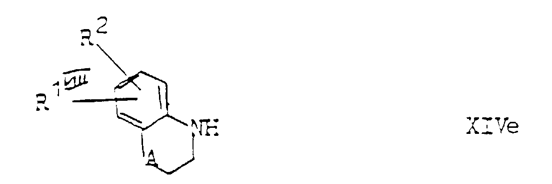

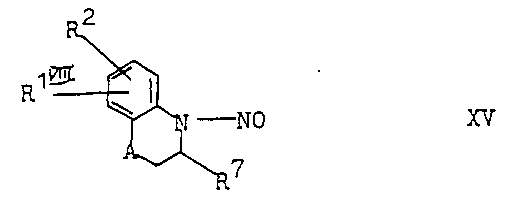

R⁶-CO-CH₃ XVI

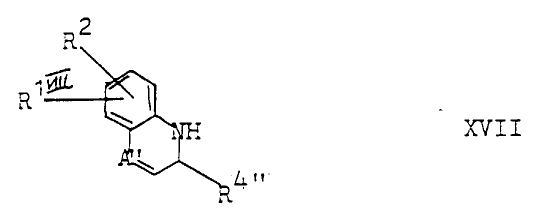

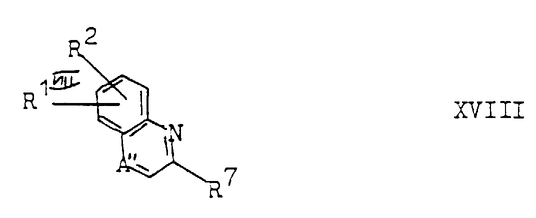

Li-R^{4"} XIX

X'-R^{4"'} XX

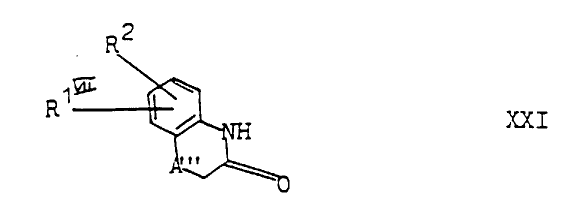

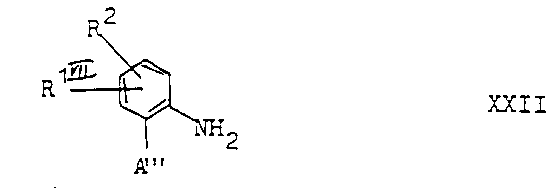

H-R^{5"} XXIII

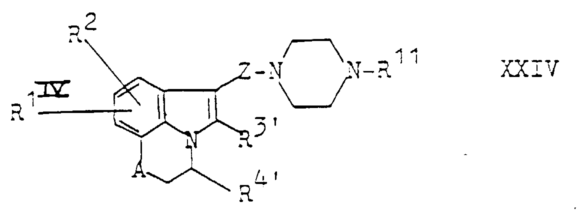

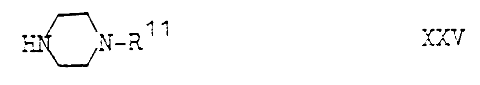

X'-Q"-Y' XXVI

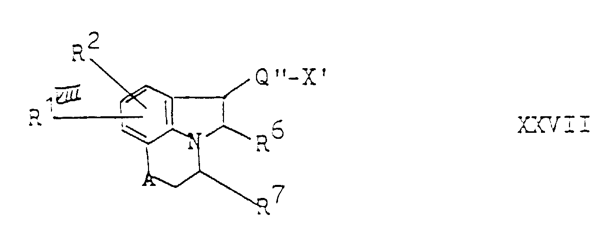

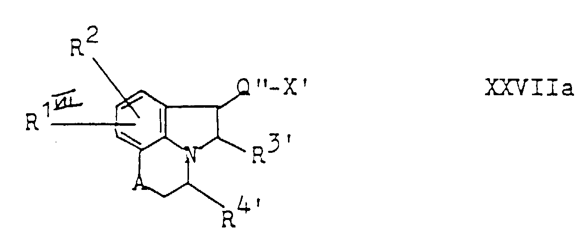

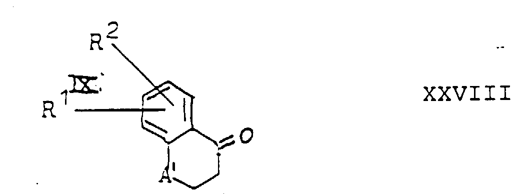

X'-CO-Q"-Y' XXIX

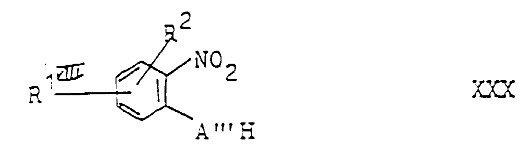

X'-CH₂-CO-R⁴ XXXI

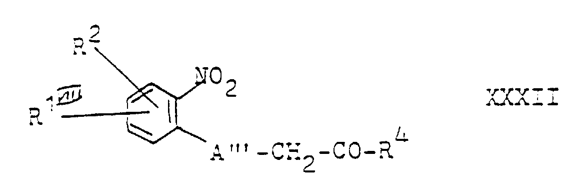

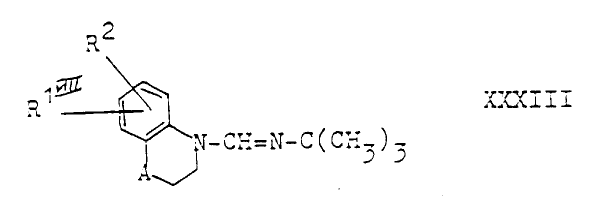

R^{7'} -X' XXXIV

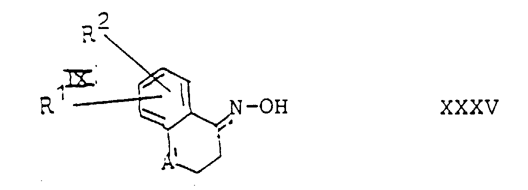

## Patentansprüche

1. Verbindungen der allgemeinen Formel I 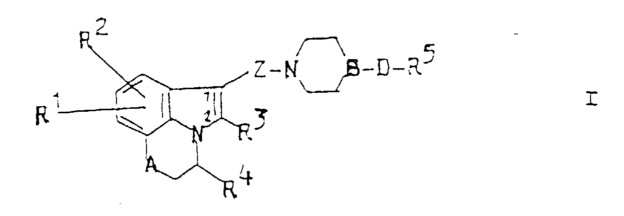 worin
R¹ Wasserstoff, C₁-C₄-Alkoxy, C₁-C₄-Alkylthio, Hydroxy, Halogen, Trifluormethyl, Nitro, Amino, C₁-C₄-Mono- oder Dialkylamino, C₁-C₇-Alkyl, welches gegebenenfalls durch Hydroxy substituiert sein kann, eine Phenyl-C₁-C₄-alkylgruppe, welche im Phenylring gegebenenfalls durch C₁-C₄-Alkyl, C₁-C₄-Alkoxy, Hydroxy oder Halogen substituiert sein kann, C₃-C₆-Cycloalkyl, C₄-C₇-Cycloalkylalkyl, C₃-C₇-Alkenyl, C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl-, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet,
R² Wasserstoff, Halogen, C₁-C₄-Alkyl oder , falls R¹ nicht Hydroxy oder eine hydroxyphenylhaltige Gruppe ist, auch C₁-C₄-Alkoxy bedeutet,
R³ Wasserstoff, C₁-C₄ Alkyl, welches gegebenenfalls durch Hydroxy substituiert sein kann, C₁-C₄-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄ Alkoxy, Hydroxy oder Hydroxyalkoxy substituiert sein kann, bedeutet, wobei jedoch R³ nur dann eine freie Hydroxygruppe enthalten kann, wenn R¹ keine Carbonyloxygruppe enthält,
R⁴ Wasserstoff, C₁-C₇-Alkyl, welches gegebenenfalls durch Hydroxy substituiert sein kann, C₃-C₇-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, bedeutet, wobei jedoch R⁴ nur dann eine freie Hydroxygruppe enthalten kann, wenn R¹ keine Carbonyloxygruppe enthält,
A eine gegebenenfalls durch C₁-C₄-Alkyl substituierte Alkylenkette mit 1 - 2 Kohlenstoffatomen, eine Bindung, Sauerstoff oder Schwefel bedeutet,
Z eine Alkylenkette mit 2 - 4 Kohlenstoffatomen, welche gegebenenfalls durch C₁-C₄-Alkyl oder, falls R¹ keine Carbonyloxygruppe enthält, auch durch Hydroxy substituiert sein kann, bedeutet,
B Stickstoff oder die CH-Gruppe bedeutet,
R⁵ einen gegebenenfalls durch C₁-C₄-Alkyl, C₁-C₄-Alkoxy oder Halogen substituierten Pyridyl- oder Phenylrest bedeutet, und
D eine Bindung darstellt oder, falls B die CH-Gruppe und R⁵ einen gegebenenfalls substituierten Phenylrest bedeuten, auch die CO-Gruppe darstellen kann,
und deren Säureadditionssalze und/oder S-Mono- oder Dioxide von schwefelhaltigen Verbindungen der Formel I.

2. Verbindungen gemäß Anspruch 1, worin R¹ Wasserstoff, gegebenenfalls durch Hydroxy substituiertes C₁-C₆-Alkyl, Halogen, Hydroxy, C₁-C₄-Alkoxy, C₁-C₄-Alkanoyl, C₁-C₄-Alkanoyloxy, C₁-C₄-Alkanoylamino, oder eine gegebenenfalls durch C₁-C₄-Alkyl, C₁-C₄-Alkoxy oder Halogen im Phenylring substituierte Benzoyl-, Benzoyloxy-, Benzoylamino-, Cinnamoyl-, Cinnamoyloxy- oder Cinnamoylaminogruppe bedeutet.

3. Verbindungen gemäß Anspruch 1 oder 2, dadurch gekennzeichnet, daß R² Wasserstoff bedeutet.

4. Verbindungen gemäß einem der vorstehenden Ansprüche, dadurch gekennzeichnet, daß R³ C₁-C₄-Alkyl bedeutet.

5. Verbindungen gemäß einem der vorstehenden Ansprüche, worin A eine gegebenenfalls durch C₁-C₄-Alkyl substituierte Methylengruppe bedeutet.

6. Verbindungen nach einem der vorstehenden Ansprüche, dadurch gekennzeichnet, daß Z eine gegebenenfalls durch Hydroxy oder C₁-C₄-Alkyl substituierte Ethylenkette bedeutet.

7. Verbindungen gemäß einem der vorstehenden Ansprüche, dadurch gekennzeichnet, daß R⁵ einen gegebenenfalls durch C₁-C₄-Alkyl, C₁-C₄-Alkoxy oder Halogen substituierten Pyridylrest bedeutet.

8. Verbindungen gemäß Anspruch 7, dadurch gekennzeichnet, daß R⁵ den 4-Methylpyrid-2-ylrest darstellt.

9. Arzneimittel enthaltend eine pharmakologisch wirksame Menge einer Verbindung gemäß Anspruch 1 und übliche pharmazeutische Hilfs- und/oder Trägerstoffe.

10. Verfahren zur Herstellung von Verbindungen der allgemeinen Formel I 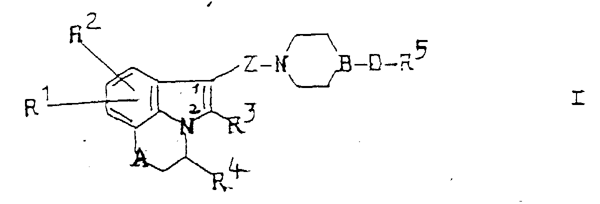 worin
R¹ Wasserstoff, C₁-C₄-Alkoxy, C₁-C₄-Alkylthio, Hydroxy, Halogen, Trifluormethyl, Nitro, Amino, C₁-C₄-Mono- oder Dialkylamino, C₁-C₇-Alkyl, welches gegebenenfalls durch Hydroxy substituiert sein kann, eine Phenyl-C₁-C₄-alkylgruppe, welche im Phenylring gegebenenfalls durch C₁-C₄-Alkyl, C₁-C₄-Alkoxy, Hydroxy oder Halogen substituiert sein kann, C₃-C₆-Cycloalkyl, C₄-C₇-Cycloalkylalkyl, C₃-C₇-Alkenyl, C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl -, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet,
R² Wasserstoff, Halogen, C₁-C₄-Alkyl oder , falls R¹ nicht Hydroxy oder eine hydroxyphenylhaltige Gruppe ist, auch C₁-C₄-Alkoxy bedeutet,
R³ Wasserstoff, C₁-C₄-Alkyl, welches gegebenenfalls durch Hydroxy substituiert sein kann, C₁-C₄-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, bedeutet, wobei jedoch R³ nur dann eine freie Hydroxygruppe enthalten kann, wenn R¹ keine Carbonyloxygruppe enthält,
R⁴ Wasserstoff, C₁-C₇-Alkyl, welches gegebenenfalls durch Hydroxy substituiert sein kann, C₃-C₇-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, bedeutet, wobei jedoch R⁴ nur dann eine freie Hydroxygruppe enthalten kann, wenn R¹ keine Carbonyloxygruppe enthält,
A eine gegebenenfalls durch C₁-C₄-Alkyl substituierte Alkylenkette mit 1 - 2 Kohlenstoffatomen, eine Bindung, Sauerstoff oder Schwefel bedeutet,
Z eine Alkylenkette mit 2 - 4 Kohlenstoffatomen, welche gegebenenfalls durch C₁-C₄-Alkyl oder, falls R¹ keine Carbonyloxygruppe enthält, auch durch Hydroxy substituiert sein kann, bedeutet,
B Stickstoff oder die OH-Gruppe bedeutet,
R⁵ einen gegebenenfalls durch C₁-C₄-Alkyl, C₁-C₄-Alkoxy oder Halogen substituierten Pyridyl- oder Phenylrest bedeutet, und
D eine Bindung darstellt oder, falls B die CH-Gruppe und R⁵ einen gegebenenfalls substituierten Phenylrest bedeuten, auch die CO-Gruppe darstellen kann,
und deren Säureadditionssalzen und/oder S-Mono- oder -Di-oxiden von schwefelhaltigen Verbindungen der Formel I, dadurch gekennzeichnet, daß man
a) zur Herstellung von Verbindungen der ailgemeinen Formel Ia 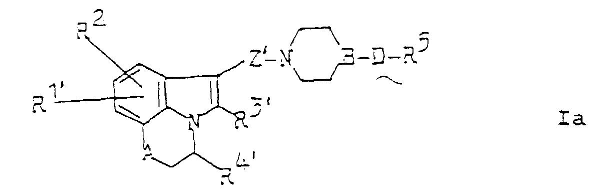 worin R^{1'} die für R¹ angegebene Bedeutung mit Ausnahme von hydroxy-substituiertem C₁-C₇-Alkyl oder Mono- oder Di-C₁-C₄-alkylamino besitzt, R^{3'} und R^{4'} die für R³ und R⁴ angegebenen Reste mit Ausnahme von C₁-C₄-Hydroxyalkylgruppen enthaltenden Resten bedeuten, Z' die für Z angegebene Bedeutung besitzt, wobei jedoch die Alkylenkette Z' nur in der dem Indolgerüst benachbarten Position einen allfälligen Hydroxysubstituenten enthalten kann, und R², R⁵, A, B und D obige Bedeutung besitzen, Verbindungen der allgemeinen Formel II 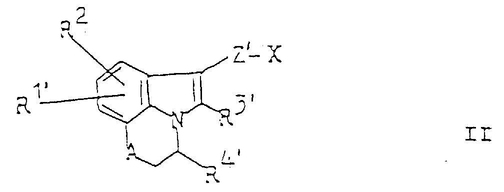 worin R^{1'}, R², R^{3'}, R^{4'}, A und Z' obige Bedeutung besitzen und X für eine aminolytisch abspaltbare Fluchtgruppe steht, mit Verbindungen der allgemeinen Formel III 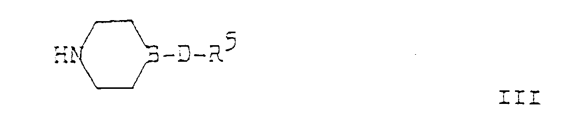 worin B, D und R⁵ obige Bedeutung besitzen, umsetzt, oder
b) zur Herstellung von Verbindungen der allgemeinen Formel Ib 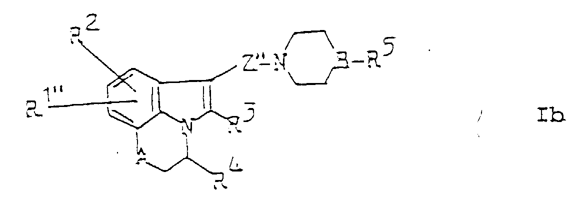 worin R², R³, R⁴, A, B und R⁵ obige Bedeutung besitzen, R^{1"} die für R¹ angegebenen Reste mit Ausnahme von CO-haltigen Resten darstellt und Z" eine -Z"'-CH₂-Kette darstellt, worin Z"' ein Alkylenkette mit 1 - 3 Kohlenstoffatomen, welche gegebenenfalls durch C₁-C₄-Alkyl oder auch durch Hydroxy substituiert sein kann, bedeutet, oder, falls R^{1"}, R³ und/oder R⁴ einen Hydroxyalkyl enthaltenden Rest bedeuten oder R^{1"} eine Mono- oder Di-C₁-C₄-alkylaminogruppe bedeudet, Z" auch die für Z' angegebene Bedeutung besitzen kann, Verbindungen der allgemeinen Formel IV 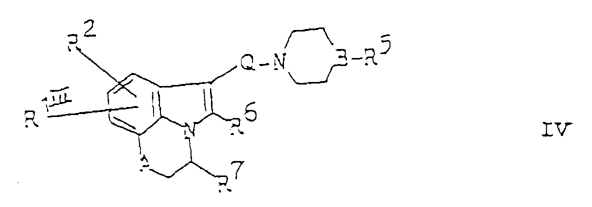 worin R², R⁵, A und B obige Bedeutung besitzen, R^{1III} die für R¹ angegebenen Reste mit Ausnahme von die -COO-Gruppe, die -CONH-Gruppe oder eine Hydroxyalkyl-Gruppe enthaltenden Resten bedeutet oder eine N-Formyl- oder N-C₁-C₄-alkanoyl-substituierte Amino- oder C₁-C₄-alkylaminogruppe bedeutet, R⁵ die für R³ angegebene Bedeutung besitzt oder eine C₁-C₄-alkoxycarbonyl- oder C₁-C₄-alkoxycarbonyl-C₁-C₄-alkylgruppe oder eine im Phenylring durch C₁-C₄-alkoxy-carbonyl-C₁-C₄-alkoxy substituierte Phenyl- oder Phenyl-C₁-C₄-alkylgruppe bedeutet, R⁷ die für R⁴ angegebene Bedeutung besitzt oder eine C₁-C₄-alkoxycarbonyl- -oder C₁-C₄-alkoxycarbonyl-C₁-C₄-alkylgruppe oder eine im Phenylring durch C₁-C₄-alkoxycarbonyl-C₁-C₄-alkoxy substituierte Phenyl- oder Phenyl-C₁-C₄-alkylgruppe bedeutet und Q eine -Q'-CO-Kette darstellt, worin Q' eine Alkylenkette mit 1 - 3 Kohlenstoffatomen, welche gegebenenfalls durch C₁-C₄-Alkyl oder durch Oxo substituiert sein kann, darstellt, oder Q eine in der dem Indolgerüst benachbarten Position durch Oxo substituierte Alkylenkette mit 2 - 4 Kohlenstoffatomen, welche gegebenenfalls durch C₁-C₄-Alkyl substituiert sein kann, bedeutet oder, falls R^{1III}, R⁶ und/oder R⁷ einen CO-haltigen Rest bedeuten, Q auch die für Z' angegebene Bedeutung besitzen kann, reduziert, oder
c) zur Herstellung von Verbindungen der allgemeinen Formel Ic 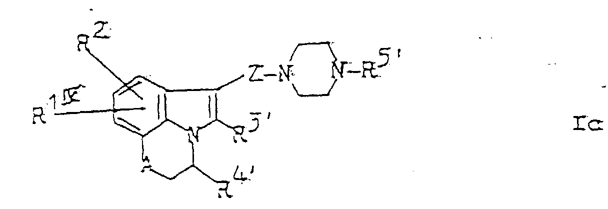 worin R², R^{3'}, R^{4'}, A und Z obige Bedeutung besitzen, R^{5'} für einen gegebenenfalls durch C₁-C₄-Alkyl, C₁-C₄-Alkoxy oder Halogen substituierten Pyridylrest steht und R^{1IV} die für R¹ angegebene Bedeutung mit Ausnahme von hydroxysubstituiertem C₁-C₇-Alkyl besitzt, Verbindungen der allgemeinen Formel V 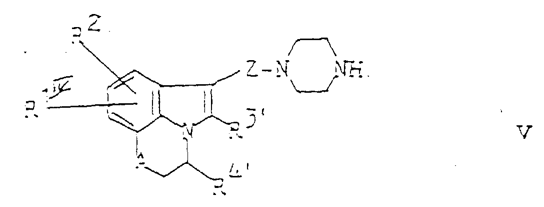 worin R^{1IV}, R², R^{3'}, R^{4'}, A und Z obige Bedeutung besitzen, mit einer Verbindung der allgemeinen Formel VI
X' -R^{5'} VI
worin R^{5'} obige Bedeutung besitzt und X' Halogen bedeutet, umsetzt, oder
d) zur Herstellung von Verbindungen der allgemeinen Formel Id 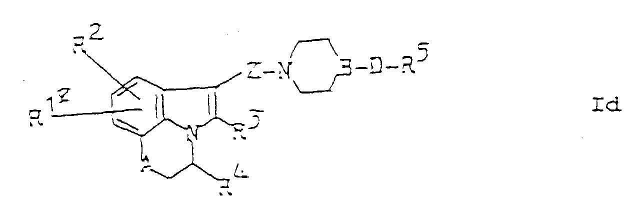 worin R², R³, R⁴, A, Z, B, D und R⁵ obige Bedeutung besitzen und R^{1V} C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl-, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet, Verbindungen der allgemeinen Formel Ie 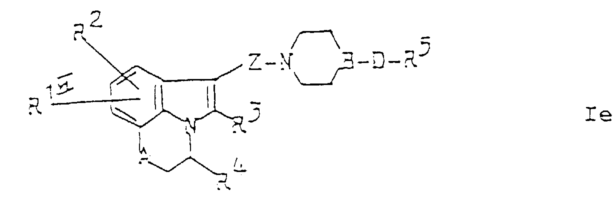 worin R², R³, R⁴, A, Z, B, D und R⁵ obige Bedeutung besitzen und R^{1VI} Wasserstoff, Amino oder, sofern R³, R⁴ und/oder Z keine freien Hydroxygruppen enthalten, auch Hydroxy bedeutet, mit Säuren oder reaktionsfähigen Säurederivaten der allgemeinen Formel VII
R⁸-Y VII
worin R⁸ C₂-C₇-Alkanoyl, eine Benzoylgruppe, deren Phenylring gegebenenfalls durch C₁-C₄-Alkyl, C₁-C₄-Alkoxy, Hydroxy oder Halogen substituiert sein kann, oder eine Cinnamoylgruppe, deren Phenylring gegebenenfalls durch C₁-C₄-Alkyl, C₁-C₄-Alkoxy, Hydroxy oder Halogen substituiert sein kann, bedeutet und Y Hydroxy oder eine reaktive Gruppe bedeutet, acyliert,
und gewünschtenfalls in erhaltenen Verbindungen der Formel I, worin R¹ Methoxy bedeutet oder eine Methoxyphenylgruppe enthält und/oder R³ und/oder R⁴ eine Methoxyphenylgruppe darstellen oder enthalten, die Methoxygruppe zur Hydroxygruppe spaltet und/oder in Verbindungen der Formel I, worin R¹, R³ und/oder R⁴ eine Hydroxyalkylgruppe mit mindestens 2 Kohlenstoffatomen enthalten, diese durch Wasserabspaltung in eine entsprechende Alkenylgruppe überführt oder in Verbindungen der Formel I, worin nur R¹ eine Hydroxyalkylgruppe enthält, diese zu der entsprechenden Alkanoylgruppe oxidiert und/oder Verbindungen der Formel I, worin R¹ Amino bedeutet, zu entsprechenden Verbindungen der Formel I, worin R¹ C₁-C₄-Mono- oder Dialkylamino bedeutet, alkyliert und/oder schwefelhaltige Verbindungen der Formel I in die entsprechenden S-Mono- oder -Dioxide überführt und gewünschtenfalls freie Verbindungen der Formel I in ihre Säureadditionssalze überführt oder die Säureadditionssalze in die freien Verbindungen der Formel I überführt.

11. Verbindungen der allgemeinen Formel IV 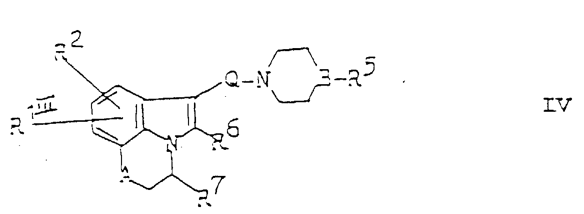 worin
R^{1III} Wasserstoff, C₁-C₄-Alkoxy, C₁-C₄-Alkylthio, Hydroxy, Halogen, Trifluormethyl, Nitro, Amino, C₁-C₄-Mono- oder Dialkylamino, C₁-C₇-Alkyl, eine Phenyl-C₁-C₄-alkylgruppe, welche im Phenylring gegebenenfalls durch C₁-C₄-Alkyl, C₁-C₄-Alkoxy Hydroxy oder Halogen substituiert sein kann, C₃-C₆-Cycloalkyl, C₄-C₇-Cycloalkylalkyl, C₃-C₇-Alkenyl, C₂-C₇-Alkanoyl, eine Benzoyl- oder Cinnamoylgruppe, deren Phenylring gegebenenfalls durch C₁-C₄-Alkyl, C₁-C₄-Alkoxy, Hydroxy oder Halogen substituiert sein kann, oder eine N-Formyl- oder N-C₁-C₄-alkanoyl-substituierte Amino- oder C₁-C₄-alkylaminogruppe bedeutet,
R² Wasserstoff, Halogen, C₁-C₄-Alkyl oder , falls R^{1"'} nicht Hydroxy oder eine hydroxyphenylhaltige Gruppe ist, auch C₁-C₄-Alkoxy bedeutet,
R⁵ Wasserstoff, C₁-C₄-Alkyl, welches gegebenenfalls durch Hydroxy substituiert sein kann, C₁-C₄-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, oder eine C₁-C₄-alkoxycarbonyl- oder C₁-C₄-alkoxycarbonyl-C₁-C₄-alkylgruppe oder eine im Phenylring durch C₁-C₄-alkoxycarbonyl-C₁-C₄-alkoxy substituierte Phenyl- oder Phenyl-C₁-C₄-alkylgruppe bedeutet,
R⁷ Wasserstoff, C₁-C₇-Alkyl, welches gegebenenfalls durch Hydroxy substituiert sein kann, C₃-C₇-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy, Hydroxy oder Hydroxyalkoxy substituiert sein kann, oder eine C₁-C₄-alkoxycarbonyl- oder C₁-C₄-alkoxycarbonyl-C₁-C₄-alkylgruppe oder eine im Phenylring durch C₁-C₄-alkoxycarbonyl-C₁-C₄-alkyl substituierte Phenyl- oder Phenyl-C₁-C₄-alkylgruppe bedeutet,
A eine gegebenenfalls durch C₁-C₄-Alkyl substituierte Alkylenkette mit 1 - 2 Kohlenstoffatomen, eine Bindung, Sauerstoff oder Schwefel bedeutet,
B Stickstoff oder die CH-Gruppe bedeutet,
R⁵ einem gegebenenfalls durch C₁-C₄-Alkyl, C₁-C₄-Alkoxy oder Halogen substituierten Pyridyl- oder Phenylrest bedeutet, und
Q eine -Q'-CO-Kette darstellt, worin Q' eine Alkylenkette mit 1 - 3 Kohlenstoffatomen, welche gegebenenfalls durch C₁-C₄-Alkyl oder durch Oxo substituiert sein kann, darstellt, oder Q eine in der dem Indolgerüst benachbarten Position durch Oxo substituierte Alkylenkette mit 2 - 4 Kohlenstoffatomen, welche gegebenenfalls durch C₁-C₄-Alkyl substituiert sein kann, bedeutet oder, falls R^{1III}, R⁶ und/oder R⁷ einen CO-haltigen Rest bedeuten, Q auch eine Alkylenkette mit 2 - 4 Kohlenstoffatomen, welche gegebenenfalls durch C₁-C₄-Alkyl oder auch in der dem Indolgerüst benachbarten Position durch Hydroxy substituiert sein kann, bedeuten kann.

12. Verbindungen der allgemeinen Formel V 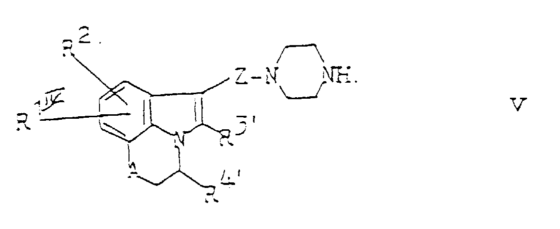 worin
R^{1IV} Wasserstoff, C₁-C₄-Alkoxy, C₁-C₄-Alkylthio, Hydroxy, Halogen, Trifluormethyl, Nitro, Amino, C₁-C₄-Mono- oder Dialkylamino, C₁-C₇-Alkyl, eine Phenyl-C₁-C₄-alkylgruppe, welche im Phenylring gegebenenfalls durch C₁-C₄-Alkyl, C₁-C₄-Alkoxy, Hydroxy oder Halogen substituiert sein kann, C₃-C₆-Cycloalkyl, C₄-C₇-Cycloalkylalkyl, C₃-C₇-Alkenyl, C₂-C₇-Alkanoyl, C₁-C₄-Alkanoyloxy, C₁-C₄-Alkanoylamino, eine Benzoyl-, Benzoyloxy- oder Benzoylaminogruppe, deren Phenylring gegebenenfalls durch C₁-C₄-Alkyl, C₁-C₄-Alkoxy, Hydroxy oder Halogen substituiert sein kann, oder eine Cinnamoyl-, Cinnamoyloxy- oder Cinnamoylaminogruppe, deren Phenylring gegebenenfalls durch C₁-C₄-Alkyl, C₁-C₄-Alkoxy, Hydroxy oder Halogen substituiert sein kann, bedeutet,
R² Wasserstoff, Halogen, C₁-C₄-Alkyl oder, falls R^{1IV} nicht Hydroxy oder eine hydroxyphenylhaltige Gruppe ist, auch C₁-C₄-Alkoxy bedeutet,
R^{3'} Wasserstoff; C₁-C₄-Alkyl, C₁-C₄-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy oder Hydroxy substituiert sein kann, bedeutet, wobei jedoch R^{3'} nur dann eine freie Hydroxygruppe enthalten kann, wenn R^{1IV} keine Carbonyloxygruppe enthält,
R^{4'} Wasserstoff, C₁-C₇-Alkyl, C₃-C₇-Alkenyl, C₃-C₆-Cycloalkyl, C₄-C₇-Cycloalkylalkyl oder eine Phenyl- oder Phenyl-C₁-C₄-alkylgruppe, welche im Phenylring gegebenenfalls durch C₁-C₄-Alkyl, Halogen, C₁-C₄-Alkoxy oder Hydroxy substituiert sein kann, bedeutet, wobei jedoch R^{4'} nur dann eine freie Hydroxygruppe enthalten kann, wenn R^{1IV} keine Carbonyloxygruppe enthält,
A eine gegebenenfalls durch C₁-C₄-Alkyl substituierte Alkylenkette mit 1 - 2 Kohlenstoffatomen, eine Bindung, Sauerstoff oder Schwefel bedeutet, und
Z eine Alkylenkette mit 2 - 4 Kohlenstoffatomen, welche gegebenenfalls durch C₁-C₄-Alkyl oder, falls R^{1IV} keine Carbonyloxygruppe enthält, auch durch Hydroxy substituiert sein kann, bedeutet.

## Claims

1. Compounds of the general formula I 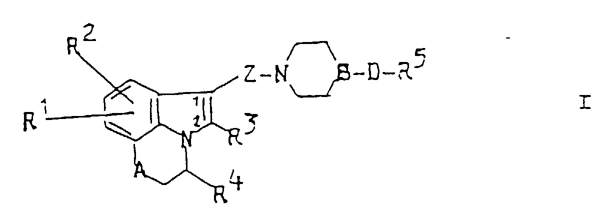 in which
R¹ denotes hydrogen, C₁-C₄-alkoxy, C₁-C₄-alkylthio, hydroxyl, halogen, trifluoromethyl, nitro, amino, C₁-C₄-mono- or dialkylamino, C₁-C₇-alkyl which can optionally be substituted by hydroxyl, or denotes a phenyl-C₁-C₄-alkyl group which can optionally be substituted in the phenyl ring by C₁-C₄-alkyl, C₁-C₄-alkoxy, hydroxyl or halogen, or denotes C₃-C₆-cycloalkyl, C4-C7-cycloalkylalkyl, C₃-C₇-alkenyl, C₂-C₇-alkanoyl, C₁-C₄-alkanoyloxy, C₁-C₄-alkanoylamino, a benzoyl, benzoyloxy or benzoylamino group whose phenyl ring can optionally be substituted by C₁-C₄-alkyl, C₁-C₄-alkoxy, hydroxyl or halogen, or denotes a cinnamoyl, cinnamoyloxy or cinnamoylamino group whose phenyl ring can optionally be substituted by C₁-C₄-alkyl, C₁-C₄-alkoxy, hydroxyl or halogen,
R² denotes hydrogen, halogen, C₁-C₄-alkyl or, if R¹ is not hydroxyl or a hydroxyphenyl-containing group, also denotes C₁-C₄-alkoxy,
R³ denotes hydrogen, C₁-C₄-alkyl which can optionally be substituted by hydroxyl, or denotes C₁-C₄-alkenyl, C₃-C₆-cycloalkyl, C₄-C₇-cycloalkylalkyl or a phenyl or phenyl-C₁-C₄-alkyl group, which can optionally be substituted in the phenyl ring by C₁-C₄-alkyl, halogen, C₁-C₄-alkoxy, hydroxyl or hydroxyalkoxy, but where R³ can only contain a free hydroxyl group if R¹ does not contain a carbonyloxy group,
R⁴ denotes hydrogen, C₁-C₇-alkyl which can optionally be substituted by hydroxyl, or denotes C₃-C₇-alkenyl, C₃-C₆-cycloalkyl, C₄-C₇-cycloalkylalkyl or a phenyl or phenyl-C₁-C₄-alkyl group which can optionally be substituted in the phenyl ring by C₁-C₄-alkyl, halogen, C₁-C₄-alkoxy, hydroxyl or hydroxyalkoxy, but where R⁴ can only contain a free hydroxyl group if R¹ does not contain a carbonyloxy group,
A denotes an alkylene chain having 1-2 carbon atoms, which is optionally substituted by C₁-C₄-alkyl, or denotes a bond, oxygen or sulphur,
Z denotes an alkylene chain having 2-4 carbon atoms, which can optionally be substituted by C₁-C₄-alkyl or, if R¹ does not contain a carbonyloxy group, also by hydroxyl,
B denotes nitrogen or the CH group,
R⁵ denotes a pyridyl or phenyl radical which is optionally substituted by C₁-C₄-alkyl, C₁-C₄-alkoxy or halogen, and
D represents a bond or, if B denotes the CH group and R⁵ denotes an optionally substituted phenyl radical, can also represent the CO group,
and their acid addition salts and/or S-mono- or dioxides of sulphur-containing compounds of the formula I.

2. Compounds according to Claim 1, in which R¹ denotes hydrogen, C₁-C₆-alkyl optionally substituted by hydroxyl, or denotes halogen, hydroxyl, C₁-C₄-alkoxy, C₁-C₄-alkanoyl, C₁-C₄-alkanoyloxy, C₁-C₄-alkanoylamino, or a benzoyl, benzoyloxy, benzoylamino, cinnamoyl, cinnamoyloxy or cinnamoylamino group optionally substituted by C₁-C₄-alkyl, C₁-C₄-alkoxy or halogen in the phenyl ring.

3. Compounds according to Claim 1 or 2, characterised in that R² denotes hydrogen.

4. Compounds according to one of the above claims, characterised in that R³ denotes C₁-C₄-alkyl.

5. Compounds according to one of the above claims, in which A denotes a methylene group optionally substituted by C₁-C₄-alkyl.

6. Compounds according to one of the above claims, characterised in that Z denotes an ethylene chain optionally substituted by hydroxyl or C₁-C₄-alkyl.

7. Compounds according to one of the above claims, characterised in that R⁵ denotes a pyridyl radical optionally substituted by C₁-C₄-alkyl, C₁-C₄-alkoxy or halogen.

8. Compounds according to Claim 7, characterised in that R⁵ represents the 4-methylpyrid-2-yl radical.

9. Medicaments containing a pharmacologically active amount of a compound according to Claim 1 and customary pharmaceutical auxiliaries and/or excipients.

10. Process for the preparation of compounds of the general formula I 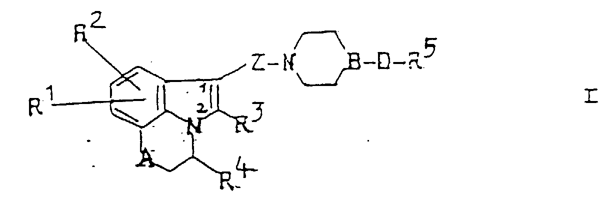 in which
R¹ denotes hydrogen, C₁-C₄-alkoxy, C₁-C₄-alkylthio, hydroxyl, halogen, trifluoromethyl, nitro, amino, C₁-C₄-mono- or dialkylamino, C₁-C₇-alkyl which can optionally be substituted by hydroxyl, or denotes a phenyl-C₁-C₄-alkyl group which can optionally be substituted in the phenyl ring by C₁-C₄-alkyl, C₁-C₄-alkoxy, hydroxyl or halogen, or denotes C₃-C₆-cycloalkyl, C₄-C₇-cycloalkylalkyl, C₃-C₇-alkenyl, C₂-C₇-alkanoyl, C₁-C₄-alkanoyloxy, C₁-C₄-alkanoylamino, a benzoyl, benzoyloxy or benzoylamino group whose phenyl ring can optionally be substituted by C₁-C₄-alkyl, C₁-C₄-alkoxy, hydroxyl or halogen, or denotes a cinnamoyl, cinnamoyloxy or cinnamoylamino group whose phenyl ring can optionally be substituted by C₁-C₄-alkyl, C₁-C₄-alkoxy, hydroxyl or halogen,
R² denotes hydrogen, halogen, C₁-C₄-alkyl or, if R¹ is not hydroxyl or a hydroxyphenylcontaining group, also denotes C₁-C₄-alkoxy,
R³ denotes hydrogen, C₁-C₄-alkyl which may optionally be substituted by hydroxyl, or denotes C₁-C₄-alkenyl, C₃-C₆-cycloalkyl, C₄-C₇-cycloalkylalkyl or a phenyl or phenyl-C₁-C₄-alkyl group, which can optionally be substituted in the phenyl ring by C₁-C₄-alkyl, halogen, C₁-C₄-alkoxy, hydroxyl or hydroxyalkoxy, but where R³ can only contain a free hydroxyl group if R¹ does not contain a carbonyloxy group,
R⁴ denotes hydrogen, C₁-C₇-alkyl which can optionally be substituted by hydroxyl, or denotes C₃-C₇-alkenyl, C₃-C₆-cycloalkyl, C₄-C₇-cycloalkylalkyl or a phenyl or phenyl-C₁-C₄-alkyl group which can optionally be substituted in the phenyl ring by C₁-C₄-alkyl, halogen, C₁-C₄-alkoxy, hydroxyl or hydroxyalkoxy, but where R⁴ can only contain a free hydroxyl group if R¹ does not contain a carbonyloxy group,
A denotes an alkylene chain having 1-2 carbon atoms, which is optionally substituted by C₁-C₄-alkyl, or denotes a bond, oxygen or sulphur,
Z denotes an alkylene chain having 2-4 carbon atoms, which can optionally be substituted by C₁-C₄-alkyl or, if R¹ does not contain a carbonyloxy group, also by hydroxyl,
B denotes nitrogen or the CH group,
R⁵ denotes a pyridyl or phenyl radical which is optionally substituted by C₁-C₄-alkyl, C₁-C₄-alkoxy or halogen, and
D represents a bond or, if B denotes the CH group and R⁵ denotes an optionally substituted phenyl radical, can also represent the CO group,
and their acid addition salts and/or S-mono- or dioxides of sulphur-containing compounds of the formula I,
characterised in that
a) for the preparation of compounds of the general formula Ia 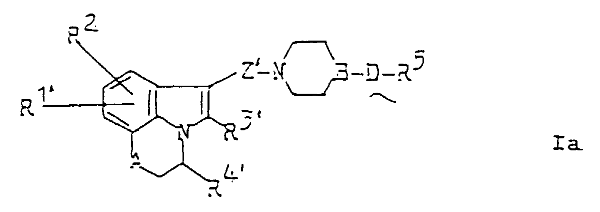 in which R^{1'} has the meaning given for R¹ with the exception of hydroxy-substituted C₁-C₇-alkyl or mono- or di-C₁-C₄-alkylamino, R^{3'} and R^{4'} denote the radicals given for R³ and R⁴ with the exception of radicals containing C₁-C₄-hydroxyalkyl groups, Z' has the meaning given for Z, but where the alkylene chain Z' can contain a possible hydroxyl substituent only in the position adjacent to the indole structure, and R², R⁵, A, B and D have the above meaning, compounds of the general formula II 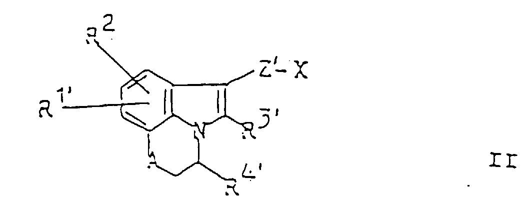 in which R^{1'} R², R^{3'}, R^{4'}, A and Z' have the above meaning and X represents a leaving group which can be eliminated by aminolysis, are reacted with compounds of the general formula III 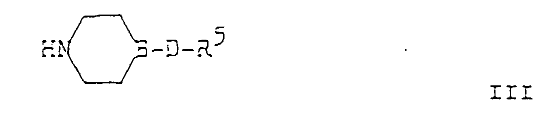 in which B, D and R⁵ have the above meaning, or
b) for the preparation of compounds of the general formula Ib 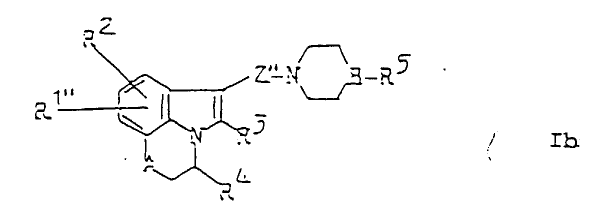 in which R², R³, R⁴, A, B and R⁵ have the above meaning, R^{1"} represents the radicals given for R¹ with the exception of CO-containing radicals and Z" represents a -Z"'-CH₂- chain, in which Z"' denotes an alkylene chain having 1-3 carbon atoms, which can optionally be substituted by C₁-C₄-alkyl or else by hydroxyl, or, if R^{1"}, R³ and/or R⁴ denote a radical containing hydroxyalkyl or R^{1"} denotes a mono- or di-C₁-C₄-alkylamino group, Z" also can have the meaning given for Z', compounds of the general formula IV 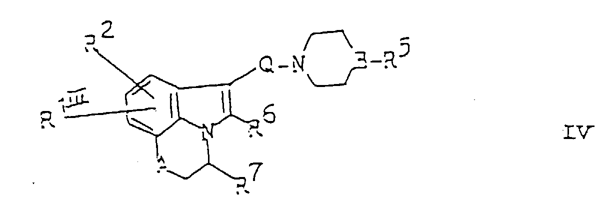 in which R², R⁵, A and B have the above meaning, R^{1III} denotes the radicals given for R¹ with the exception of radicals containing the -COO- group, the -CONH group or radicals containing a hydroxyalkyl group or denotes an N-formylsubstituted or N-C₁-C₄-alkanoyl-substituted amino or C₁-C₄-alkylamino group, R⁶ has the meaning given for R³ or denotes a C₁-C₄-alkoxycarbonyl or C₁-C₄-alkoxycarbonyl-C₁-C₄-alkyl group or a phenyl or phenyl-C₁-C₄-alkyl group substituted in the phenyl ring by C₁-C₄-alkoxycarbonyl-C₁-C₄-alkoxy, R⁷ has the meaning given for R⁴ or denotes a C₁-C₄-alkoxycarbonyl or C₁-C₄-alkoxycarbonyl-C₁-C₄-alkyl group or a phenyl or phenyl-C₁-C₄-alkyl group substituted in the phenyl ring by C₁-C₄-alkoxycarbonyl-C₁-C₄-alkoxy and Q represents a -Q'-CO chain, in which Q' represents an alkylene chain having 1-3 carbon atoms, which can optionally be substituted by C₁-C₄-alkyl or by oxo, or Q denotes a alkylene chain having 2-4 carbon atoms, which is substituted in the position adjacent to the indole structure by oxo and which can optionally be substituted by C₁-C₄-alkyl, or, if R^{1III}, R⁶ and/or R⁷ denote a CO-containing radical, Q also can have the meaning given for Z', are reduced, or
c) for the preparation of compounds of the general formula Ic 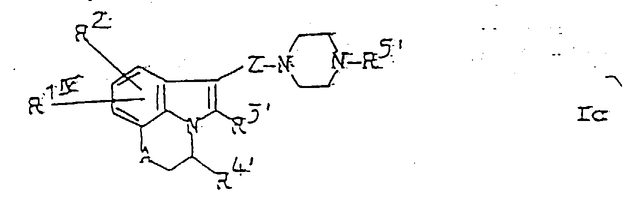 in which R², R^{3'}, R^{4'}, A and Z have the above meaning, R^{5'} represents a pyridyl radical which is optionally substituted by C₁-C₄-alkyl, C₁-C₄-alkoxy or halogen and R^{1IV} has the meaning given for R¹ with the exception of hydroxy-substituted C₁-C₇-alkyl, compounds of the general formula V 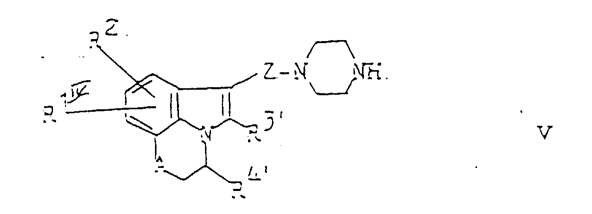 in which R^{1IV}, R², R^{3'}, R^{4'}, A and Z have the above meaning, are reacted with a compound of the general formula VI
X' - R^{5'} VI
in which R^{5'} has the above meaning and X' denotes halogen, or
d) for the preparation of compounds of the general formula Id 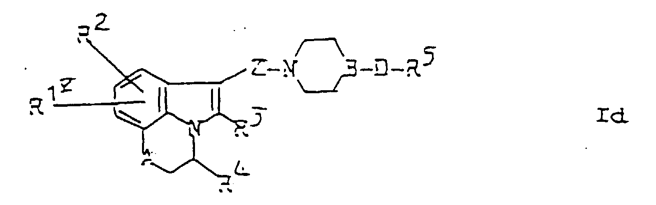 in which R², R³, R⁴, A, Z, B, D and R⁵ have the above meaning and R^{1V} denotes C₂-C₇-alkanoyl, C₁-C₄-alkanoyloxy, C₁-C₄-alkanoylamino, a benzoyl, benzoyloxy or benzoylamino group whose phenyl ring can optionally be substituted by C₁-C₄-alkyl, C₁-C₄-alkoxy, hydroxyl or halogen, or denotes a cinnamoyl, cinnamoyloxy or cinnamoylamino group whose phenyl ring can optionally be substituted by C₁-C₄-alkyl, C₁-C₄-alkoxy, hydroxyl or halogen, compounds of the general formula Ie 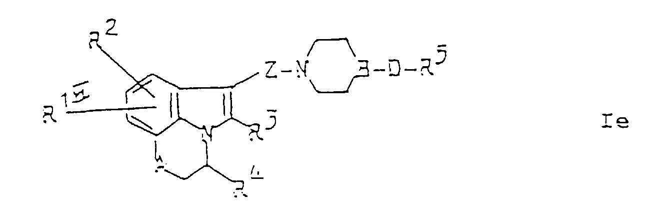 in which R², R³, R⁴, A, Z, B, D and R⁵ have the above meaning and R^{1VI} denotes hydrogen, amino or, if R³, R⁴ and/or Z do not contain any free hydroxyl groups, also denotes hydroxyl, are acylated with acids or reactive acid derivatives of the general formula VII
R⁸-Y VII
in which R⁸ denotes C₂-C₇-alkanoyl, a benzoyl group whose phenyl ring can optionally be substituted by C₁-C₄-alkyl, C₁-C₄-alkoxy, hydroxyl or halogen, or denotes a cinnamoyl group whose phenyl ring can optionally be substituted by C₁-C₄-alkyl, C₁-C₄-alkoxy, hydroxyl or halogen, and Y denotes hydroxyl or a reactive group,
and, if desired, in resulting compounds of the formula I, in which R¹ denotes methoxy or contains a methoxyphenyl group and/or R³ and/or R⁴ represent or contain a methoxyphenyl group, the methoxy group is cleaved to give the hydroxyl group and/or in compounds of the formula I in which R¹, R³ and/or R⁴ contain a hydroxyalkyl group having at least 2 carbon atoms, this group is converted by elimination of water into a corresponding alkenyl group or in compounds of the formula I in which only R¹ contains a hydroxyalkyl group, this group is oxidised to the corresponding alkanoyl group and/or compounds of the formula I in which R¹ denotes amino are alkylated to give corresponding compounds of the formula I in which R¹ denotes C₁-C₄-mono- or dialkylamino, and/or sulphur-containing compounds of the formula I are converted into the corresponding S-mono- or -dioxides and, if desired, free compounds of the formula I are converted into their acid addition salts or the acid addition salts are converted into the free compounds of the formula I.

11. Compounds of the general formula IV 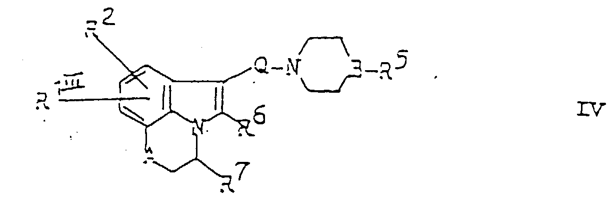 in which
R^{1III} denotes hydrogen, C₁-C₄-alkoxy, C₁-C₄-alkylthio, hydroxyl, halogen, trifluoromethyl, nitro, amino, C₁-C₄-mono- or dialkylamino, C₁-C₇-alkyl, a phenyl-C₁-C₄-alkyl group which can optionally be substituted in the phenyl ring by C₁-C₄-alkyl, C₁-C₄-alkoxy, hydroxyl or halogen, or denotes C₃-C₆-cycloalkyl, C₄-C₇-cycloalkylalkyl, C₃-C₇-alkenyl, C₂-C₇-alkanoyl, a benzoyl or cinnamoyl group whose phenyl ring can optionally be substituted by C₁-C₄-alkyl, C₁-C₄-alkoxy, hydroxyl or halogen, or denotes an N-formylsubstituted or N-C₁-C₄-alkanoyl-substituted amino or C₁-C₄-alkylamino group,
R² denotes hydrogen, halogen, C₁-C₄-alkyl or, if R^{1"'} is not hydroxyl or a hydroxyphenylcontaining group, also denotes C₁-C₄-alkoxy,
R⁶ denotes hydrogen, C₁-C₄-alkyl which can optionally be substituted by hydroxyl, or denotes C₁-C₄-alkenyl, C₃-C₆-cycloalkyl, C₄-C₇-cycloalkylalkyl or a phenyl or phenyl-C₁-C₄-alkyl group which can optionally be substituted in the phenyl ring by C₁-C₄-alkyl, halogen, C₁-C₄-alkoxy, hydroxyl or hydroxyalkoxy, or denotes a C₁-C₄-alkoxycarbonyl or C₁-C₄-alkoxycarbonyl-C₁-C₄-alkyl group or a phenyl or phenyl-C₁-C₄-alkyl group substituted in the phenyl ring by C₁-C₄-alkoxycarbonyl-C₁-C₄-alkoxy,
R⁷ denotes hydrogen, C₁-C₇-alkyl which can optionally be substituted by hydroxyl, or denotes C₃-C₇-alkenyl, C₃-C₆-cycloalkyl, C₄-C₇-cycloalkylalkyl or a phenyl or phenyl-C₁-C₄-alkyl group which can optionally be substituted in the phenyl ring by C₁-C₄-alkyl, halogen, C₁-C₄-alkoxy, hydroxyl or hydroxyalkoxy, or denotes a C₁-C₄-alkoxycarbonyl or C₁-C₄-alkoxycarbonyl-C₁-C₄-alkyl group or a phenyl or phenyl-C₁-C₄-alkyl group substituted in the phenyl ring by C₁-C₄-alkoxycarbonyl-C₁-C₄-alkyl,
A denotes an alkylene chain having 1-2 carbon atoms, which is optionally substituted by C₁-C₄-alkyl, a bond, oxygen or sulphur,
B denotes nitrogen or the CH group,
R⁵ denotes a pyridyl or phenyl radical which is optionally substituted by C₁-C₄-alkyl, C₁-C₄-alkoxy or halogen, and
Q represents a -Q'-CO chain, in which Q' represents an alkylene chain having 1-3 carbon atoms, which can optionally be substituted by C₁-C₄-alkyl or by oxo, or Q denotes an alkylene chain having 2-4 carbon atoms substituted in the position adjacent to the indole structure by oxo, which can optionally be substituted by C₁-C₄-alkyl, or, if R^{1III}, R⁶ and/or R⁷ denote a CO-containing radical, Q can also denote an alkylene chain having 2-4 carbon atoms, which can optionally be substituted by C₁-C₄-alkyl or else by hydroxyl in the position adjacent to the indole structure.

12. Compounds of the general formula V 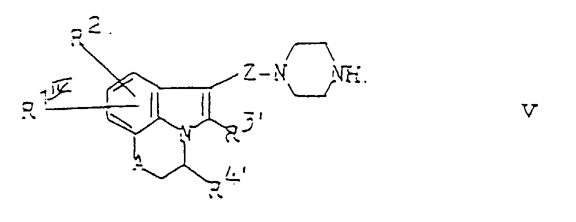 in which
R^{1IV} denotes hydrogen, C₁-C₄-alkoxy, C₁-C₄-alkylthio, hydroxyl, halogen, trifluoromethyl, nitro, amino, C₁-C₄-mono- or dialkylamino, C₁-C₇-alkyl, a phenyl-C₁-C₄-alkyl group which can optionally be substituted in the phenyl ring by C₁-C₄-alkyl, C₁-C₄-alkoxy, hydroxyl or halogen, or denotes C₃-C₆-cycloalkyl, C₄-C₇-cycloalkylalkyl, C₃-C₇-alkenyl, C₂-C₇-alkanoyl, C₁-C₄-alkanoyloxy, C₁-C₄-alkanoylamino, a benzoyl, benzoyloxy or benzoylamino group whose phenyl ring can optionally be substituted by C₁-C₄-alkyl, C₁-C₄-alkoxy, hydroxyl or halogen, or denotes a cinnamoyl, cinnamoyloxy or cinnamoylamino group whose phenyl ring can optionally be substituted by C₁-C₄-alkyl, C₁-C₄-alkoxy, hydroxyl or halogen,
R² denotes hydrogen, halogen, C₁-C₄-alkyl or, if R^{1IV} is not hydroxyl or a hydroxyphenyl-containing group, also denotes C₁-C₄-alkoxy,
R^{3'} denotes hydrogen, C₁-C₄-alkyl, C₁-C₄-alkenyl, C₃-C₆-cycloalkyl, C₄-C₇-cycloalkylalkyl or a phenyl or phenyl-C₁-C₄-alkyl group which can optionally be substituted in the phenyl ring by C₁-C₄-alkyl, halogen, C₁-C₄-alkoxy or hydroxyl, but where R^{3'} can only contain a free hydroxyl group if R^{1IV} does not contain a carbonyloxy group,
R^{4'} denotes hydrogen, C₁-C₇-alkyl, C₃-C₇-alkenyl, C₃-C₆-cycloalkyl, C₄-C7-cycloalkylalkyl or a phenyl or phenyl-C₁-C₄-alkyl group which can optionally be substituted in the phenyl ring by C₁-C₄-alkyl, halogen, C₁-C₄-alkoxy or hydroxyl, but where R^{4'} can only contain a free hydroxyl group if R^{1IV} does not contain a carbonyloxy group,
A denotes an alkylene chain having 1-2 carbon atoms, which is optionally substituted by C₁-C₄-alkyl, or denotes a bond, oxygen or sulphur, and
z denotes an alkylene chain having 2-4 carbon atoms, which can optionally be substituted by C₁-C₄-alkyl or, if R^{1IV} does not contain a carbonyloxy group, also by hydroxyl.

## Revendications

1. Composés de formule générale I 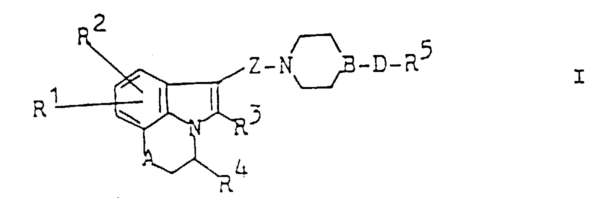 dans laquelle
R¹ représente un atome d'hydrogène ou d'halogène, ou un groupe alcoxy en C₁-C_{4,} alkylthio en C₁-C₄, hydroxy, trifluorométhyle, nitro, amino, mono- ou dialkylamino en C₁-C₄ , alkyle en C₁-C₇ qui peut éventuellement être substitué par le radical hydroxy, un groupe phényl-alkyle en C₁-C₄ qui peut éventuellement être substitué sur le noyau phényle par un substituant alkyle en C₁-C₄ , alcoxy en C₁-C₄ , hydroxy ou halogène, un radical cycloalkyle en C₃-C₆, cycloalkylalkyle en C₄-C₇, alcényle en C₃-C₇, alcanoyle en C₂-C₇, alcanoyloxy en C₁-C₄ , alcanoylamino en C₁-C₄ , un groupe benzoyle, benzoyloxy ou benzoylamino dont le noyau phényle peut éventuellement être substitué par un substituant alkyle en C₁-C₄, alcoxy en C₁-C₄, hydroxy ou halogène, ou un groupe cinnamoyle, cinnamoyloxy ou cinnamoylamino, dont le noyau phényle peut éventuellement être substitué par un subtituant alkyle en C₁-C₄, alcoxy en C₁-C₄, hydroxy ou halogène;
R² représente un atome d'hydrogène ou d'halogène ou un groupe alkyle en C₁-C₄, ou, lorsque R¹ n'est pas un groupe hydroxy ni un groupe contenant le radical hydroxyphényle, également un groupe alcoxy en C₁-C₄;
R³ représente un atome d'hydrogène ou un radical alkyle en C₁-C₄ qui peut éventuellement être substitué par le groupe hydroxy, un radical alcényle en C₁-C₄ , cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C_{4,} halogène, alcoxy en C₁-C₄ , hydroxy ou hydroxyalcoxy, R³ ne pouvant toutefois contenir qu'un seul groupe hydroxy libre lorsque R¹ ne contient pas de groupe carbonyloxy;
R⁴ représente un atome d'hydrogène ou un radical alkyle en C₁-C₇ qui peut éventuellement être substitué par le groupe hydroxy, un radical alcényle en C₃-C₇, cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄ , halogène, alcoxy en C₁-C₄ , hydroxy ou hydroxyalcoxy, R⁴ ne pouvant toutefois contenir qu'un seul groupe hydroxy libre lorsque R¹ ne contient pas de groupe carbonyloxy;
A représente une chaîne alkylène ayant 1 ou 2 atomes de carbone, éventuellement substituée par un groupe alkyle en C₁-C₄ , ou une liaison ou un atome d'oxygène ou de soufre;
Z représente une chaîne alkylène ayant de 2 à 4 atomes de carbone, qui peut éventuellement être substituée par un groupe alkyle en C₁-C₄ ou, lorsque R¹ ne contient pas de groupe carbonyloxy, également par le radical hydroxy;
B représente un atome d'azote ou le groupe CH;
R⁵ représente un radical pyridyle ou phényle éventuellement substitué par un substituant alkyle en C₁-C₄, alcoxy en C₁-C₄ ou halogène; et
D représente une liaison, ou, lorsque B représente le groupe CH et R⁵ représente un radical phényle éventuellement substitué, également le groupe CO;
et leurs sels d'addition avec des acides et/ou S-mono- ou -dioxydes de composés soufrés de formule I.

2. Composés selon la revendication 1, dans lesquels R¹ représente un atome d'hydrogène ou d'halogène ou un groupe alkyle en C₁-C₆ éventuellement substitué par le radical hydroxy, un groupe hydroxy, alcoxy en C₁-C₄, alcanoyle en C₁-C₄, alcanoyloxy en C₁-C₄, alcanoylamino en C₁-C₄, ou un groupe benzoyle, benzoyloxy, benzoylamino, cinnamoyle, cinnamoyloxy ou cinnamoylamino, éventuellement substitués sur le noyau phényle par un substituant alkyle en C₁-C₄, alcoxy en C₁-C₄ ou halogène.

3. Composés selon la revendication 1 ou 2, caractérisés en ce que R² représente un atome d'hydrogène.

4. Composés selon l'une des revendications précédentes, caractérisés en ce que R³ représente un groupe alkyle en C₁-C₄ .

5. Composés selon l'une des revendications précédentes, dans lesquels A représente un groupe méthylène éventuellement substitué par un radical alkyle en C₁-C₄.

6. Composés selon l'une des revendications précédentes, caractérisés en ce que Z représente une chaîne éthylène éventuellement substituée par un groupe hydroxy ou alkyle en C₁-C₄.

7. Composés selon l'une des revendications précédentes, caractérisés en ce que R⁵ représente un radical pyridyle éventuellement substitué par un substituant alkyle en C₁-C₄ , alcoxy en C₁-C₄ ou halogène.

8. Composés selon la revendication 7, caractérisés en ce que R⁵ représente le radical 4-méthylpyrid-2-yle.

9. Médicament contenant une quantité pharmacologiquement efficace d'un composé selon la revendication 1 et des véhicules et/ou adjuvants pharmaceutiques usuels.

10. Procédé pour la préparation de composés de formule générale I 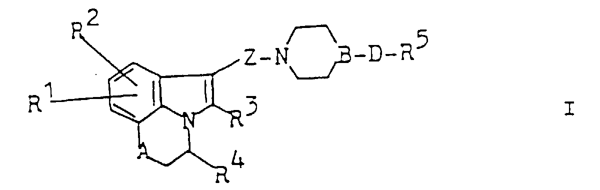 dans laquelle
R¹ représente un atome d'hydrogène ou d'halogène, ou un groupe alcoxy en C₁-C₄ , alkylthio en C₁-C₄ , hydroxy, trifluorométhyle, nitro, amino, mono- ou dialkylamino en C₁-C₄, alkyle en C₁-C₇ qui peut éventuellement être substitué par le radical hydroxy, un groupe phényl-alkyle en C₁-C₄ qui peut éventuellement être substitué sur le noyau phényle par un substituant alkyle en C₁-C₄ , alcoxy en C₁-C₄ , hydroxy ou halogène, un radical cycloalkyle en C₃-C₆, cycloalkylalkyle en C₄-C₇, alcényle en C₃-C₇, alcanoyle en C₂-C₇, alcanoyloxy en C₁-C₄, alcanoylamino en C₁-C₄ , un groupe benzoyle, benzoyloxy ou benzoylamino dont le noyau phényle peut éventuellement être substitué par un substituant alkyle en C₁-C₄ , alcoxy en C₁-C₄ , hydroxy ou halogène, ou un groupe cinnamoyle, cinnamoyloxy ou cinnamoylamino, dont le noyau phényle peut éventuellement être substitué par un subtituant alkyle en C₁-C₄, alcoxy en C₁-C₄, hydroxy ou halogène;
R² représente un atome d'hydrogène ou d'halogène ou un groupe alkyle en C₁-C₄ , ou, lorsque R¹ n'est pas un groupe hydroxy ni un groupe contenant le radical hydroxyphényle, également un groupe alcoxy en C₁-C₄ ;
R³ représente un atome d'hydrogène ou un radical alkyle en C₁-C₄ qui peut éventuellement être substitué par le groupe hydroxy, un radical alcényle en C₁-C₄ , cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄, halogène, alcoxy en C₁-C₄ , hydroxy ou hydroxyalcoxy, R³ ne pouvant toutefois contenir qu'un seul groupe hydroxy libre lorsque R¹ ne contient pas de groupe carbonyloxy;
R⁴ représente un atome d'hydrogène ou un radical alkyle en C₁-C₇ qui peut éventuellement être substitué par le groupe hydroxy, un radical alcényle en C₃-C₇, cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄ , halogène, alcoxy en C₁-C₄ , hydroxy ou hydroxyalcoxy, R⁴ ne pouvant toutefois contenir qu'un seul groupe hydroxy libre lorsque R¹ ne contient pas de groupe carbonyloxy;
A représente une chaîne alkylène ayant 1 ou 2 atomes de carbone, éventuellement substituée par un groupe alkyle en C₁-C₄ , ou une liaison ou un atome d'oxygène ou de soufre;
Z représente une chaîne alkylène ayant de 2 à 4 atomes de carbone, qui peut éventuellement être substituée par un groupe alkyle en C₁-C₄ ou, lorsque R¹ ne contient pas de groupe carbonyloxy, également par le radical hydroxy;
B représente un atome d'azote ou le groupe CH;
R⁵ représente un radical pyridyle ou phényle éventuellement substitué par un substituant alkyle en C₁-C₄ , alcoxy en C₁-C₄ ou halogène; et
D représente une liaison, ou, lorsque B représente le groupe CH et R⁵ représente un radical phényle éventuellement substitué, également le groupe CO;
et de leurs sels d'addition avec des acides et/ou de S-mono- ou -dioxydes de composés soufrés de formule I, caractérisé en ce que
a) pour la préparation de composés de formule générale Ia 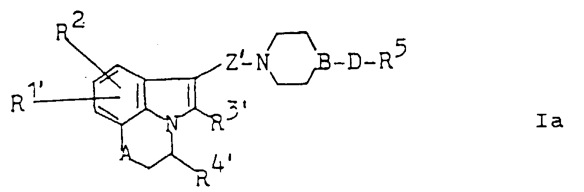 dans laquelle R^{1'} a la signification donnée pour R¹, à l'exception d'un radical alkyle en C₁-C₇ substitué par le groupe hydroxy, ou d'un radical mono- ou di(alkyl en C₁-C₄)-amino, R^{3'} et R^{4'} représentent les radicaux indiqués pour R³ et R⁴, à l'exception de radicaux contenant des groupes hydroxyalkyle en C₁-C₄ , Z' a la signification donnée pour Z, la chaîne alkylène Z' ne pouvant toutefois contenir qu'un éventuel substituant hydroxy en position voisine du squelette indole, et R², R⁵, A, B et D ont les significations données plus haut, on fait réagir des composés de formule générale II 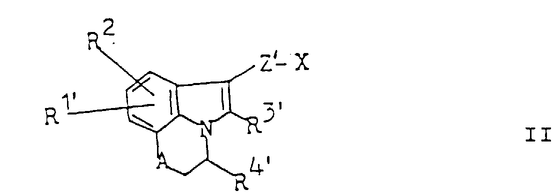 dans laquelle R^{1'}, R², R^{3'}, R^{4'}, A et Z' ont les significations données plus haut, et X représente un groupe séparable par aminolyse, avec des composés de formule générale III 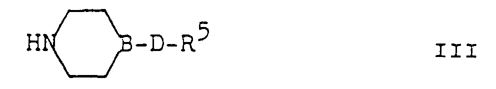 dans laquelle B, D et R⁵ ont les significations données plus haut, ou
b) pour la préparation de composés de formule générale Ib 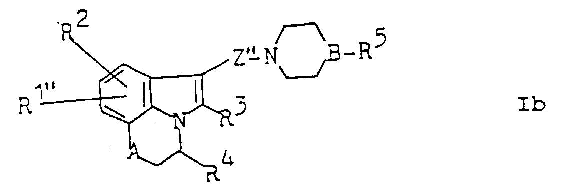 dans laquelle R², R³, R⁴, A, B et R⁵ ont les significations données plus haut, R^{1"} représente les radicaux indiqués pour R¹ à l'exception de radicaux contenant CO, et Z" représente une chaîne -Z"'-CH₂, dans laquelle Z"' représente une chaîne alkylène ayant de 1 à 3 atomes de carbone, qui peut éventuellement être substituée par un groupe alkyle en C₁-C₄ ou bien par le groupe hydroxy, ou, lorsque R^{1"}, R³ et/ou R⁴ représentent un radical contenant un groupe hydroxyalkyle, ou R^{1"} représente un groupe mono- ou di(alkyl en C₁-C₄ )amino, Z" peut également avoir la signification indiquée pour Z', on réduit des composés de formule générale IV 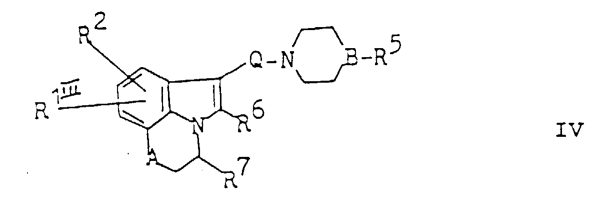 dans laquelle R², R⁵, A et B ont les significations données plus haut, R^{1III} représente les radicaux indiqués pour R¹ à l'exception du groupe -COO, du groupe -CONH ou d'un radical contenant un groupe hydroxyalkyle, ou représente un groupe amino ou (alkyl en C₁-C₄)-amino substitué par un radical N-formyle ou N-alcanoyle en C₁-C₄, R⁶ a la signification donnée pour R³ ou représente un groupe (alcoxy en C₁-C₄)-carbonyle ou (alcoxy en C₁-C₄)-carbonyl-alkyle en C₁-C₄ ou un groupe phényle ou phényl-alkyle en C₁-C₄ substitué sur le noyau phényle par un groupe (alcoxy en C₁-C₄)-carbonyl-alcoxy en C₁-C₄, R⁷ a la signification donnée pour R⁴ ou représente un groupe (alcoxy en C₁-C₄ )-carbonyle ou (alcoxy en C₁-C₄ )-carbonyl-alkyle en C₁-C₄ ou un groupe phényle ou phényl-alkyle en C₁-C₄ substitué sur le noyau phényle par un groupe (alcoxy en C₁-C₄ )-carbonyl-alcoxy en C₁-C₄ , et Q représente une chaîne -Q'-CO-, dans laquelle Q' représente une chaîne alkylène ayant de 1 à 3 atomes de carbone, qui peut éventuellement être substituée par un groupe alkyle en C₁-C₄ ou par le groupe oxo, ou Q représente une chaîne alkylène ayant de 2 à 4 atomes de carbone, substituée par le groupe oxo en la position voisine du squelette indole, qui peut éventuellement être substituée par un groupe alkyle en C₁-C₄, ou, lorsque R^{1III}, R⁶ et/ou R⁷ représentent un radical contenant CO, Q peut également avoir la signification donnée pour Z', ou
c) pour la préparation de composés de formule générale Ic 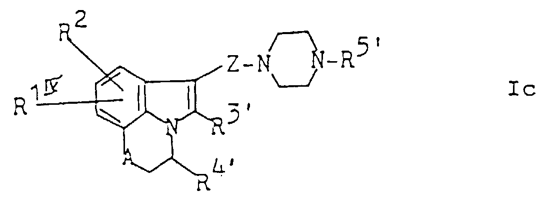 dans laquelle R², R^{3'}, R^{4'}, A et Z ont les significations données plus haut, R^{5'} représente un radical pyridyle éventuellement substitué par un substituant alkyle en C₁-C₄, alcoxy en C₁-C₄ ou halogène, et R^{1IV} a la signification donnée pour R¹, à l'exception d'un groupe alkyle en C₁-C₇ substitué par le radical hydroxy, on fait réagir des composés de formule générale V 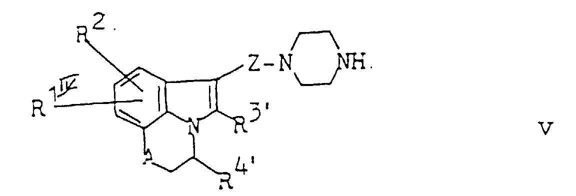 dans laquelle R^{1IV}, R², R^{3'}, R^{4'}, A et Z ont les significations données plus haut, avec un composé de formule générale VI
X' -R^{5'} VI
dans laquelle R^{5'} a la signification donnée plus haut et X' représente un atome d'halogène, ou
d) pour la préparation de composés de formule générale Id 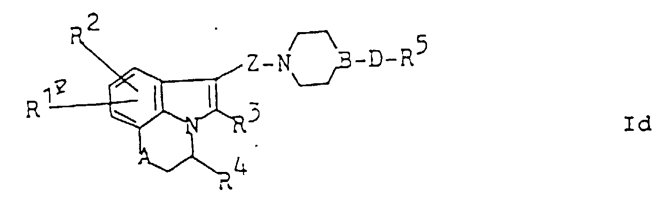 dans laquelle R², R³, R⁴, A, Z, B, D et R⁵ ont les significations données plus haut et R^{1V} représente un radical alcanoyle en C₂-C₇, alcanoyloxy en C₁-C₄, alcanoylamino en C₁-C₄ , un radical benzoyle, benzoyloxy ou benzoylamino dont le noyau phényle peut éventuellement être substitué par un substituant alkyle en C₁-C₄ , alcoxy en C₁-C₄ , hydroxy ou halogène, ou un radical cinnamoyle, cinnamoyloxy ou cinnamoylamino, dont le noyau phényle peut éventuellement être substitué par un substituant alkyle en C₁-C₄ , alcoxy en C₁-C₄ , hydroxy ou halogène, on soumet à une acylation des composés de formule générale Ie 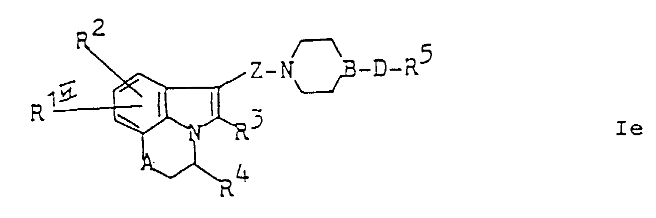 dans laquelle R², R³, R⁴, A, Z, B, D et R⁵ ont les significations données plus haut, et R^{1VI} représente un atome d'hydrogène, le groupe amino ou, lorsque R³, R⁴ et/ou Z ne contiennent pas de groupes hydroxy libres, également le groupe hydroxy, avec des acides ou des dérivés réactifs d'acides de formule générale VII
R⁸-Y VII
dans laquelle R⁸ représente un groupe alcanoyle en C₂-C₇, un groupe benzoyle dont le noyau phényle peut éventuellement être substitué par un substituant alkyle en C₁-C₄, alcoxy en C₁-C₄ , hydroxy ou halogène, ou un groupe cinnamoyle dont le noyau phényle peut éventuellement être substitué par un substituant alkyle en C₁-C₄, alcoxy en C₁-C₄ , hydroxy ou halogène, et Y représente le groupe hydroxy ou un groupe réactif,
et, si on le désire, dans des composés de formule I obtenus, dans lesquels R¹ représente le groupe méthoxy ou contient un groupe méthoxyphényle, et/ou R³ et/ou R⁴ représentent ou contiennent un groupe méthoxyphényle, on dissocie le groupe méthoxy en le groupe hydroxy, et/ou dans des composés de formule I, dans lesquels R¹, R³ et/ou R⁴ contiennent un groupe hydroxyalkyle ayant au moins 2 atomes de carbone, on convertit celui-ci par déshydratation en un groupe alcényle correspondant, ou, dans des composés de formule I dans lesquels seul R¹ contient un groupe hydroxyalkyle, on oxyde celui-ci pour aboutir au groupe alcanoyle correspondant, et/ou on soumet à une alkylation des composés de formule I, dans lesquels R¹ représente le groupe amino, pour aboutir aux composés de formule I correspondants dans lesquels R¹ représente un groupe mono- ou dialkylamino en C₁-C₄, et/ou on convertit des composés soufrés de formule I en les S-mono- ou -dioxydes correspondants, et, si on le désire, on convertit des composés de formule I libres en leurs sels d'addition avec des acides, ou on convertit les sels d'addition avec des acides en les composés de formule I libres.

11. Composés de formule générale IV 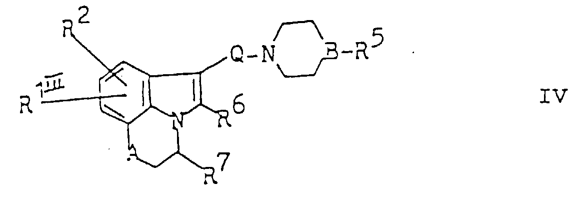 dans laquelle
R^{1III} représente un atome d'hydrogène ou d'halogène, ou un groupe alcoxy en C₁-C₄, alkylthio en C₁-C₄, hydroxy, trifluorométhyle, nitro, amino, mono- ou dialkylamino en C₁-C₄ , alkyle en C₁-C₇, un groupe phényl-alkyle en C₁-C₄ qui peut éventuellement être substitué sur le noyau phényle par un substituant alkyle en C₁-C₄ , alcoxy en C₁-C₄, hydroxy ou halogène, un radical cycloalkyle en C₃-C₆, cycloalkylalkyle en C₄-C₇, alcényle en C₃-C₇, alcanoyle en C₂-C₇, un groupe benzoyle ou cinnamoyle dont le noyau phényle peut éventuellement être substitué par un subtituant alkyle en C₁-C₄, alcoxy en C₁-C₄, hydroxy ou halogène, ou un groupe amino ou alkylamino en C₁-C₄ substitué par un radical N-formyle ou N-alcanoyle en C₁-C₄ ;
R² représente un atome d'hydrogène ou d'halogène ou un groupe alkyle en C₁-C₄, ou, lorsque R^{1III} n'est pas un groupe hydroxy ni un groupe contenant le radical hydroxyphényle, également un groupe alcoxy en C₁-C₄ ;
R⁶ représente un atome d'hydrogène ou un radical alkyle en C₁-C₄ qui peut éventuellement être substitué par le groupe hydroxy, un radical alcényle en C₁-C_{4,} cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄ , halogène, alcoxy en C₁-C₄ , hydroxy ou hydroxyalcoxy, ou un groupe alcoxycarbonyl en C₁-C₄ ou (alcoxycarbonyl en C₁-C₄)-alkyle en C₁-C₄, ou un groupe phényle ou phényl-alkyle en C₁-C₄ substitué sur le noyau phényle par un radical (alcoxycarbonyl en C₁-C₄ )-alcoxy en C₁-C₄;
R⁷ représente un atome d'hydrogène ou un radical alkyle en C₁-C₇ qui peut éventuellement être substitué par le groupe hydroxy, un radical alcényle en C₃-C₇, cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄ , halogène, alcoxy en C₁-C₄ , hydroxy ou hydroxyalcoxy, ou un groupe alcoxycarbonyl en C₁-C₄ ou (alcoxycarbonyl en C₁-C₄)-alkyle en C₁-C₄, ou un groupe phényle ou phényl-alkyle en C₁-C₄ substitué sur le noyau phényle par un radical (alcoxycarbonyl en C₁-C₄ )-alkyle en C₁-C₄ ;
A représente une chaîne alkylène ayant 1 ou 2 atomes de carbone, éventuellement substituée par un groupe alkyle en C₁-C₄ , ou une liaison ou un atome d'oxygène ou de soufre;
B représente un atome d'azote ou le groupe CH;
R⁵ représente un radical pyridyle ou phényle éventuellement substitué par un substituant alkyle en C₁-C₄ , alcoxy en C₁-C₄ ou halogène; et
Q représente une chaîne -Q'-CO- dans laquelle Q' représente une chaîne alkylène ayant de 1 à 3 atomes de carbone, qui peut éventuellement être substituée par un groupe alkyle en C₁-C₄ ou par le groupe oxo, ou Q représente une chaîne alkylène ayant de 2 à 4 atomes de carbone, substituée par le groupe oxo dans la position voisine du squelette indole, et qui peut éventuellement être substituée par un groupe alkyle en C₁-C₄, ou, lorsque R^{1III}, R⁶ et/ou R⁷ représentent un radical contenant le groupe -CO-, Q peut également représenter une chaîne alkylène ayant de 2 à 4 atomes de carbone, qui peut éventuellement être substituée par un groupe alkyle en C₁-C₄ ou bien, dans la position voisine du squelette indole, par le groupe hydroxy.

12. Composés de formule générale V 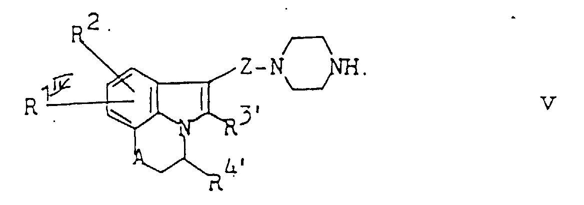 dans laquelle
R^{1IV} représente un atome d'hydrogène ou d'halogène, ou un groupe alcoxy en C₁-C₄, alkylthio en C₁-C₄, hydroxy, trifluorométhyle, nitro, amino, mono- ou dialkylamino en C₁-C₄ , alkyle en C₁-C₇, un groupe phényl-alkyle en C₁-C₄ qui peut éventuellement être substitué sur le noyau phényle par un substituant alkyle en C₁-C₄ , alcoxy en C₁-C₄, hydroxy ou halogène, un radical cycloalkyle en C₃-C₆, cycloalkylalkyle en C₄-C₇, alcényle en C₃-C₇, alcanoyle en C₂-C₇, alcanoyloxy en C₁-C₄, alcanoylamino en C₁-C₄ , un groupe benzoyle, benzoyloxy ou benzoylamino dont le noyau phényle peut éventuellement être substitué par un substituant alkyle en C₁-C₄ , alcoxy en C₁-C₄, hydroxy ou halogène, ou un groupe cinnamoyle, cinnamoyloxy ou cinnamoylamino, dont le noyau phényle peut éventuellement être substitué par un subtituant alkyle en C₁-C_{4,} alcoxy en C₁-C_{4,} hydroxy ou halogène;
R² représente un atome d'hydrogène ou d'halogène ou un groupe alkyle en C₁-C₄, ou, lorsque R^{1IV} n'est pas un groupe hydroxy ni un groupe contenant le radical hydroxyphényle, également un groupe alcoxy en C₁-C₄ ;
R^{3'} représente un atome d'hydrogène ou un radical alkyle en C₁-C₄ , alcényle en C₁-C₄, cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄, halogène, alcoxy en C₁-C₄ ou hydroxy, R^{3'} ne pouvant toutefois contenir qu'un seul groupe hydroxy libre lorsque R^{1IV} ne contient pas de groupe carbonyloxy;
R^{4'} représente un atome d'hydrogène ou un radical alkyle en C₁-C₇, alcényle en C₃-C₇, cycloalkyle en C₃-C₆, cycloalkylalkyle en C₄-C₇ ou un groupe phényle ou phényl-alkyle en C₁-C₄ qui peut éventuellement être substitué sur le noyau phényle par un substituant alkyle en C₁-C₄, halogène, alcoxy en C₁-C₄ ou hydroxy, R^{4'} ne pouvant toutefois contenir qu'un seul groupe hydroxy libre lorsque R^{1IV} ne contient pas de groupe carbonyloxy;
A représente une chaîne alkylène ayant 1 ou 2 atomes de carbone, éventuellement substituée par un groupe alkyle en C₁-C₄ , ou une liaison ou un atome d'oxygène ou de soufre;
Z représente une chaîne alkylène ayant de 2 à 4 atomes de carbone, qui peut éventuellement être substituée par un groupe alkyle en C₁-C₄ ou,lorsque R^{1IV} ne contient pas de groupe carbonyloxy, également par le radical hydroxy.
